# EUROPÄISCHE PATENTSCHRIFT

(11) **EP 3 237 341 B1**
(45) Veröffentlichungstag und Bekanntmachung des Hinweises auf die Patenterteilung: **28.08.2019**
(21) Anmeldenummer: 15832942.5
(22) Anmeldetag: 22.12.2015
(51) Int. Cl.: A23L 3/02, A23L 3/18, A23L 2/46, A23L 3/00, A23L 3/28, A61L 2/02, A61L 2/10, A61L 9/00, C02F 9/00, C12H 1/16, B65B 55/02, C02F 1/32, C02F 1/02, C02F 1/28, C02F 1/44, C02F 103/02

(54) **VERFAHREN UND VORRICHTUNG ZUR BEHANDLUNG VON LEBENSMITTELN IN GESCHLOSSENEN BEHÄLTNISSEN ZUR AUFNAHME VON LEBENSMITTELN**
METHOD AND DEVICE FOR TREATING FOOD IN CLOSED CONTAINERS FOR HOLDING FOODS
PROCÉDÉ ET DISPOSITIF DE TRAITEMENT DE PRODUITS ALIMENTAIRES DANS DES RÉCIPIENTS DESTINÉS À CONTENIR DES PRODUITS ALIMENTAIRES

(30) Priorität: 22.12.2014 AT 509352014
(43) Veröffentlichungstag der Anmeldung: 01.11.2017
(73) Patentinhaber: Red Bull GmbH, 5330 Fuschl am See (AT)
(72) Erfinder: CONCIN, Roland, 5330 Fuschl am See (AT); EDER, Harald, 5301 Eugendorf (AT); RINDERER, Christian, 5330 Fuschl am See (AT); RINDERER, Matthias, 5330 Fuschl am See (AT); VIECHTBAUER, Volker, 5330 Fuschl am See (AT)
(74) Vertreter: Burger, Hannes
(86) Internationale Anmeldenummer: PCT/AT2015/050326
(87) Internationale Veröffentlichungsnummer: WO 2016/100996

(56) Entgegenhaltungen:
- JP-A- 2000 246 206
- JP-A- 2000 246 206
- JP-A- 2005 137 949
- JP-A- 2005 137 949
- JP-A- 2014 128 802
- FAEHNRICH M ET AL: "AUFBEREITUNG VON SCHWACH BELASTETEN PROZESSABWAESSERN IN DER LEBENSMITTELINDUSTRIE", CHEMIE INGENIEUR TECHNIK, WILEY VCH. VERLAG, WEINHEIM; DE, Bd. 69, Nr. 8, 1. August 1997 (1997-08-01) , XP000829070, ISSN: 0009-286X, DOI: 10.1002/CITE.330690819
- FAEHNRICH M ET AL: "AUFBEREITUNG VON SCHWACH BELASTETEN PROZESSABWAESSERN IN DER LEBENSMITTELINDUSTRIE", CHEMIE INGENIEUR TECHNIK, WILEY VCH. VERLAG, WEINHEIM; DE, vol. 69, no. 8, 1 August 1997 (1997-08-01) , page 1147/1148, XP000829070, ISSN: 0009-286X, DOI: 10.1002/CITE.330690819

## Beschreibung

Die Erfindung betrifft ein Verfahren zur Behandlung von Lebensmitteln in Behältnissen zur Aufnahme von Lebensmitteln, sowie eine Vorrichtung für die Behandlung von Lebensmitteln in Behältnissen zur Aufnahme von Lebensmitteln. Insbesondere betrifft die Erfindung ein Verfahren und eine Vorrichtung zur Behandlung von Genussmitteln, insbesondere alkoholischen und nichtalkoholischen Getränken. Die Lebensmittel, insbesondere die Genussmittel bzw. die Behältnisse zur Aufnahme der Lebensmittel, werden dabei mittels einer Prozessflüssigkeit in zumindest einer Behandlungszone behandelt. Erfindungsgemäß wird im Behandlungsbetrieb eine der im Kreis geführten Prozessflüssigkeit zur Bildung zumindest eines Stromes der Prozessflüssigkeit herangezogen, und mittels einer Membranfiltrationsanlage filtriert.

Verfahren bzw. Vorrichtungen zur Behandlung von Produkten und/oder Behältnissen sind in verschiedenen Varianten aus dem Stand der Technik bekannt. Häufig werden die Produkte, zum Beispiel Lebensmittel, und/oder die Behältnisse dabei mittels einer temperierten Prozessflüssigkeit behandelt, wie dies zum Beispiel bei Pasteurisierungen von Lebensmittelprodukten in sogenannten Pasteuren der Fall ist. In den meisten Fällen wird als Prozessflüssigkeit Wasser bzw. eine wässrige Lösung eingesetzt, welche indirekt auf die Produkte bzw. direkt auf die Behältnisse einwirkt.

Zwecks Vermeidung großer Mengen an Flüssigkeitsabfall bzw. Abwasser wird die Prozessflüssigkeit bzw. das Prozesswasser häufig zumindest teilweise im Kreis durch die Vorrichtung bzw. die Behandlungszone(n) geführt, die Prozessflüssigkeit wird also in einem Umlaufverfahren wiederverwendet. Diese Vorgangsweise bedingt allerdings ein erhöhtes Potential für eine Verschmutzung bzw. Kontamination der Prozessflüssigkeit. Anlagen zur Behandlung von Produkten bzw. Behältnissen sind in den meisten Fällen begehbar, es sind also keineswegs Bedingungen mit kontrollierter Luftumwälzung oder dergleichen gegeben. Zusätzlich zur Umgebungsluft sind weitere Schmutzquellen, wie zum Beispiel das Bedienungspersonal oder andere Personen, Förderelemente für die Behältnisse bzw. die Produkte, etwaig vorhandene Kühleinrichtungen für die Prozessflüssigkeit, insbesondere luftgekühlte Kühltürme, oder aber die frisch in das Verfahren bzw. die Vorrichtung eingebrachte Prozessflüssigkeit selbst, vorhanden. Weiters kann ein Eintrag von Verunreinigungen durch den Behandlungsprozess selbst erfolgen, etwa durch Beschädigungen der Behältnisse und Verunreinigung der Prozessflüssigkeit durch das Produkt, oder durch Ablösen von Partikeln, beispielsweise Farbpartikeln oder dergleichen, von der Außenseite der Behältnisse.

In der Vergangenheit wurden einige Verfahren vorgeschlagen, um Verunreinigungen aus einer Prozessflüssigkeit zu entfernen. Es handelt sich hierbei um Filtrationsmaßnahmen zur Entfernung verhältnismäßig grober bzw. großer Partikel, wie etwa Glasscherben, Sand, Kies und dergleichen. Als Beispiel sei die EP 2 722 089 A1 genannt. In der EP 2 722 089 A1 ist eine Vorrichtung zur thermischen Behandlung von Produkten in Behältern beschrieben. Die Behälter werden dabei mit einer Prozessflüssigkeit berieselt bzw. besprüht, und die Prozessflüssigkeit, beispielsweise Wasser, wird zum zumindest teilweisen Wiederverwenden in einem Umlaufkreislauf geführt. Zur Reinigung der Prozessflüssigkeit kommt eine Schwerkraftsedimentationsvorrichtung zum Einsatz, mittels welcher grobkörnige bzw. große Partikel, wie Glasscherben oder Sand abgeschieden werden können.

Die aus dem Stand der Technik bekannten Methoden beschreiben Filtrationsmethoden zur Abscheidung von verhältnismäßig großen Partikeln aus einer Prozessflüssigkeit, mittels Abscheidungsvorrichtungen wie zum Beispiel konventionellen Maschensieben, Siebbändern oder Stecksieben, oder eben Sedimentationsvorrichtungen.

Diese aus dem Stand der Technik bekannten Maßnahmen sind jedoch nicht geeignet, um insbesondere Verschmutzungen mit verhältnismäßig kleiner Partikelgröße aus der Prozessflüssigkeit zu entfernen. Dies betrifft besonders Partikelverunreinigungen, welche durch konventionelle Filtersiebe und sonstige Abscheidevorrichtungen nicht entfernt werden können, bzw. sich zum Beispiel nicht oder nur sehr langsam aus einem Prozesswasserstrom absetzen.

Im Besonderen können sich gerade in Vorrichtungen zur thermischen Behandlung von Produkten bzw. Behältnissen mittels einer Prozessflüssigkeit bzw. eines Prozesswassers, mit der Zeit im beträchtlichen Ausmaß Mikroorganismen im Prozesswasser vermehren. Solche Mikroorganismen, beispielsweise Bakterien, Pilzsporen, aber auch Viren, können dabei sowohl mit frischem Prozesswasser, oder aber auch durch Bedienungspersonal oder andere Personen in die Vorrichtung eingebracht werden. In weiterer Folge kann es beispielsweise auch zu einer Algenbildung bzw. einem Algenwachstum kommen. Bei einer thermischen Behandlung weist das Prozesswasser oftmals zumindest zonenweise Temperaturen auf, welche ein Wachstum bzw. eine Vermehrung von Mikroorganismen sogar noch begünstigen. Grundsätzlich kann dabei nicht gänzlich ausgeschlossen werden, dass zum Beispiel Bakterienkulturen im Zuge der Behandlung auf die Außenseite der Behälter gelangen, und dort zumindest teilweise auch nach dem Behandlungsvorgang verbleiben.

Als besonders problematisch ist die hinsichtlich einer Filtration mittels konventionellen Sieben ungünstige Partikelgrößenverteilung in einem typischen Prozesswasser anzusehen. Insbesondere begünstigt der Einsatz von Chemikalien wie etwa Tensiden sehr kleine Partikelgrößen, welche mit konventionellen Abscheidevorrichtungen nicht oder nur in unzureichendem Ausmaß aus der Prozessflüssigkeit entfernt werden können. Daher muss der Produktions- bzw. Behandlungsbetrieb in derzeit bekannten Verfahren zur Behandlung von Produkten bzw. Behältnissen, in welchen die Prozessflüssigkeit zumindest teilweise im Kreis geführt wird, in verhältnismäßig kurzen Intervallen unterbrochen werden, um die Behandlungsvorrichtung zu reinigen und zu entkeimen. Solche Reinigungsvorgänge sind in der Regel sehr aufwendig, und führen insbesondere zu Produktionsverlusten, und damit einhergehend zu finanziellen Verlusten.

Des Weiteren sind aus dem Stand der Technik grundsätzlich Reinigungs- bzw. Wasseraufbereitungsmethoden bekannt, welche Membranfiltrationstechniken nutzen. Zum Beispiel ist aus Faehnrich M et al:" Aufbereitung von schwach belasteten Prozessabwässern in der Lebensmittelindustrie", Chemie Ingenieur Technik, Wiley Vch. Verlag, Weinheim; DE, Bd. 69, Nr.8, 1. August 1997 bekannt, Abwasser, welches beim Spülen von Fleischprodukten mittels Kühlduschen anfällt zu sammeln, mittels Nanofiltration und weiteren Methoden zu reinigen und wiederzuverwenden.

Aus der JP 2000 246206 A ist ein Verfahren und eine Waschanlage für offene Transportbehältnisse zum Transportieren von Lebensmitteln bekannt. Die Transportbehältnisse werden hierbei in mehreren Waschstufen mittels einer Tensidhaltigen Waschflüssigkeit gereinigt. Die jeweilige Waschflüssigkeit dieser Waschstufen kann in einem Kreislauf zumindest teilweise wieder derselben Waschstufe zugeführt werden. Bei einer dieser Waschstufen ist ein zusätzlicher zweiter Wasserkreislauf mit einem Membranfiltermodul offenbart, auf welchen zweiten Kreislauf im Falle in Abhängigkeit vom Verschmutzungsgrad der Waschflüssigkeit dieser Waschstufe umgeschaltet wird.

Die JP 2005 137949 A offenbart ein Sammeln von schwach belasteten Prozesswasser und dessen Aufbereitung und einmalige Wiederverwendung. Das Abwasser mit niedrigem Totalgehalt an organischem Kohlenstoff (TOC) wird mittels Membranfiltration aufbereitet, und nach einmaliger Wiederverwendung aus dem Prozess abgeführt.

Die JP 2014 128802 A offenbart eine Methode und ein System zur Entfernung von Aldehyden aus Roh- und Abwässern durch chemische Behandlung mit Hydrogensulfit-Ionen in einem Mischtank. Nach einer Reaktionszeit in einem Mischtank wird das chemisch behandelte Wasser einer Umkehrosmose zugeführt, um durch die chemische Behandlung entstandene Substanzen zu entfernen. Das System wird hierzu im Batch-Betrieb betrieben.

Aufgabe der Erfindung ist es daher, ein verbessertes Verfahren und eine verbesserte Vorrichtung zu entwickeln, welche die im Stand der Technik noch vorhandenen Defizite zu beheben vermögen. Insbesondere sollen ein im Vergleich zum Stand der Technik, verbessertes Verfahren bzw. eine verbesserte Vorrichtung zur Behandlung von Lebensmitteln in Behältnissen bereitgestellt werden, bei welchem Verfahren bzw. bei welcher Vorrichtung die Prozessflüssigkeit zur Wiederverwendung im Verfahren im Kreis geführt werden kann, aber die damit einhergehenden Nachteile durch die kontinuierlich steigende Verunreinigung bzw. Verkeimung der Prozessflüssigkeit möglichst hintangehalten sind.

Die Aufgabe der Erfindung wird durch die Merkmale des Anspruchs 1 gelöst.

Die Lebensmittel in Behältnissen werden dabei in eine Behandlungszone eingebracht bzw. durch eine Behandlungszone befördert. Der Behandlungszone oder den Behandlungszonen wird jeweils zumindest ein Flüssigkeitsstrom der Prozessflüssigkeit zum Einwirken auf die Lebensmittel in Behältnissen zugeführt, und nach erfolgter Behandlung der Lebensmittelprodukte aus der Behandlungszone wieder abgeführt, wobei die Prozessflüssigkeit zur Behandlung der Lebensmittel bzw. der Behältnisse zwecks Wiederverwendung im Verfahren im Kreis wieder in die Behandlungszone oder in die Behandlungszonen geführt wird.

Im kontinuierlichen, laufenden Behandlungsbetrieb wird aus der pro Zeiteinheit insgesamt durch alle vorhandenen Behandlungszonen geführten Prozessflüssigkeit, pro Zeiteinheit eine Teilmenge der Prozessflüssigkeit zur Bildung wenigstens eines Stromes der Prozessflüssigkeit herangezogen bzw. entnommen, und der wenigstens eine gebildete Strom der Prozessflüssigkeit zur Reinigung und Entkeimung der Prozessflüssigkeit, insbesondere zur Entfernung von Partikeln aus der Prozessflüssigkeit, mittels zumindest einer Membranfiltrationsanlage filtriert, und ein filtrierter Strom nach dem Filtrationsvorgang zumindest teilweise einem die Prozessflüssigkeit beinhaltenden bzw. führenden Element und/oder einer Behandlungszone wieder zugeführt.

Unter einem die Prozessflüssigkeit beinhaltenden bzw. führenden Element, bzw. Führungselement für die Prozessflüssigkeit wird hier und im Folgenden jegliches Element verstanden, welches zur Beinhaltung der Prozessflüssigkeit bzw. zur Leitung eines Flüssigkeitsstromes der Prozessflüssigkeit ausgestaltet ist. Das können beispielsweise Rohrleitungen, Kanäle und dergleichen sein, in welchen die Prozessflüssigkeit zum Beispiel einer Behandlungszone zugeführt oder aus einer Behandlungszone abgeführt wird. Weiters werden unter dem Begriff "Führungselement" beispielsweise, aber nicht ausschließend Sammelbecken, Tanks, oder in den oder außerhalb der Behandlungszonen angeordneten Auffangeinrichtungen für die Prozessflüssigkeit oder dergleichen verstanden.

Unter einer Behandlungszone wird hier und im Folgenden eine Zone verstanden, in welcher die Lebensmittel indirekt und die Behältnisse direkt mit der Prozessflüssigkeit in Kontakt gebracht werden. Dabei können die physikalischen und/oder chemischen und/oder sonstigen Parameter der Prozessflüssigkeit gezielt für den jeweiligen Behandlungszweck der Zone eingestellt sein bzw. werden. Ein häufig angewandtes Verfahren ist die Pasteurisierung von Lebensmittelprodukten, bei welchem sich die Lebensmittel bereits in verschlossenen Behältnissen befinden, und die Prozessflüssigkeit auf die Außenseite der Behältnisse einwirkt. Dabei kann die Prozessflüssigkeit auf die Außenseite der Behältnisse geschüttet, bzw. die Behältnisse mit der Prozessflüssigkeit berieselt oder besprüht werden. Letztlich ist unter dem Begriff Behandlungszone somit zumindest im weitesten Sinne auch ein die Prozessflüssigkeit beinhaltendes bzw. führendes Element, bzw. Führungselement für die Prozessflüssigkeit zu verstehen.

Unter einem Flüssigkeitsstrom der Prozessflüssigkeit wird hier und im Folgenden jegliche Art von geführt bewegter Prozessflüssigkeit verstanden, unabhängig davon wie der Flüssigkeitsstrom bzw. dessen Führung ausgestaltet sind. Das heißt, dass unter dem Begriff "Flüssigkeitsstrom" zum Beispiel ein Strom einer bewegten Prozessflüssigkeit in Führungselementen, wie etwa Rohrleitungen, Kanälen, Becken, Tanks etc. genauso umfasst ist, wie auch beispielsweise ein unter Umgebungsluftdruck frei fallender Berieselungsstrom oder Sprühstrom der Prozessflüssigkeit in einer Behandlungszone oder ein Strom der Prozessflüssigkeit in einer Rückkühleinrichtung oder dergleichen.

Durch die im Anspruch 1 angegebenen Maßnahmen wird ein Verfahren bereitgestellt, welches sich besonders für die Entfernung von Verschmutzungen bzw. Kontaminationen mit sehr kleiner Partikelgröße, wie etwa Bakterienkolonien aus der Prozessflüssigkeit bzw. zur Entkeimung der Prozessflüssigkeit hervorragend eignet. Damit lässt sich eine wesentliche Verbesserung gegenüber bekannten Verfahren erreichen, welche hinsichtlich einer Entfernung kleiner Partikel sowie Mikroorganismen aus einer zumindest teilweise im Kreis geführten Prozessflüssigkeit nur sehr eingeschränkt, oder gar nicht wirksam sind. Auf diese Weise kann insbesondere eine Verkeimung der durch alle vorhandenen Behandlungszonen geführten Prozessflüssigkeit wirksam hintangehalten werden. Die Mikrofiltration bzw. Ultrafiltration mittels der Membranfiltrationsanlage(n) kann dabei vorteilhafterweise im laufenden Behandlungsbetrieb durchgeführt werden, und ist auch vergleichsweise effizient und energiesparend.

Die genaue Anzahl, sowie Art und Ort der Einbindung der Membranfiltrationsanlage(n) bzw. die Anknüpfung einer Membranfiltrationsanlage an die die Prozessflüssigkeit leitenden Führungselemente und/oder Behandlungszone(n), kann dabei unter Berücksichtigung von baulichen Merkmalen bzw. Prozessparametern und dergleichen festgelegt bzw. vorgenommen werden. Jedoch können sich bei bestimmten Anordnungsvarianten Vorteile für das Verfahren zur Behandlung von Lebensmitteln bzw. Behältnissen ergeben, welche nachstehend noch näher erläutert werden.

Zur Filtration großkörniger Verunreinigungen in der Prozessflüssigkeit können beispielsweise zusätzlich Schmutzfänger oder dergleichen in den Führungselementen angeordnet sein. Zum Beispiel kann auch eine Schwerkraftsedimentationsvorrichtung, wie sie in der bereits zitierten EP 2 722 089 A1 beschrieben ist, zur Abscheidung von großen bzw. großkörnigen Partikeln eingesetzt werden.

Durch die in Anspruch 1 angegebenen Maßnahmen wird außerdem erreicht, dass aufwendige und kostspielige Unterbrechungen des Produktions- bzw. Behandlungsbetriebs zwecks Reinigung und Entkeimung der Behandlungsvorrichtung möglichst hintangehalten werden können, bzw. zumindest die Zeitintervalle zwischen solchen Reinigungsprozessen wesentlich vergrößert werden können. Zusätzlich kann durch die Membranfiltration die Menge an chemischen Stabilisatoren zur Aufbereitung der Prozessflüssigkeit, insbesondere die benötigten Mengen an Tensiden, Korrosionsinhibitoren und/oder Desinfektionsmitteln reduziert werden, bzw. kann der Einsatz von Reinigungschemikalien zumindest größtenteils eingespart bzw. minimiert werden.

Durch die kontinuierliche Entfernung von Verunreinigungen, insbesondere von Partikeln von Mikroorganismen, wie zum Beispiel Bakterienkolonien, kann weiters der Einsatz von Bioziden zumindest stark reduziert werden. Zusätzlich bzw. in weiterer Folge kann eine wesentliche Verbesserung hinsichtlich der Umweltbelastung erzielt werden, bzw. auch die Belastung einer dem Verfahren bzw. der Vorrichtung nachgeschalteten Einrichtung, wie etwa einer Kläranlage, minimiert werden.

Außerdem können die erfindungsgemäßen Maßnahmen auch olfaktorische Faktoren verbessern, da eine Bildung ungewünschter bzw. unangenehmer Gerüche hintangehalten werden kann. Diverse Mikroorganismen, insbesondere Bakterien sind bekannt dafür, übel riechende Duftstoffe als Stoffwechselprodukte zu generieren. Durch Entfernen dieser Mikroorganismen aus der Prozessflüssigkeit, kann eine unangenehme Geruchsbelastung weitestgehend vermieden werden.

Bei längerem Verbleib von Bakterienkulturen in einer Prozessflüssigkeit unter gleichzeitigem Einsatz von Bioziden, können sich Bakterienstämme an die eingesetzten Biozide "gewöhnen" bzw. gegenüber den eingesetzten Bioziden resistente Bakterienstämme gebildet werden. Das heißt, dass Biozide zur Entfernung von Mikroorganismen aus der Prozessflüssigkeit mit der Zeit unwirksam werden können. Durch die kontinuierliche Entfernung der Mikroorganismen aus der Prozessflüssigkeit durch Mikrofiltration mittels zumindest einer Membranfiltrationsanlage, kann die Bildung resistenter Keime möglichst hintangehalten werden, bzw. kann der Einsatz von Bioziden insgesamt minimiert werden, da ein effizientes und wirksames Mittel bereitgestellt ist, um generell Mikroorganismen aller Art aus der Prozessflüssigkeit kontinuierlich zu entfernen. Durch die Filtration mittels zumindest einer Membranfiltrationsanlage können beispielsweise auch biozidresistente und/oder chlorresistente Bakterienstämme wirksam aus der Prozessflüssigkeit entfernt werden.

Selbstverständlich können auch andere organische und anorganische Klein- und Kleinstpartikel mittels der zumindest einen Membranfiltrationsanlage aus der Prozessflüssigkeit entfernt werden. Dadurch ergibt sich auch gleichzeitig eine weitere Verbesserung hinsichtlich des Mikroorganismenwachstums bzw. hinsichtlich der Wachstumsrate von Mikroorganismen in der Prozessflüssigkeit, da organische und anorganische Klein- und Kleinstpartikel oft gute Nährgrundlagen bzw. "Nährböden", beispielsweise für Bakterien bilden können. Durch das Entfernen von unerwünschten Partikelverunreinigungen mittels der Membranfiltration, kann auch der Einsatz von sonst notwendigen Chemikalien zur Eliminierung solcher Verunreinigungen, wie zum Beispiel von Tensiden, wirksam gesenkt werden.

Weiters können durch die erfindungsgemäßen Maßnahmen auch staubartige Verschmutzungen aus der Prozessflüssigkeit entfernt werden. Solche Verschmutzungen können beispielsweise im Zuge der Herstellung von Behältnissen durch formgebende bzw. spanende Bearbeitungsschritte wie etwa Schneiden, Fräsen, Bohren oder dergleichen verursacht werden. Bei der Herstellung von Behältnissen kann zum Beispiel Glas- oder Metallstaub, insbesondere Aluminiumstaub anfallen, welche Stäube mit den Behältnissen in das Verfahren zur Behandlung von Lebensmitteln bzw. Behältnissen eingebracht werden können.

Schließlich kann durch die erfindungsgemäßen Maßnahmen auch eine Verschmutzung der Außenseite der Behältnisse, sowie eine Verschmutzung der Oberflächen der Vorrichtung zur Behandlung der Lebensmittel bzw. Behältnisse durch die Prozessflüssigkeit selbst wirksam hintangehalten werden. Dies bringt weitere Vorteile hinsichtlich allfälliger Nachbehandlungs- bzw. zusätzlicher Reinigungsschritte, deren Ausmaß zumindest verringert werden kann. Gegebenenfalls kann aufgrund der erfindungsgemäßen Maßnahmen eine Nachreinigung der Behältnisse mit Waschchemikalien und/oder eine Sterilisation der Behältnisse erübrigt werden.

Die Maßnahme, die zu behandelnden Lebensmittel vor der Behandlung in Behältnisse abzufüllen, die Behältnisse zu verschließen, und die Prozessflüssigkeit auf die Außenseite der Behältnisse so zu applizieren, dass die Prozessflüssigkeit die Außenseite der Behältnisse umströmt, stellt eine besonders effiziente Maßnahme zur Behandlung von Lebensmitteln dar, da nach der Behandlung ein bereits fertig abgefülltes Handelsprodukt bereitgestellt werden kann. Außerdem kann so die Gefahr einer Rekontamination der Lebensmittel im Zuge eines an die Behandlung anschließenden Abfüllschrittes ausgeschlossen werden.

Durch die Maßnahme, vor der Behandlung der Lebensmittel die Temperatur der Prozessflüssigkeit einzustellen, und die Behandlung der Lebensmittel durch indirekte Wärmeübertragung mittels der Prozessflüssigkeit durchzuführen, kann ein direkter Kontakt der Lebensmittel mit Heiz- und/oder Kühlmitteln, aber auch mit einer Prozessflüssigkeit vermieden werden. Weiters stellt dieses Verfahrensmerkmal eine effektive Methode zur Einstellung einer Behandlungstemperatur für die Lebensmittel dar und auf diese Art und Weise können die Lebensmittel besonders effizient behandelt werden.

Weiters kann es zweckmäßig sein, die Lebensmittelprodukte in zumindest einer Behandlungszone mittels einer erhitzten Prozessflüssigkeit zu pasteurisieren. Dadurch kann für das Lebensmittel eine längere Haltbarkeit erreicht werden.

Die Maßnahme, die Temperaturen der jeweiligen Flüssigkeitsströme der Prozessflüssigkeit in kontrollierter Art und Weise vor der Zuführung in eine Behandlungszone separat für jede Behandlungszone einzustellen, bringt den Vorteil, dass das gesamte Verfahren zur Behandlung der Lebensmittel bzw. Behältnisse besser kontrollierbar ist. Insbesondere kann so eine ungewünschte Beschädigung der Lebensmittel und/oder der Behältnisse aufgrund zu raschen bzw. zu hohen Temperaturwechseln hintangehalten werden.

Dabei kann es besonders hinsichtlich einer Pasteurisierung der Lebensmittel sinnvoll sein, die zu behandelnden Lebensmittel, insbesondere Genussmittelprodukte, aufeinanderfolgend in zumindest einer Behandlungszone zu erwärmen, in zumindest einer Behandlungszone zu pasteurisieren, und in zumindest einer Behandlungszone abzukühlen. Einerseits kann durch eine langsame Erwärmung in zumindest einer Erwärmungszone eine schonende Erwärmung für die Lebensmittel gewährleistet werden. Durch das aktive Abkühlen in zumindest einer Abkühlzone nach dem Pasteurisieren, kann andererseits eine sogenannte "Überpasteurisierung" aufgrund hoher Temperatur der Lebensmittel für einen zu langen Zeitraum wirksam verhindert werden. Eine solche Überpasteurisierung bedingt häufig unerwünschte Veränderungen im Lebensmittel, bzw. kann den Geschmack und/oder Geruch des Lebensmittels negativ beeinflussen. Durch die angegebenen Verfahrensschritte zur sequentiellen Behandlung der Lebensmittel, insbesondere Genussmittelprodukte, ist eine gut steuerbare und für die Lebensmittel sehr schonende Verfahrensführung ermöglicht. Beispielsweise kann die Temperatur der Prozessflüssigkeit für mehrere Erwärmungszonen schrittweise von Raumtemperatur auf beispielsweise 65 °C erhöht werden, kann in einer oder mehreren Zonen die Prozessflüssigkeit mit Pasteurisierungstemperatur, zum Beispiel 80 bis 85 °C eingebracht werden, und daran anschließend kann die Prozessflüssigkeit in mehreren Kühlzonen für die Lebensmittel bzw. Behältnisse wiederum zonenweise mit einer sequentiell niedrigeren Temperatur zum Abkühlen der Lebensmittel bzw. Behältnisse, eingebracht werden.

Alternativ zu den angegebenen Temperaturbereichen, welche beispielhaft für eine Pasteurisierung von Lebensmitteln angegeben sind, können für andere Behandlungsverfahren selbstverständlich auch andere Temperaturbereiche zweckmäßig sein. Als zusätzliches Beispiel wird an dieser Stelle eine Heißdampfsterilisation angeführt, bei welcher auch Temperaturen der Prozessflüssigkeit bzw. eines Prozesswassers über 100°C angewendet werden können, sodass das Prozesswasser zumindest in Sterilisationszonen im gasförmigen Zustand auf die Behältnisse einwirkt.

Als besonders zweckmäßig hat sich erwiesen, wenn ein Flüssigkeitsstrom wenigstens einer Behandlungszone zum Erwärmen der Lebensmittel und/oder der Behältnisse bei einer Temperatur zwischen 40 °C und 50 °C zugeführt wird. Dadurch ist eine möglichst schonende Vorerwärmung der Lebensmittel bereitgestellt, und können allzu große Temperaturdifferenzen bzw. Temperatursprünge im Zuge einer Vorwärmung der Lebensmittel bzw. der Behältnisse hintangehalten werden.

Hinsichtlich der Reinigungseffizienz für die Prozessflüssigkeit im kontinuierlichen Behandlungsbetrieb kann es zweckmäßig sein, die im kontinuierlichen Behandlungsbetrieb aus zumindest einem die Prozessflüssigkeit beinhaltenden und/oder führenden Element pro Zeiteinheit insgesamt zur Bildung wenigstens eines Stromes der Prozessflüssigkeit herangezogenen Prozessflüssigkeitsmengen so zu wählen, dass durch die Filtration des Stromes bzw. der Ströme eine Entfernungsrate für Mikroorganismen erzielbar ist, welche größer ist, als die Wachstumsrate dieser Mikroorganismen in der Prozessflüssigkeit im gleichen Zeitintervall. Dadurch kann insbesondere erreicht werden, dass die Gesamtmenge an Mikroorganismen in der Prozessflüssigkeit möglichst minimiert ist, und eine Zunahme der Gesamtmenge an Mikroorganismen in der Prozessflüssigkeit im Zuge der kontinuierlichen Behandlung der Lebensmittel und/oder Behältnisse wirksam unterbunden ist.

Überraschenderweise hat sich herausgestellt, dass pro Zeiteinheit die Filtration einer verhältnismäßig kleinen Teilmenge der Prozessflüssigkeit, bezogen auf die Summe der Mengen aller pro Zeiteinheit insgesamt durch die Vorrichtung bzw. die Behandlungszonen geführten Flüssigkeitsströme der Prozessflüssigkeit, völlig genügt, um ein ausreichendes Filtrationsergebnis zu erzielen, bzw. eine Prozessflüssigkeit mit ausreichender Reinheit zu erhalten. Dadurch können die bauliche Größe und/oder die Anzahl der Membranfiltrationsanlage(n) vergleichsweise gering gehalten werden, ohne hinsichtlich der Reinheit der Prozessflüssigkeit Kompromisse eingehen zu müssen. Außerdem kann so die zur Reinigung der Prozessflüssigkeit aufzuwendende Energiemenge weiter reduziert werden.

Außerdem ist es sinnvoll, die zur Bildung wenigstens eines zu filtrierenden Stromes der Prozessflüssigkeit herangezogene Prozessflüssigkeitsmenge aus zumindest einem die Prozessflüssigkeit beinhaltenden bzw. führenden Element pro Zeiteinheit insgesamt so zu wählen bzw. einzustellen, dass - bezogen auf die pro Zeiteinheit insgesamt durch alle vorhandenen Behandlungszonen geführte Menge an Prozessflüssigkeit - pro Zeiteinheit zumindest 1 % und weniger als 25 % zur Bildung wenigstens eines Stromes der Prozessflüssigkeit herangezogen werden, und diese pro Zeiteinheit gebildete Teilmenge der Prozessflüssigkeit mittels zumindest einer Membranfiltrationsanlage filtriert wird. Dadurch wird einerseits gewährleistet, dass im laufenden Behandlungsbetrieb eine ausreichend hohe Anzahl an Verunreinigungen, insbesondere Kleinpartikel, aus der Prozessflüssigkeit entfernt wird. Andererseits kann so auch eine allzu aufwendige bauliche Ausgestaltung einer Membranfiltrationsanlage, bzw. der Einsatz einer großen Anzahl an Membranfiltrationsanlagen vermieden werden. Schließlich ist durch diesen Prozentsatzbereich für die pro Zeiteinheit zu filtrierende Menge an Prozessflüssigkeit ein Bereich angegeben, innerhalb welchem eine sinnvolle Anpassung an verschiedene Verfahrensführungen bzw. Verfahrenszwecke, insbesondere die Eingangsqualität der Prozessflüssigkeit und die im Behandlungsverfahren selbst vorhandene Verschmutzungsrate betreffend, vorgenommen werden kann. Bevorzugt wird die zur Bildung wenigstens eines zu filtrierenden Stromes der Prozessflüssigkeit herangezogene Prozessflüssigkeitsmenge aus zumindest einem die Prozessflüssigkeit beinhaltenden bzw. führenden Element pro Zeiteinheit insgesamt so eingestellt, dass - bezogen auf die pro Zeiteinheit insgesamt durch alle vorhandenen Behandlungszonen geführte Menge an Prozessflüssigkeit - pro Zeiteinheit zwischen 2 % und 10 %, und insbesondere zwischen 2,5 % und 7 % zur Bildung wenigstens eines Stromes der Prozessflüssigkeit herangezogen werden und diese pro Zeiteinheit gebildete Teilmenge der Prozessflüssigkeit mittels zumindest einer Membranfiltrationsanlage filtriert wird.

Zusätzlich kann es sinnvoll sein, wenn im kontinuierlichen Behandlungsbetrieb das insgesamt in der Vorrichtung zur Behandlung von Lebensmitteln bzw. Behältnissen enthaltene Volumen an Prozessflüssigkeit, pro Tag zumindest 1 mal und bevorzugt zwischen 2 mal und 10 mal über eine oder mehrere Membranfiltrationsanlage(n) geführt bzw. filtriert wird. So kann wiederum sichergestellt werden, dass in Abhängigkeit von verschiedenen Verfahrensparametern, beispielweise von der Eingangsqualität der Prozessflüssigkeit, von der im Behandlungsverfahren selbst vorhandenen Verschmutzungsrate, und insbesondere von der Mikroorganismenwachstumsrate in der Prozessflüssigkeit, eine ausreichende Menge der Prozessflüssigkeit durch Bildung von zumindest einem Strom und Filtration des gebildeten Stromes bzw. der gebildeten Ströme mittels wenigstens einer Membranfiltrationsanlage, gereinigt wird.

Unabhängig von der Art der Einbindung einer Membranfiltrationsanlage in die Vorrichtung zum Behandeln der Lebensmittel bzw. Behältnisse kann es sinnvoll sein, die Teilmengen an Prozessflüssigkeit zur Bildung wenigstens eines zu filtrierenden Stromes der Prozessflüssigkeit bei einer Temperatur der Prozessflüssigkeit von weniger als 80 °C und insbesondere weniger als 50 °C heranzuziehen bzw. zu entnehmen. Dadurch kann die Betriebsfähigkeit bzw. Lebensdauer einer Membranfiltrationsanlage wesentlich verlängert werden. Vor allem kann so die Langzeitstabilität der eigentlichen Filterelemente einer Membranfiltrationsanlage und insbesondere die Langzeitstabilität der eigentlichen Filtermembranen deutlich verbessert werden. Dies vor allem dann, wenn die Filterelemente bzw. Filtermembranen aus Kunststoff bestehen. Daher kann auch ein kostenintensiver Austausch von Filtermembranen bzw. Filtermembran-Modulen vermieden, bzw. zeitlich möglichst lange hinausgezögert werden.

Bei Verwendung von anderen Materialien für die Filterelemente bzw. Filtermembranen, zum Beispiel bei Verwendung keramischer Materialien, kann die Prozessflüssigkeit auch höhere Temperaturen, beispielsweise 100°C oder mehr aufweisen. Derartige Temperaturen sind zum Beispiel bei einer Heißdampfsterilisation durchaus üblich.

Bei einer Weiterbildung kann aber auch vorgesehen sein, dass zur Bildung wenigstens eines zu filtrierenden Stromes der Prozessflüssigkeit, Prozessflüssigkeit mit einer Temperatur zwischen 40 °C und 50 °C herangezogen wird. Bei einer Prozessflüssigkeitstemperatur in diesem Bereich sind besonders gute Filtrationsergebnisse durch eine Membranfiltration bzw. Mikro- und/oder Ultrafiltration erzielbar. Dies unter anderem deshalb, da insbesondere eine Verstopfung von Filtermembranen durch Schmiermittel wie zum Beispiel Paraffine bzw. Wachse, in diesem Temperaturbereich vermieden werden kann. Solche Schmiermittel werden häufig im Zuge der Herstellung von Behältnissen benutzt, bleiben nach der Herstellung teilweise an den Behältnissen haften, und können so in die Prozessflüssigkeit eingebracht werden. Durch Membranfiltration der Prozessflüssigkeit im angegebenen Temperaturbereich kann eine Prozessflüssigkeit mit besonders niedrigem Verschmutzungsgrad erzielt werden.

Hierbei kann auch vorgesehen sein, dass zur Bildung wenigstens eines zu filtrierenden Stromes der Prozessflüssigkeit, Prozessflüssigkeit aus einem temperierbaren Durchflussbehälter herangezogen wird. Auf diese Weise kann die Temperatur eines zu filtrierenden Stromes der Prozessflüssigkeit gezielt eingestellt werden, und die Filtrationseffizienz maximiert werden.

Durch die Maßnahme, einen mikro- oder ultrafiltrierten Strom der Prozessflüssigkeit bei Umgebungsdruck bzw. in freiem Gefälle wieder in zumindest ein die Prozessflüssigkeit beinhaltendes bzw. führendes Element und/oder in eine Behandlungszone zurückzuführen, wird der Vorteil erreicht, dass ein zusätzliches Fördermittel zum Einbringen bzw. Zurückführen eines filtrierten Stromes in die Prozessflüssigkeit nicht benötigt wird. Bei der Mikro- bzw. Ultrafiltration in einer Membranfiltrationsanlage kommt es zwangsläufig zu einem Druckverlust über die Filteranlage, sodass im Ablauf der Membranfiltrationsanlage nur ein geringer Vordruck vorhanden ist. Durch die Rückführung des filtrierten Stromes der Prozessflüssigkeit in freiem Gefälle kann aber auf das Aufbauen eines zusätzlichen Vordrucks zur Zwangsförderung eines filtrierten Stromes verzichtet werden. Dies stellt außerdem eine baulich einfach zu realisierende Variante dar.

Dabei kann es zweckmäßig sein, dass ein filtrierter Strom der Prozessflüssigkeit zumindest teilweise einem durch eine Behandlungszone bewegten Flüssigkeitsstrom der Prozessflüssigkeit nach dessen Einwirken auf die Lebensmittel bzw. die Behältnisse zugeführt wird. Diese Variante der Zuführung eines filtrierten Stromes in eine Behandlungszone ist besonders dann vorteilhaft, wenn ein Flüssigkeitsstrom der Prozessflüssigkeit unter einem gewissen Vordruck in die Behandlungszone eingebracht wird. Der Vordruck kann zum Beispiel für ein Zerstäuben der Prozessflüssigkeit notwendig sein, um die Behältnisse in der Behandlungszone möglichst gleichmäßig zu besprühen. Diese Variante des Einbringens eines filtrierten Stromes ist beispielsweise auch sinnvoll, um einen unerwünschten Einfluss des filtrierten Stromes auf die Lebensmittel bzw. Behältnisse in der Behandlungszone zu vermeiden. Dies kann zum Beispiel aufgrund eines nicht geeigneten Temperaturniveaus des filtrierten Stromes der Prozessflüssigkeit gegeben sein.

Es kann aber auch zweckmäßig sein, einen filtrierten Strom der Prozessflüssigkeit zumindest teilweise einem durch eine Behandlungszone bewegten Flüssigkeitsstrom der Prozessflüssigkeit vor dessen Einwirken auf die Lebensmittel bzw. die Behältnisse zuzuführen. Insbesondere kann so für die Behandlung der Lebensmittel bzw. Behältnisse in einer Behandlungszone eine Prozessflüssigkeit mit sehr hoher Qualität bzw. Reinheit und mit sehr geringer Keimzahl bereitgestellt werden.

Besonders vorteilhaft kann ein zumindest teilweises Zuführen eines filtrierten Stromes der Prozessflüssigkeit einer im Verfahren zur Behandlung von Lebensmitteln in Behältnissen zur Aufnahme der Produkte am Ende des Prozesses angeordneten Behandlungszone, zum Spülen bzw. Reinigen der Außenseite von mit dem Lebensmittelprodukt befüllten und verschlossenen Behältnissen sein. Ein solcher Verfahrensschritt wird für gewöhnlich gegen Ende eines Verfahrens zur Behandlung von Lebensmitteln bzw. Behältnissen ausgeführt, nachfolgend erfolgt allenfalls noch ein Trocknungsschritt oder sonstiger Nachbehandlungsschritt. Bei einem solchen Verfahrensschritt zum Spülen bzw. Waschen von Behältnissen ist eine Verschmutzung und/oder Verkeimung der Prozessflüssigkeit besonders kritisch, da unter Umständen Schmutzreste, wie etwa auch Bakterien oder Bakterienreste auf der Oberfläche der Behältnisse verbleiben können. Daher ist die Zuführung eines mittels einer Membranfiltrationsanlage filtrierten bzw. gereinigten Stromes in eine solche Behandlungszone zum Spülen von Behältnissen vorteilhaft.

Es kann aber genauso zweckmäßig sein, wenn ein filtrierter Strom der Prozessflüssigkeit zumindest teilweise einer im Verfahren zur Behandlung von Lebensmitteln in Behältnissen zur Aufnahme der Lebensmittel eingangs angeordneten Behandlungszone zum Reinigen bzw. Spülen der Innenseite und der Außenseite von ungefüllten Behältnissen zugeführt wird. Auch hierbei kann eine Prozessflüssigkeit mit hoher Reinheit zum Behandeln der Behältnisse eingesetzt werden.

Eine weitere zweckmäßige Verfahrensmaßnahme kann dadurch erreicht werden, dass ein Strom der Prozessflüssigkeit nach Filtration mittels der Membranfiltrationsanlage in einen Vorlagebehälter geführt wird, und via einem am Vorlagebehälter ausgestalteten Überlauf oder wirkungsäquivalenten Element wieder in zumindest ein die Prozessflüssigkeit beinhaltendes bzw. führendes Element und/oder in eine Behandlungszone zurückgeführt wird. Insbesondere wird so ein filtrierter Vorrat an Prozessflüssigkeit gesammelt bzw. bereitgestellt, welcher für verschiedene Zwecke eingesetzt werden kann.

Beispielsweise ist eine zweckmäßige Nutzung des in einem Vorlagebehälter gesammelten Filtrats der Prozessflüssigkeit in einer Ausführungsvariante des Verfahrens dadurch erreichbar, dass in festlegbaren Zeitintervallen eine Membranfiltrationsanlage zwecks Reinigung der Filtermembranen im laufenden Betrieb vom Rest der Vorrichtung zur Behandlung von Lebensmitteln in Behältnissen strömungstechnisch getrennt wird und das in dem Vorlagebehälter gesammelte Filtrat der Prozessflüssigkeit unter Umkehrung der Flussrichtung über die Filtermembranen im Vergleich zum Filtrationsbetrieb, durch eine Membranfiltrationsanlage geführt wird, die Membranfiltrationsanlage also mittels des Filtrats unter Rückspülung gereinigt wird. Im Zuge der kontinuierlichen Filtration von Teilströmen der Prozessflüssigkeit bilden sich mit der Zeit naturgemäß Rückstände an den Filtermembranen bzw. Modulen aus. Allenfalls können besonders kleine Partikel auch in eine Filtermembran bzw. einen Porenkanal einer Filtermembran eindringen und dort verbleiben. Im Falle von eingedrungenen Mikroorganismen kann es aufgrund eines Wachstums, zum Beispiel von Bakterienkolonien mit der Zeit auch zu einer Verkeimung einer Filtermembran kommen. Ein derartiger Vorgang wird allgemein auch als 'Fouling' oder 'Bio-Fouling' bezeichnet. Insgesamt führen Beläge auf der Membranoberfläche und/oder in eine Membran eingedrungene und verbleibende Partikel oder Substanzen zu Verstopfungen auf und in einer Membran, und damit zu einem geringer werdenden Durchflussvermögen und zu einer Verringerung der Filtrationsleistung einer Filtermembran. Durch die angegebene, periodische Reinigung der Filtermembran-Module mittels des gesammelten Filtrats der Prozessflüssigkeit unter Umkehrung der Flussrichtung können Verstopfungen bzw. das Zuwachsen von Poren einer Membran möglichst hintangehalten werden. Dabei kann die Rückspülung durch Gaseintrag, beispielsweise durch Eintrag von Druckluft in ein Filtermembranmodul zusätzlich unterstützt werden. Weiters können etwaig notwendige Entkeimungen einer Filtermembran, wie beispielsweise durch Chlor, oder sogar ein kostspieliger Austausch von Filtermembranen oder Membranmodulen zumindest möglichst lange hinausgezögert werden. Schließlich ist auch ein Einbringen von antimikrobiell wirkenden Silber-Nanopartikeln in die Filtermembranen zwecks Verhinderung einer Verkeimung einer Filtermembran vermeidbar, bzw. kann der Einsatz von Silber-Nanopartikeln zumindest deutlich reduziert werden.

Im Zuge einer Reinigung unter Umkehrung der Flussrichtung über die Filtermembranen der Membranfilteranlage fällt ein verunreinigter Flüssigkeitsabfall an. Dabei kann es sinnvoll sein, diesen Flüssigkeitsabfall unmittelbar aus der Vorrichtung zur Behandlung von Lebensmitteln bzw. Behältnissen abzuführen und durch eine entsprechende Menge an frischer Prozessflüssigkeit zu ersetzen.

Weiters kann es von Vorteil sein, bei Bedarf sowohl im Behandlungsbetrieb bzw. im Filtrationsbetrieb, als auch im Reinigungsbetrieb für die Membranfiltrationsanlage, einem zu filtrierenden Strom der Prozessflüssigkeit bzw. einem filtrierten Strom oder Filtrat der Prozessflüssigkeit mittels einer Dosiereinrichtung Chemikalien aus einer oder mehreren Chemikalienquellen beizumengen. Bei geeigneter Anordnung der Dosiereinrichtung, zum Beispiel in einem Ableitungselement für einen filtrierten Strom der Prozessflüssigkeit oder einer dem Ableitungselement zugeordneten Bypass- bzw. Rückspülleitung, kann ein Beimengen aus denselben Chemikalienquellen sowohl im Behandlungsbetrieb, als auch im Reinigungsbetrieb für die Membranfilteranlage erfolgen. Die Art und Menge einzusetzender Chemikalien richtet sich dabei nach dem jeweiligen Bedarf, und die Zuführung von Chemikalien kann von einem Bediener der Vorrichtung, bzw. einer automatischen Steuervorrichtung für die Vorrichtung, bedarfsabhängig durchgeführt werden. Dadurch, dass die Zufuhr von Chemikalien sowohl im Filtrationsbetrieb, als auch im Reinigungsbetrieb ermöglicht ist, kann ein flexibles, gezieltes Zudosieren von Chemikalien aus denselben Chemikalienquellen bedarfsabhängig durchgeführt werden. Beispiele für Chemikalien, welche sich in beiden Fällen eignen sind Tenside zu generellen Reinigung, oder Chlor zur Desinfektion der Filtermembranen oder anderen Elementen der Vorrichtung zur Behandlung von Lebensmitteln bzw. Behältnissen. Weitere, üblicherweise eingesetzte Chemikalien umfassen zum Beispiel organische Säuren zur pH-Wert-Stabilisierung, Komplexbildner, Korrosionsinhibitoren und Biozide.

Außerdem kann es zweckmäßig sein, im Filtrationsbetrieb nachfolgend auf die Membranfiltration einen filtrierten Strom zum Entfernen bzw. Abscheiden gelöster bzw. suspendierter oder dispergierter Substanzen über eine Adsorptionsvorrichtung zu führen. Insbesondere können auf diese Weise auch unerwünschte, nicht koagulierte Bestandteile aus der Prozessflüssigkeit entfernt werden, welche durch die Membranfiltrationsanlage nicht entfernbar sind.

Für möglichst hohe Prozesssicherheit kann es vorteilhaft sein, die Qualität bzw. Reinheit der Prozessflüssigkeit mittels geeigneter Sensoren zumindest in den Zu- und/oder Ableitungen einer Behandlungszone und/oder in einer Behandlungszone kontinuierlich zu überwachen. Insbesondere können Messungen der Trübung zum Zwecke des Erfassens der Reinheit der Prozessflüssigkeit bzw. der Partikelkonzentration in der Prozessflüssigkeit dienlich sein. Alternativ und/oder zusätzlich sind auch Messungen an Stichproben der Prozessflüssigkeit sinnvoll, besonders um Schwankungen im Gehalt an Bakterienkulturen zu erfassen bzw. Mikroorganismenwachstumsraten zu ermitteln.

Ein weiterer Vorteil ergibt sich durch die Ausgestaltungsvariante, dass ein zu filtrierender Strom der Prozessflüssigkeit bedarfsabhängig aus unterschiedlichen, die Prozessflüssigkeit beinhaltenden bzw. führenden Führungselementen gebildet wird, mittels zumindest einer Membranfiltrationsanlage filtriert wird und ein filtrierter Strom nach dem Membranfiltrationsvorgang in zumindest ein die Prozessflüssigkeit beinhaltendes bzw. führendes Führungselement und/oder in zumindest eine Behandlungszone zurückgeführt wird. Insbesondere kann es vorteilhaft sein, eine Membranfiltrationsanlage mittels strömungstechnischen Schalt- und/oder Verteilerelementen derart in die Vorrichtung zur Behandlung von Produkten und/oder Behältnissen einzubinden, dass eine Membranfiltrationsanlage verschiedenen und/oder mehreren verschiedenen Führungselementen zur Beinhaltung bzw. Führung der Prozessflüssigkeit und/oder Behandlungszonen zugeordnet werden kann. Durch die Möglichkeit des eingangsseitigen Umschaltens einer Membranfiltrationsanlage auf verschiedene Quellen der Prozessflüssigkeit zur Bildung eines zu filtrierenden Stromes kann in flexibler Art und Weise auf zonenweise Schwankungen in der Qualität bzw. dem Verunreinigungsgrad der Prozessflüssigkeit, insbesondere auf Schwankungen in der Partikelkonzentration, rasch und effizient reagiert werden. Dabei besteht grundsätzlich die Möglichkeit von einer Prozessflüssigkeitsquelle auf eine andere Prozessflüssigkeitsquelle umzuschalten, also verschiedene Flüssigkeitsströme in verschiedenen Führungselementen zur Bildung des zu filtrierenden Stromes heranzuziehen. Wahlweise können aber auch mehrere Flüssigkeitsströme der Prozessflüssigkeit gleichzeitig zur Bildung eines zu filtrierenden Stromes der Prozessflüssigkeit herangezogen werden. Im diesem Fall erfolgt die Bildung des zu filtrierenden Stromes durch Mischen der herangezogenen bzw. entnommenen Teilmengen aus den verschiedenen Flüssigkeitsströmen der Prozessflüssigkeit.

Weiters kann eine Ausführungsvariante zweckmäßig sein, bei welcher Strom der Prozessflüssigkeit zur Filtration mittels einer Membranfiltrationsanlage, durch Umschalten zwischen oder Mischen von unterschiedlichen Flüssigkeitsströmen der Prozessflüssigkeit aus verschiedenen Führungselementen, in Abhängigkeit von im Zuge von Inline-Messungen und/oder Stichprobenmessungen erhaltenen Messwerten gebildet wird. Ebenso kann es von Vorteil sein, einen filtrierten Strom der Prozessflüssigkeit in Abhängigkeit von im Zuge von Inline-Messungen und/oder Stichprobenmessungen erhaltenen Messwerten, durch Einleiten oder Verteilen des filtrierten Stromes der Prozessflüssigkeit in verschiedene Flüssigkeitsströme der Prozessflüssigkeit in verschiedenen Führungselementen bzw. Behandlungszonen zurückzuführen.

Schließlich kann eine Ausführungsvariante zweckmäßig sein, bei welcher einer Behandlungszone mehr als eine Membranfiltrationsanlage zugeordnet wird, und den einer jeweiligen Behandlungszone zugeführten Flüssigkeitsströmen der Prozessflüssigkeit bedarfsabhängig eine bestimmte Menge zur Bildung eines zu filtrierenden Stromes der Prozessflüssigkeit entnommen wird und ein zu filtrierender Strom mittels einer oder mehrerer jeweilig einer Behandlungszone zugeordneten Membranfiltrationsanlage(n) gefiltert wird. Durch diese Maßnahme kann der Filtrationsbetrieb auch dann aufrecht erhalten werden, wenn zwecks einer allenfalls nötigen Reparatur und/oder Wartung eine der Membranfiltrationsanlagen von der Vorrichtung zur Behandlung von Produkten und/oder Behältnissen abgetrennt werden muss.

Die Aufgabe der Erfindung wird aber auch durch die im unabhängigen Anspruch 20 definierte Vorrichtung gelöst.

Die Vorrichtung umfasst zumindest eine Behandlungszone zum Behandeln der Lebensmittel in Behältnissen, Transportmittel zum Transportieren der Lebensmittel in Behältnissen durch die Behandlungszone(n), sowie Führungselemente zum Zuführen von Flüssigkeitsströmen der Prozessflüssigkeit in eine Behandlungszone und Führungselemente zum Abführen von Flüssigkeitsströmen der Prozessflüssigkeit aus einer Behandlungszone. Weiters umfasst die Vorrichtung weitere Führungselemente zur Beinhaltung und/oder Führung der Prozessflüssigkeit in der Vorrichtung und wenigstens ein Fördermittel zur Förderung von Flüssigkeitsströmen der Prozessflüssigkeit in den Führungselementen, wobei die Führungselemente derart ausgestaltet und angeordnet sind, dass die Prozessflüssigkeit zur Behandlung der Lebensmittel im Kreis wieder in die Behandlungszone oder in die Behandlungszonen geführt werden kann.

Insbesondere ist in der Vorrichtung zumindest eine Membranfiltrationsanlage angeordnet, welche derart in der Vorrichtung angeordnet und derart mit den Führungselementen und/oder mit den Behandlungszonen strömungstechnisch leitungsverbunden ist, dass zumindest eine Teilmenge oder die gesamte Menge der pro Zeiteinheit insgesamt durch alle vorhandenen Behandlungszonen geführten Prozessflüssigkeit zur Bildung wenigstens eines Stromes der Prozessflüssigkeit herangezogen werden kann, der gebildete Strom oder die gebildeten Ströme mittels der zumindest einen Membranfiltrationsanlage filtriert werden kann und ein filtrierter Strom der Prozessflüssigkeit zumindest teilweise einem Führungselement und/oder einer Behandlungszone wieder zugeführt werden kann.

Durch diese Merkmale kann eine Vorrichtung bereitgestellt werden, welche sich besonders für die Entfernung von Verschmutzungen bzw. Kontaminationen mit sehr kleiner Partikelgröße, wie etwa Mikroorganismenkolonien, aus der Prozessflüssigkeit hervorragend eignet. Damit lässt sich eine wesentliche Verbesserung gegenüber bekannten Vorrichtungen erreichen, welche hinsichtlich einer Entfernung kleiner Partikel aus einer zumindest teilweise im Kreis geführten Prozessflüssigkeit nur sehr eingeschränkt, oder gar nicht wirksam sind. Außerdem kann mit einer derartigen Vorrichtung eine Verkeimung der durch alle vorhandenen Behandlungszonen geführten Prozessflüssigkeit wirksam hintangehalten werden. Die Mikro- bzw. Ultrafiltration via die zumindest eine Membranfiltrationsanlage kann dabei vorteilhafterweise im laufenden Behandlungsbetrieb durchgeführt werden und ist auch vergleichsweise effizient und energiesparend.

Das Merkmal, dass wenigstens eine Behandlungszone zur Applikation der Prozessflüssigkeit auf die Außenseite von verschlossenen Behältnissen ausgestaltet ist, wobei die Prozessflüssigkeit die Außenseite eines verschlossenen Behältnisses umströmt, stellt ein besonders effizientes bauliches Merkmal für die Behandlung von Lebensmitteln dar, da nach der Behandlung ein bereits fertig abgefülltes Handelsprodukt bereitgestellt werden kann. Außerdem kann so die Gefahr einer Rekontamination der Lebensmittel in einer an die Behandlungszone(n) anschließenden Abfüllzone ausgeschlossen werden, bzw. ist eine den Behandlungszonen nachgeschaltete Abfüllzone erübrigt.

Durch das Anordnen bzw. Ausgestalten von zumindest einem Heizmittel in der Vorrichtung kann die Prozessflüssigkeit erwärmt werden. Dadurch können in weiterer Folge mittels der erwärmten Prozessflüssigkeit die Lebensmittel in Behältnissen gezielt erwärmt werden. Weiters kann dadurch zumindest für eine oder mehrere Behandlungszonen vor der Zuführung der Prozessflüssigkeit in die entsprechende Behandlungszone die Temperatur der Prozessflüssigkeit auf ein zur Pasteurisation der Lebensmittel geeignetes Temperaturniveau eingestellt werden.

Durch das Anordnen bzw. Ausgestalten von zumindest einem Kühlmittel in der Vorrichtung kann die Prozessflüssigkeit abgekühlt werden. Dadurch können in weiterer Folge mittels der abgekühlten Prozessflüssigkeit die Lebensmittel bzw. die Behältnisse gezielt gekühlt werden. Außerdem kann es von Vorteil sein, in der Vorrichtung aufeinanderfolgend zumindest eine Behandlungszone zur Erwärmung der Lebensmittel und/oder Behältnisse, zumindest eine Behandlungszone zur Pasteurisation der Lebensmittel und zumindest eine Behandlungszone zur Abkühlung der Lebensmittel und/oder Behältnisse anzuordnen. Durch diese Ausgestaltungsform kann eine Vorrichtung zur Behandlung von Lebensmitteln bzw. Behältnissen bereitgestellt werden, mittels welcher die Lebensmittel besonders schonend pasteurisiert werden können und eine Schädigung des pasteurisierten Lebensmittels wirksam unterbunden werden kann.

Weiters kann es zweckmäßig sein, die Vorrichtung derart auszugestalten, dass die Anzahl und die Filtrationskapazität der Membranfiltrationsanlage(n) derart festgelegt sind, dass die im kontinuierlichen Behandlungsbetrieb aus zumindest einem die Prozessflüssigkeit beinhaltenden und/oder führenden Element pro Zeiteinheit insgesamt zur Bildung wenigstens eines zu filtrierenden Stromes der Prozessflüssigkeit herangezogene Prozessflüssigkeitsmenge so gewählt werden kann, dass durch die Filtration des Stromes oder der Ströme eine Entfernungsrate für Mikroorganismen erzielbar ist, welche größer ist, als die Wachstumsrate dieser Mikroorganismen in der Prozessflüssigkeit im gleichen Zeitintervall. Durch dieses Merkmal ist eine Vorrichtung geschaffen, in welcher durch die Membranfiltrationsanlage(n) die Gesamtmenge an Mikroorganismen in der Prozessflüssigkeit möglichst niedrig gehalten werden kann und eine Zunahme der Gesamtmenge an Mikroorganismen in der Prozessflüssigkeit im Zuge der kontinuierlichen Behandlung der Lebensmittel und/oder Behältnisse wirksam unterbunden ist.

Außerdem ist eine Ausgestaltungsform der Vorrichtung sinnvoll, bei welcher die Anzahl und die Filtrationskapazität der Membranfiltrationsanlage(n) derart festgelegt sind, dass bezogen auf die pro Zeiteinheit insgesamt durch alle vorhandenen Behandlungszonen geführte Menge an Prozessflüssigkeit, pro Zeiteinheit zumindest 1 % und weniger als 25 % zur Bildung wenigstens eines Stromes herangezogen werden können, und diese pro Zeiteinheit gebildete Teilmenge der Prozessflüssigkeit mittels der Membranfiltrationsanlage(n) filtriert werden kann. Dadurch kann einerseits gewährleistet werden, dass im laufenden Behandlungsbetrieb eine ausreichend hohe Anzahl an Verunreinigungen, insbesondere Kleinpartikel, aus der Prozessflüssigkeit entfernt wird. Andererseits kann so auch eine allzu aufwendige bauliche Ausgestaltung einer Membranfiltrationsanlage bzw. der Einsatz einer großen Anzahl an Membranfiltrationsanlage(n) vermieden werden. Bevorzugt wird die Anzahl und die gesamte Filtrationskapazität der Membranfiltrationsanlagen derart festgelegt, dass - bezogen auf die pro Zeiteinheit insgesamt durch alle vorhandenen Behandlungszonen geführte Menge an Prozessflüssigkeit - pro Zeiteinheit zwischen 2 % und 10 % und insbesondere zwischen 2,5 und 7 % zur Bildung wenigstens eines zu filtrierenden Stromes der Prozessflüssigkeit herangezogen werden und diese pro Zeiteinheit gebildete Teilmenge der Prozessflüssigkeit mittels zumindest einer Membranfiltrationsanlage filtriert wird.

Zusätzlich kann es zweckmäßig sein, die Anzahl und die Filtrationskapazität der Membranfiltrationsanlage(n) derart festzulegen, dass das insgesamt in der Vorrichtung zur Behandlung von Lebensmitteln in Behältnissen enthaltene Volumen an Prozessflüssigkeit pro Tag zumindest 1 Mal und bevorzugt zwischen 2 Mal und 10 Mal mittels der Membranfiltrationsanlage(n) filtriert werden kann. Durch diese Ausgestaltungsform der Vorrichtung für die Behandlung von Lebensmitteln bzw. Behältnissen kann sichergestellt werden, dass in Abhängigkeit von verschiedenen Verfahrensparametern, beispielsweise von der Eingangsqualität der Prozessflüssigkeit, von der im Behandlungsverfahren selbst vorhandenen Verschmutzungsrate und insbesondere von der Mikroorganismenwachstumsrate in der Prozessflüssigkeit, eine ausreichende Menge der Prozessflüssigkeit mittels der Membranfiltrationsanlage(n) gereinigt werden kann.

Außerdem kann es vorteilhaft sein, die zumindest eine Membranfiltrationsanlage für die Bildung eines zu filtrierenden Stromes der Prozessflüssigkeit an solchen Stellen mit Führungselementen strömungstechnisch bzw. leitungstechnisch zu verbinden, an welchen die in den Führungselementen beinhaltete bzw. geführte Prozessflüssigkeit eine Temperatur von weniger als 80 °C und insbesondere weniger als 50 °C aufweist. Dadurch kann die Betriebsfähigkeit bzw. Lebensdauer einer Membranfiltrationsanlage wesentlich verlängert werden, insbesondere dann, wenn die Filterelemente bzw. Filtermembranen aus Kunststoff bestehen. Bei Verwendung von anderen Materialien für die Filterelemente bzw. Filtermembranen, zum Beispiel bei Verwendung keramischer Materialien, kann die Prozessflüssigkeit auch höhere Temperaturen, beispielsweise 100°C oder mehr aufweisen. Derartige Temperaturen sind zum Beispiel bei einer Heißdampfsterilisation durchaus üblich.

Bei einer Weiterbildung kann auch vorgesehen sein, dass ein Zuleitungselement einer Membranfiltrationsanlage mit einem temperierbaren Durchflussbehälter für die Prozessflüssigkeit leitungsverbunden ist. So kann die Temperatur eines zu filtrierenden Stromes der Prozessflüssigkeit mittels des temperierbaren Durchflussbehälter gezielt eingestellt werden, und damit die Filtrationseffizienz optimiert werden.

Von Vorteil kann weiters eine Ausgestaltungsform der Vorrichtung sein, bei welcher zur Rückführung eines filtrierten Stromes der Prozessflüssigkeit nach erfolgter Filtration Ableitungselemente von zumindest einer Membranfiltrationsanlage mit zumindest einem Führungselement und/oder zumindest einer Behandlungszone derart leitungsverbunden sind, dass der zumindest eine filtrierte Strom der Prozessflüssigkeit dem oder den Führungselement(en) und/oder der oder den Behandlungszone(n) unter Einwirkung von Schwerkraft in freiem Gefälle zugeführt werden kann. Dadurch kann erreicht werden, dass ein zusätzliches Fördermittel zum Einbringen bzw. Zurückführen eines filtrierten Stromes der Prozessflüssigkeit erübrigt ist, was eine baulich einfach zu realisierende Variante und effiziente Variante der Vorrichtung darstellt.

Dabei kann beispielsweise zumindest eine Membranfiltrationsanlage ausgangseitig mit einer Öffnung in einer Behandlungszone strömungstechnisch leitungsverbunden sein, sodass ein filtrierter Strom der Prozessflüssigkeit in die Behandlungszone abfließen kann. Die Öffnung in einer Behandlungszone kann dabei zum Beispiel an einem oberen Ende der Behandlungszone angeordnet sein, sodass ein filtrierter Strom der Prozessflüssigkeit zumindest teilweise einem durch eine Behandlungszone bewegten Flüssigkeitsstrom der Prozessflüssigkeit vor dessen Einwirken auf die Lebensmittel bzw. die Behältnisse zuführbar ist. Andererseits kann es aber auch zweckmäßig sein, die Öffnung an einem unteren Ende der Behandlungszone anzuordnen, um einen unerwünschten, beispielsweise thermischen, Einfluss des filtrierten Stromes auf die Lebensmittel bzw. Behältnisse in der Behandlungszone zu vermeiden.

Eine weitere vorteilhafte Ausgestaltungsform der Vorrichtung kann dadurch bereitgestellt werden, dass zumindest eine Behandlungszone zur Spülung der Außenseite von mit Lebensmitteln befüllten und verschlossenen Behältnissen ausgestaltet ist, welche zumindest eine Behandlungszone, bezogen auf die Transportrichtung der Behältnisse durch die Behandlungszonen, am Ende der Behandlungszonenstrecke angeordnet ist und welche zur Spülung der Behältnisse durch Zuführung eines filtrierten Stromes der Prozessflüssigkeit mit zumindest einem Ableitungselement einer Membranfiltrationsanlage leitungsverbunden ist. Dadurch ist eine Behandlungszone zur finalen Reinigung der Außenseite der Behältnisse mittels eines filtrierten Stromes der Prozessflüssigkeit bereitgestellt, in welcher die Außenseite der Behältnisse mittels einer Prozessflüssigkeit mit sehr hoher Reinheit und mit geringem Keimgehalt gespült werden kann.

Es kann aber auch zweckmäßig sein, in der Vorrichtung zur Behandlung der Lebensmittel bzw. Behältnisse zumindest eine Behandlungszone zur Reinigung der Innenseite und der Außenseite von ungefüllten und unverschlossenen Behältnissen auszubilden, welche zumindest eine Behandlungszone, bezogen auf die Transportrichtung der Behältnisse durch die Behandlungszonen eingangsseitig der Behandlungszonenstrecke angeordnet ist und welche zur Reinigung der Behältnisse durch Zuführung eines filtrierten Stromes der Prozessflüssigkeit mit zumindest einem Ableitungselement einer Membranfiltrationsanlage leitungsverbunden ist. Durch diese Ausgestaltungsvariante der Vorrichtung ist eine Behandlungszone geschaffen, in welcher eine Behandlung der Behältnisse mittels einer Prozessflüssigkeit mit hoher Reinheit erfolgen kann.

Von Vorteil kann auch eine Ausgestaltungsvariante sein, bei welcher in einem Ableitungselement der zumindest einen Membranfiltrationsanlage ein Vorlagebehälter mit Überlauf ausgestaltet ist. Dadurch kann ein filtrierter Vorrat an Prozessflüssigkeit gesammelt bzw. bereitgestellt werden, welcher für verschiedene Zwecke eingesetzt werden kann.

Zum Zwecke einer Reinigung einer Membranfiltrationsanlage kann es beispielsweise zweckmäßig sein, Absperrmittel in den Zuleitungselementen und den Ableitungselementen zum strömungstechnischen Abtrennen der zumindest einen Membranfiltrationsanlage vom Rest der Vorrichtung anzuordnen und in dem Vorlagebehälter und/oder einer sich zwischen dem Vorlagebehälter und dem Ableitungselement der Membranfiltrationsanlage erstreckenden Rückspülleitung zumindest ein Fördermittel anzuordnen, welches zur Förderung des in dem Vorlagebehälter gesammelten Filtrats der Prozessflüssigkeit in - verglichen mit der Flussrichtung über die Filtermembranen im Filtrationsbetrieb - gegenläufiger Richtung durch die zumindest eine Membranfiltrationsanlage ausgebildet ist. Dadurch kann im laufenden Behandlungsbetrieb der Vorrichtung zur Behandlung der Lebensmittel bzw. Behältnisse eine Membranfiltrationsanlage mittels des Filtrats unter Rückspülung gereinigt werden. So können zum Beispiel Verstopfungen in einer Membran bzw. das Zuwachsen von Poren einer Membran möglichst hintangehalten werden, ohne dass eine Unterbrechung des Behandlungsbetriebs der Vorrichtung nötig ist.

Während eines Rückspülens/Reinigens kann jedoch auch eine Filtrierung durchgeführt werden, falls mehrere Anlagen gemeinsam/parallel betrieben werden.

Dabei kann es weiters zweckmäßig sein, zur Abführung des im Zuge der Reinigung unter Umkehrung der Flussrichtung über die Filtermembranen der Membranfiltrationsanlage anfallenden Flüssigkeitsabfalls, der Membranfiltrationsanlage zumindest eine absperrbare Flüssigkeitsabfallleitung zuzuordnen und in der Vorrichtung zumindest eine absperrbare Zufuhrvorrichtung zum Ersetzen des abgeführten Flüssigkeitsabfalls durch frische Prozessflüssigkeit anzuordnen. Dadurch ist es möglich, den Flüssigkeitsabfall unmittelbar aus der Vorrichtung zur Behandlung von Lebensmitteln bzw. Behältnissen abzuführen und durch eine entsprechende Menge an frischer Prozessflüssigkeit zu ersetzen.

Außerdem kann es sinnvoll sein, dass in einem Ableitungselement und/oder in der Rückspülleitung der zumindest einen Membranfiltrationsanlage eine Dosiereinrichtung angeordnet ist, mittels welcher der Prozessflüssigkeit bzw. einem Filtrat der Prozessflüssigkeit sowohl im Filtrationsbetrieb, als auch im Reinigungsbetrieb für die Membranfiltrationsanlage Chemikalien aus einer oder mehreren Chemikalienquellen beigemengt werden können. Bei Anordnung einer mit der oder den Chemikalienquelle(n) leitungsverbundenen Dosiereinrichtung können so der Prozessflüssigkeit bedarfsabhängig Chemikalien, wie zum Beispiel Chlor, Biozide, Tenside und andere wirksame Chemikalien sowohl im Filtrationsbetrieb, als auch im Reinigungsbetrieb einer Membranfiltrationsanlage beigemengt werden.

Zusätzlich kann eine Ausgestaltungsform vorteilhaft sein, bei welcher in einem Ableitungselement zum Ableiten eines filtrierten Stromes der Prozessflüssigkeit aus einer Membranfiltrationsanlage eine Adsorptionsvorrichtung angeordnet ist. So können unerwünschte, nicht koagulierte Bestandteile aus der Prozessflüssigkeit entfernt werden, welche durch die Membranfiltrationsanlage nicht entfernbar sind. Beispielsweise können Kohlenstoffverbindungen mittels Aktivkohle in einer derartigen Adsorptionsvorrichtung aus der Prozessflüssigkeit entfernt werden.

Im Hinblick auf verbesserte Prozesssicherheit kann es sinnvoll sein, dass in Führungselementen und/oder in Behandlungszonen Sensoren zur kontinuierlichen Überwachung des Verunreinigungsgrades, insbesondere durch Messen der Trübung der Prozessflüssigkeit, angeordnet sind. So kann der Verschmutzungsgrad der Prozessflüssigkeit zumindest abschnittsweise erfasst und kontinuierlich überwacht werden.

Eine weitere, vorteilhafte Ausgestaltungsform kann dadurch gebildet sein, dass einem Zuleitungselement einer Membranfiltrationsanlage zumindest ein Umschaltmittel zugeordnet ist, welches mit wenigstens zwei verschiedenen die Prozessflüssigkeit beinhaltenden Führungselementen strömungstechnisch derart leitungsverbunden ist, dass eine Bildung des zu filtrierenden Stromes der Prozessflüssigkeit wahlweise aus einem der Flüssigkeitsströme oder mehreren Flüssigkeitsströmen der Prozessflüssigkeit in den Führungselementen oder aus mehreren Flüssigkeitsströmen der Prozessflüssigkeit erfolgen kann. Dadurch ist ein eingangsseitiges Umschalten einer Membranfiltrationsanlage auf verschiedene Quellen der Prozessflüssigkeit zur Bildung eines zu filtrierenden Stromes ermöglicht. So kann beispielsweise in flexibler Art und Weise auf zonenweise Schwankungen der Qualität bzw. des Verunreinigungsgrades der Prozessflüssigkeit, insbesondere auf Schwankungen der Partikelkonzentration, rasch und effizient reagiert werden.

Weiters kann eine Ausgestaltungsform von Vorteil sein, bei welcher einem Zuleitungselement einer Membranfiltrationsanlage zumindest ein Mischmittel zugeordnet ist, welches mit wenigstens zwei verschiedenen die Prozessflüssigkeit beinhaltenden Führungselementen strömungstechnisch derart leitungsverbunden ist, dass eine Bildung eines zu filtrierenden Stromes der Prozessflüssigkeit wahlweise aus einem der Flüssigkeitsströme oder mehreren Flüssigkeitsströmen der Prozessflüssigkeit in den Führungselementen erfolgen kann, oder die Bildung eines zu filtrierenden Stromes der Prozessflüssigkeit durch die Membranfiltrationsanlage durch Entnahme und Mischung festlegbarer Teilmengen aus mehreren Flüssigkeitsströmen der Prozessflüssigkeit erfolgen kann. Dadurch ist eine gleichzeitige Entnahme von Teilmengen an Prozessflüssigkeit aus verschiedenen Führungselementen und die Bildung eines zu filtrierenden Stromes der Prozessflüssigkeit durch Mischen der entnommenen Teilmengen an Prozessflüssigkeit ermöglicht.

Es kann aber auch zweckmäßig sein, einem Ableitungselement einer Membranfiltrationsanlage zumindest ein Umschaltelement zuzuordnen, welches mit wenigstens einem die Prozessflüssigkeit beinhaltenden Führungselement und/oder wenigstens einer Behandlungszone strömungstechnisch derart leitungsverbunden ist, dass die Zuführung eines filtrierten Stromes der Prozessflüssigkeit in das wenigstens eine Führungselement und/oder die wenigstens eine Behandlungszone kontrolliert festlegbar ist. Auf diese Weise kann ein filtrierter Strom der Prozessflüssigkeit bedarfsabhängig einem oder mehreren Führungselement(en) und/oder einer oder mehreren Behandlungszone(n) zugeführt werden. Dies kann zum Beispiel zweckmäßig sein, um abschnittsweise gezielte Temperaturen für die Prozessflüssigkeit in der Vorrichtung zur Behandlung der Lebensmittel bzw. Behältnisse einzustellen. Generell kann durch diese Ausgestaltungsform eine Vorrichtung bereitgestellt werden, bei welcher ein filtrierter Strom der Prozessflüssigkeit bedarfsabhängig in verschiedene Führungselemente bzw. Behandlungszonen geleitet werden kann.

Von Vorteil kann aber auch eine Ausgestaltungsvariante sein, bei welcher einem Ableitungselement einer Membranfiltrationsanlage zumindest ein Verteilerelement zugeordnet ist, welches mit wenigstens einem die Prozessflüssigkeit beinhaltenden Führungselement und/oder wenigstens einer Behandlungszone strömungstechnisch derart leitungsverbunden ist, dass eine Zuführung eines filtrierten Stromes der Prozessflüssigkeit in das wenigstens eine Führungselement und/oder die wenigstens eine Behandlungszone kontrolliert festlegbar ist, oder festlegbare Mengen des filtrierten Stromes der Prozessflüssigkeit in das wenigstens eine Führungselement und/oder die wenigstens eine Behandlungszone zuführbar sind. Durch dieses Merkmal kann eine Vorrichtung bereitgestellt werden, bei welcher die gezielte Zuführung bestimmter Teilmengen eines filtrierten Stromes der Prozessflüssigkeit in verschiedene Führungselemente und/oder Behandlungszonen in kontrollierter Art und Weise ermöglicht ist.

Zum besseren Verständnis der Erfindung wird diese anhand der nachfolgenden Figuren näher erläutert. Die Erfindung wird durch die Ansprüche definiert.

Es zeigen jeweils in stark vereinfachter, schematischer Darstellung:
- Fig. 1: ein Ausführungsbeispiel einer an sich bekannten Vorrichtung zur Behandlung von Lebensmitteln und/oder Behältnissen mit Behandlungszonen, in stark vereinfachter, schematischer und unmaßstäblicher Darstellung;
- Fig. 2: ein beispielhaftes, an sich bekanntes R&I Schema einer Vorrichtung zur Behandlung von Lebensmitteln und/oder Behältnissen, in stark vereinfachter Darstellung;
- Fig. 3: ausschnittsweise ein Teilschema einer Vorrichtung mit Membranfiltrationsanlagen, in stark vereinfachter Darstellung;
- Fig. 4: ausschnittsweise ein weiteres Teilschema einer nicht vom Schutzbereich umfassten Vorrichtung mit Membranfiltrationsanlagen, in stark vereinfachter Darstellung;
- Fig. 5: ausschnittsweise ein weiteres Teilschema einer Vorrichtung mit Membranfiltrationsanlagen, in stark vereinfachter Darstellung;
- Fig. 6: ausschnittsweise ein weiteres Teilschema einer Vorrichtung mit Membranfiltrationsanlagen, in stark vereinfachter Darstellung;
- Fig. 7: ausschnittsweise ein weiteres Teilschema einer Vorrichtung mit Membranfiltrationsanlagen, in stark vereinfachter Darstellung;
- Fig. 8: ausschnittsweise ein weiteres Teilschema einer Vorrichtung mit Membranfiltrationsanlagen, in stark vereinfachter Darstellung;
- Fig. 9: eine Ausgestaltungsform einer Membranfiltrationsanlage, schematisch und in stark vereinfachter Darstellung;
- Fig. 10: ausschnittsweise ein weiteres Teilschema einer Vorrichtung mit Membranfiltrationsanlagen, in stark vereinfachter Darstellung;
- Fig. 11: ausschnittsweise ein weiteres Teilschema einer Vorrichtung mit Membranfiltrationsanlagen, in stark vereinfachter Darstellung.

Einführend sei festgehalten, dass in den unterschiedlich beschriebenen Ausführungsformen gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Auch sind die in der Beschreibung gewählten Lageangaben, wie z.B. oben, unten, seitlich usw. auf die unmittelbar beschriebene sowie dargestellte Figur bezogen und sind diese Lageangaben bei einer Lageänderung sinngemäß auf die neue Lage zu übertragen.

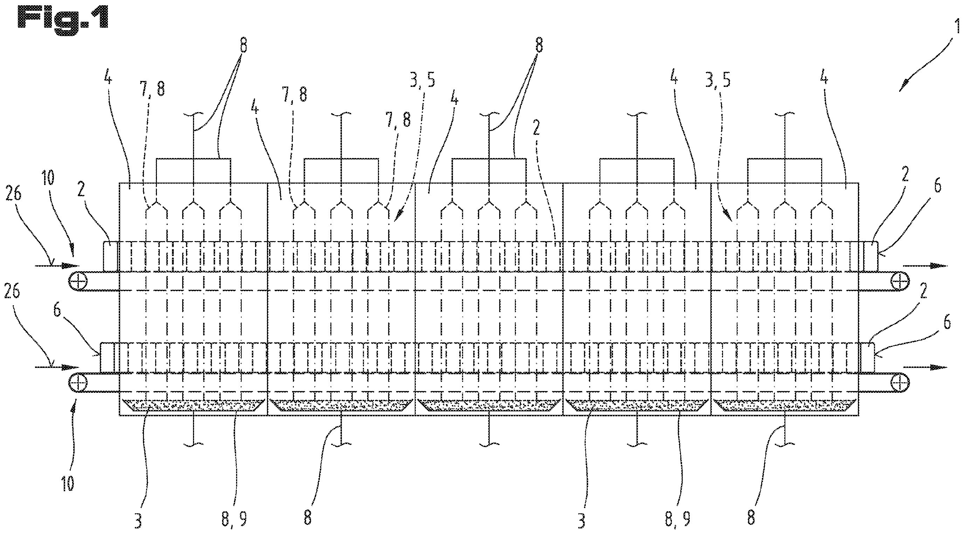

In der Fig. 1 ist ein Beispiel für eine Anordnung von Behandlungszonen einer Vorrichtung 1 zur Behandlung von Lebensmitteln und/oder für die Behandlung von Behältnissen 2 zur Aufnahme von Lebensmitteln, in schematischer und stark vereinfachter Darstellungsweise, gezeigt. Dabei werden die Lebensmittel bzw. die Behältnisse 2 mittels einer Prozessflüssigkeit 3 in zumindest einer Behandlungszone 4 behandelt. Im in Fig. 1 gezeigten Ausführungsbeispiel sind die zu behandelnden Lebensmittel in verschlossenen Behältnissen 2 befindlich, und werden mittels der Prozessflüssigkeit 3 dadurch behandelt, dass die Außenseite 6 der Behältnisse 2 durch einen Flüssigkeitsstrom 5 der Prozessflüssigkeit 3 umströmt wird. Der Flüssigkeitsstrom 5 der Prozessflüssigkeit 3 durch eine Behandlungszone 4 wird im in Fig. 1 dargestellten Ausführungsbeispiel dadurch generiert, dass die Prozessflüssigkeit 3 mittels Verteilungsvorrichtungen, wie zum Beispiel Sprühdüsen 7, an der Oberseite einer Behandlungszone 4 verteilt wird, und der Flüssigkeitsstrom 5 der Prozessflüssigkeit 3 die Behandlungszonen 4 von oben nach unten durchquert. Einer Behandlungszone 4 wird ein Flüssigkeitsstrom 5 der Prozessflüssigkeit 3 mittels baulich geeigneten Führungselementen 8, wie etwa mit den Sprühdüsen 7 leitungsverbundenen Rohrleitungen, zugeführt. In analoger Art und Weise wird ein Flüssigkeitsstrom 5 der Prozessflüssigkeit 3 nach Durchtritt durch eine Behandlungszone 4 mittels weiterer, einem unteren Bereich einer Behandlungszone 4 zugeordneter Führungselemente 8, wieder aus einer Behandlungszone 4 entfernt. Die zur Ableitung eines Flüssigkeitsstromes 5 der Prozessflüssigkeit 3 aus einer Behandlungszone 4 vorgesehenen Führungselemente 8 können beispielsweise durch Sammelwannen 9 gebildet sein, welche den durch eine Behandlungszone 4 rieselenden Flüssigkeitsstrom 5 der Prozessflüssigkeit 3 am unteren Ende der Behandlungszone 4 sammelt. Die mittels der Sammelwannen 8, 9 aufgefangene Prozessflüssigkeit 3 kann dann mittels mit den Sammelwannen 8, 9 leitungsverbundenen Führungselementen 8, bzw. Rohrleitungen 8 aus einer Behandlungszone 4 abgeführt werden, wie dies schematisch in Fig. 1 angedeutet ist.

Die Behältnisse 2 können mittels geeigneter Transportmittel 10, wie zum Beispiel Förderbändern oder dergleichen, durch die Behandlungszonen 4 hindurchtransportiert werden, beispielsweise in zwei Ebenen von links nach rechts, wie in Fig. 1 durch die Pfeile 26 veranschaulicht ist.

Im in Fig. 1 gezeigten Ausführungsbeispiel können die beiden auf der in Fig. 1 linken Seite dargestellten Behandlungszonen 4, beispielsweise zur sukzessiven Erwärmung der Behältnisse 2 bzw. in den Behältnissen befindlichen Lebensmittel herangezogen werden. In weiterer Folge kann die in Fig. 1 mittig dargestellte Behandlungszone 4 beispielsweise zur Pasteurisierung der Lebensmittel benutzt werden und die beiden in Fig. 1 auf der rechten Seite dargestellten Behandlungszonen 4 zur sequentiellen Abkühlung der Lebensmittel bzw. Behältnisse benutzt werden. Die entsprechenden Behandlungsschritte zur Erwärmung, Pasteurisierung und Abkühlung können dabei durch die Zuführung eines jeweils geeignet temperierten Flüssigkeitsstromes 5 der Prozessflüssigkeit 3 in die jeweilige Behandlungszone 4 erfolgen.. Dabei kann es zweckmäßig sein, dass ein Flüssigkeitsstrom 5 wenigstens einer Behandlungszone 4 zum Erwärmen der Lebensmittel und/oder Behältnisse bei einer Temperatur zwischen 40°C und 50°C zugeführt wird.

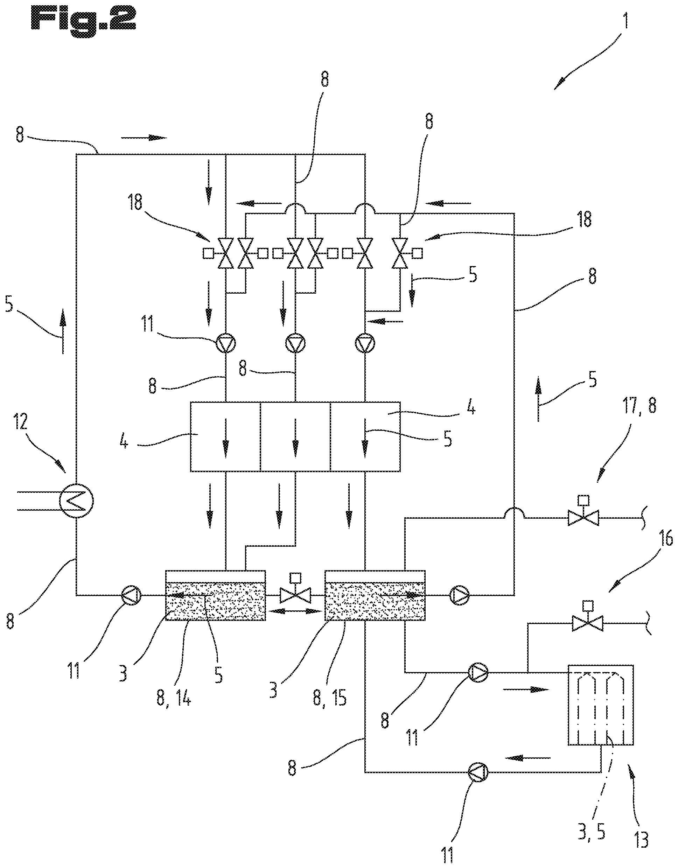

Zum Zweck der Zuführung eines Flüssigkeitsstromes 5 der Prozessflüssigkeit 3 in die jeweilige Behandlungszone 4, können den Behandlungszonen 4 jeweils Fördermittel 11 zugeordnet sein, wie dies aus dem in Fig.2 dargestellten Fließschema ersichtlich ist. Um unnötige Wiederholungen zu vermeiden, werden in der Fig. 2 für gleiche Teile gleiche Bezugszeichen bzw. Bauteilbezeichnungen wie in den vorangegangenen Fig. 1 verwendet, wobei in der Fig. 2 zur besseren Übersichtlichkeit nur drei Behandlungszonen 4 dargestellt sind. Die in Fig. 2 links dargestellte Behandlungszone 4 kann dabei beispielsweise wiederum zur Erwärmung der Behältnisse bzw. Lebensmittel benutzt werden, während die in der Fig. 2 mittig gezeichnete Behandlungszone 4 zur Pasteurisierung und die in der Fig. 2 rechts gezeichnete Behandlungszone 4 zur Abkühlung der Behältnisse bzw. Lebensmittel vorgesehen sein kann.

Das in Fig. 2 dargestellte Ausführungsbeispiel eines Fließschemas einer Vorrichtung 1 umfasst ein Heizmittel 12 zur Erwärmung die Prozessflüssigkeit 3, sowie ein Kühlmittel 13 zur Abkühlung der Prozessflüssigkeit 3. Im Falle des in Fig. 2 gezeigten Ausführungsbeispiels wird die Prozessflüssigkeit 3 dem Heizmittel 12 mittels eines weiteren Fördermittels 11 aus einem als Flüssigkeitstank 14 ausgestalteten Führungselement 8 via als Rohrleitungen oder dergleichen ausgestalteten Führungselementen 8 zugeführt, bzw. durch das Heizmittel 12 hindurchgeführt. Die Erwärmung der Prozessflüssigkeit 3 im Heizmittel 12 kann auf verschiedenste Art und Weise erfolgen, beispielsweise durch Wärmeübertragung auf die Prozessflüssigkeit durch ein Heizmedium, zum Beispiel Sattdampf. Im Prinzip kann jegliche Wärmequelle zur Erhitzung der Prozessflüssigkeit 3 genutzt werden, wobei es zum Zweck einer Pasteurisierung von Lebensmitteln zweckmäßig sein kann, dass das Heizmittel 12 zur Erwärmung der Prozessflüssigkeit 3 auf eine Temperatur von zumindest 80 °C ausgebildet ist. Der so erwärmte Flüssigkeitsstrom 5 der Prozessflüssigkeit 3 kann nach Durchleiten durch das Heizmittel 12 den Behandlungszonen 4 über Führungselemente 8, zum Beispiel Rohrleitungen, zugeführt werden.

Alternativ oder zusätzlich zu dem in Fig. 1 und Fig. 2 dargestellten Ausführungsbeispielen sind auch andere Methoden zur Behandlung der Lebensmittel bzw. Behältnisse denkbar. Beispielsweise kann eine Prozessflüssigkeit, insbesondere Prozesswasser zur Behandlung von Lebensmitteln und/oder Behältnissen auch über den Siedepunkt des Prozesswassers, also auf eine Temperatur über 100 °C erhitzt werden, und einer Behandlungszone als Heißdampf zugeführt werden. Dies ist zum Beispiel zum Zwecke einer Sterilisation zweckmäßig.

Zur Abkühlung der Prozessflüssigkeit 3 kann die Prozessflüssigkeit 3 dem Kühlmittel 13 wie in Fig. 2 dargestellt, zum Beispiel aus einem Flüssigkeitstank 15 zugeführt werden. Im in Fig. 2 gezeigten Ausführungsbeispiel ist das Kühlmittel 13 mit dem Flüssigkeitstank, zum Beispiel einem Kaltwassertank 15 über Führungselemente 8 derart leitungsverbunden, dass ein Flüssigkeitsstrom 5 der Prozessflüssigkeit 3 mittels eines Fördermittels 11 aus dem Flüssigkeitstank 15 entnehmbar ist und nach erfolgter Kühlung der Flüssigkeitsstrom 5 der Prozessflüssigkeit 3 wieder in den Flüssigkeitstank 15 rückführbar ist. Das Kühlmittel 13 kann dabei beispielsweise als Kühlturm oder Wärmetauscher ausgeführt sein, in welchem die Prozessflüssigkeit 3 durch in Gegenrichtung strömende Luft oder ein anderes Kühlmedium gekühlt wird.

Wie weiters aus Fig. 2 ersichtlich ist, sind die Führungselemente 8 zur Beinhaltung bzw. Führung der Prozessflüssigkeit 3 bzw. Flüssigkeitsströmen 5 der Prozessflüssigkeit 3 in der Vorrichtung 1 derart ausgestaltet bzw. angeordnet, dass die Prozessflüssigkeit 3 zumindest teilweise im Kreis wieder in die Behandlungszonen 4 zurückgeführt werden kann. Zur besseren Veranschaulichung sind in Fig. 2 die Strömungsrichtungen für die Flüssigkeitsströme 5 der Prozessflüssigkeit 3 für den Behandlungsbetrieb der Vorrichtung 1 mittels der Pfeile angedeutet. Zur Abführung einer Teilmenge der Prozessflüssigkeit 3 sind absperrbare Entleerungsvorrichtungen 16 vorgesehen und zur Zuführung frischer Prozessflüssigkeit 3 ist zumindest ein absperrbares, als Zufuhrvorrichtung 17 ausgestaltetes, Führungselement 8 angeordnet. Im in Fig. 2 gezeigten Ausführungsbeispiel sind in den jeweils eingangsseitig an den Behandlungszonen 4 angeordneten Führungselementen 8 Durchflussregeleinrichtungen 18 vorgesehen, mittels welcher Durchflussregeleinrichtungen 18 die Flüssigkeitsströme 5 der Prozessflüssigkeit 3 in unterschiedliche Temperaturniveaus kontrolliert abgemischt werden können. Dadurch ist eine gezielte Einstellung der Temperatur der Flüssigkeitsströme 5 der Prozessflüssigkeit 3 für jede Behandlungszone 4 separat ausführbar. Anstelle der dargestellten Durchflussregeleinrichtungen 18 können auch Drei-Weg-Mischventile oder andere geeignete Mittel zur kontrollierten Abmischung bzw. Einstellung der Temperatur eines Flüssigkeitsstromes 5 der Prozessflüssigkeit 3 angeordnet sein.

Selbstverständlich stellt das in Fig. 2 gezeigte Ausführungsbeispiel lediglich eine Ausgestaltungsform einer Vorrichtung 1 zur Behandlung von Produkten bzw. Behältnissen dar. Beispielsweise ist es bei einigen Ausführungsformen für Vorrichtungen zur Behandlung von Lebensmitteln und/oder Behältnissen üblich, einen Flüssigkeitsstrom nach Ableitung aus einer Behandlungszone direkt einer anderen Behandlungszone zuzuführen. Dies ist zum Beispiel dann zweckmäßig, wenn ein aus einer Behandlungszone abgeleiteter Flüssigkeitsstrom der Prozessflüssigkeit ein zum Behandeln der Lebensmittel und/oder Behältnisse in einer anderen Behandlungszone geeignetes Temperaturniveau aufweist.

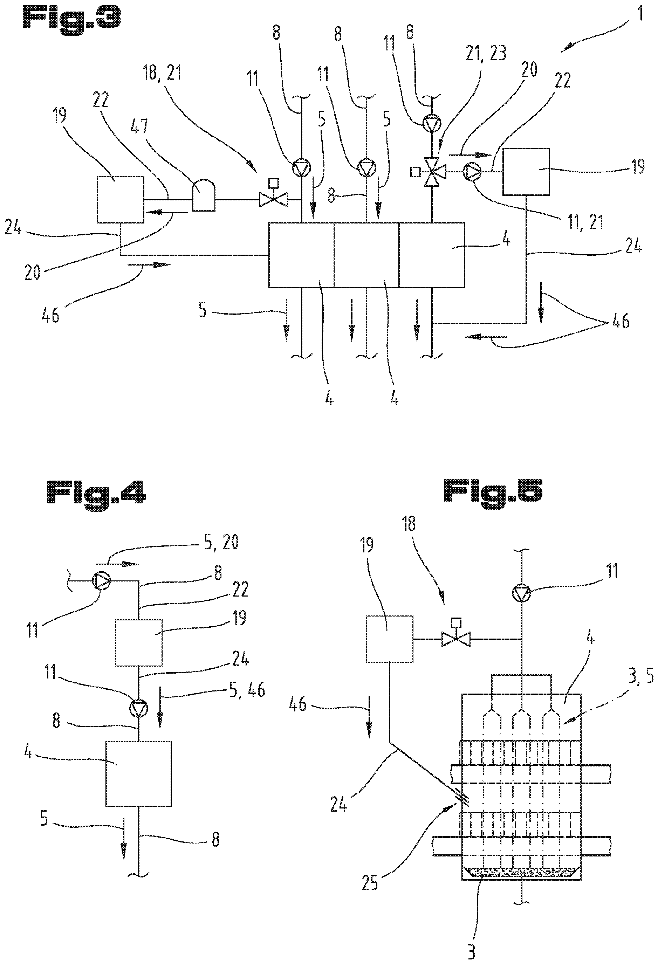

Wie in der Fig. 3 dargestellt ist, umfasst die Vorrichtung 1 zumindest eine Membranfiltrationsanlage 19, wobei die zumindest eine Membranfiltrationsanlage 19 derart in der Vorrichtung 1 angeordnet bzw. derart mit den Führungselementen 8 und/oder mit den Behandlungszonen 4 strömungstechnisch leitungsverbunden ist, dass zumindest eine Teilmenge oder die gesamte Menge der pro Zeiteinheit insgesamt durch alle vorhandenen Behandlungszonen 4 geführten Prozessflüssigkeit zur Bildung wenigstens eines Stromes 20 der Prozessflüssigkeit herangezogen und/oder entnommen, der gebildete Strom 20 oder die gebildeten Ströme 20 mittels der zumindest einen Membranfiltrationsanlage 19 filtriert, und ein filtrierter Strom 46 der Prozessflüssigkeit zumindest teilweise einem Führungselement 8 und/oder einer Behandlungszone 4 wieder zugeführt werden kann. In der Fig. 3 werden für gleiche Teile gleiche Bezugszeichen bzw. Bauteilbezeichnungen wie in den vorangegangenen Fig. 1 und Fig. 2 verwendet. Um unnötige Wiederholungen zu vermeiden, wird auf die detaillierte Beschreibung in den bzw. zu den vorangegangenen Fig. 1 und Fig. 2 hingewiesen bzw. Bezug genommen.

Im Prinzip kann jeder beliebige Flüssigkeitsstrom 5 der Prozessflüssigkeit zur Bildung eines zu filtrierenden Stromes 20 der Prozessflüssigkeit herangezogen werden, bzw. können Teilmengen der Prozessflüssigkeit aus jedem beliebigen Flüssigkeitsstrom 5 zur Bildung eines zu filtrierenden Stromes 20 entnommen werden. Ebenso kann die Rückführung eines filtrierten Stromes 46 der Prozessflüssigkeit im Prinzip in jedes beliebige Führungselement 8für die Prozessflüssigkeit und/oder in jede beliebige Behandlungszone 4 erfolgen. Allerdings ergeben sich bei bestimmten Anordnungsvarianten der Einbindung einer oder mehrerer Membranfiltrationsvorrichtung(en) 19 Vorteile, welche nachstehend anhand weiterer, mittels Figuren dargestellter Ausführungsbeispiele noch näher erläutert werden.

Bevorzugt werden die Anzahl und die Filtrationskapazität der Membranfiltrationsanlage(n) 19 in der Vorrichtung 1 derart festgelegt bzw. ausgestaltet, dass die im kontinuierlichen Behandlungsbetrieb aus zumindest einem die Prozessflüssigkeit beinhaltenden und/oder führenden Element 8 pro Zeiteinheit insgesamt zur Bildung wenigstens eines Stromes 20 der Prozessflüssigkeit 3 herangezogene Prozessflüssigkeitsmenge so gewählt werden kann, dass durch die Filtration des Stromes 20 bzw. der Ströme 20 eine Entfernungsrate für Mikroorganismen erzielbar ist, welche größer ist, als die Wachstumsrate dieser Mikroorganismen in der Prozessflüssigkeit 3 im gleichen Zeitintervall.

Die Anzahl und die Filtrationskapazität der Membranfiltrationsanlage(n) 19 können auch derart gewählt bzw. festgelegt werden, dass, bezogen auf die pro Zeiteinheit insgesamt durch alle vorhandenen Behandlungszonen 4 geführte Menge an Prozessflüssigkeit, pro Zeiteinheit zumindest 1 % und weniger als 25 % zur Bildung wenigstens eines zu filtrierenden Stromes 20 herangezogen werden können und diese pro Zeiteinheit gebildete Teilmenge der Prozessflüssigkeit mittels der Membranfiltrationsanlage(n) 19 filtriert werden kann. Besonders vorteilhaft ist eine Ausgestaltungsform, bei welcher, bezogen auf die pro Zeiteinheit insgesamt durch alle vorhandenen Behandlungszonen 4 geführte Menge an Prozessflüssigkeit, pro Zeiteinheit zwischen 2 % und 10 % und insbesondere zwischen 2,5 und 7 % zur Bildung wenigstens eines Stromes 20 der Prozessflüssigkeit herangezogen werden und diese pro Zeiteinheit gebildete Teilmenge der Prozessflüssigkeit mittels zumindest einer Membranfiltrationsanlage 19 filtriert wird.

Zusätzlich werden die Anzahl und die Filtrationskapazität der Membranfiltrationsanlage(n) 19 bevorzugt derart festgelegt, dass das insgesamt in der Vorrichtung 1 zur Behandlung von Lebensmitteln und/oder Behältnissen enthaltene Volumen an Prozessflüssigkeit, pro Tag zumindest 1 Mal und bevorzugt zwischen 2 Mal und 10 Mal mittels der Membranfiltrationsanlage(n) 19 filtriert werden kann.

Eine Möglichkeit zur Bildung und Filtration eines Stromes 20 der Prozessflüssigkeit ist in Fig.3 dargestellt, wobei in Fig. 3 beispielhaft zwei Membranfiltrationsanlagen 19 in einer ausschnittsweise dargestellten Vorrichtung 1 zur Behandlung von Lebensmitteln und/oder Produkten, angeordnet sind. In Fig. 3 werden für gleiche Teile gleiche Bezugszeichen bzw. Bauteilbezeichnungen, wie in den vorangegangenen Fig. 1 und 2 verwendet. Um unnötige Wiederholungen zu vermeiden, wird auf die detaillierte Beschreibung in den bzw. zu den vorangegangenen Fig. 1, 2 hingewiesen bzw. Bezug genommen.

Im in Fig. 3 gezeigten Ausführungsbeispiel sind die beiden dargestellten Membranfiltrationsanlagen 19 jeweils Bypass-artig zwischen einem einen Flüssigkeitsstrom 5 führenden Führungselement 8 und einer Behandlungszone 4 (links in Fig. 3) oder einem weiteren Führungselement 8 (rechts in Fig. 3) angeordnet. Im gezeigten Ausführungsbeispiel wird mittels geeigneter Verteilungsmittel 21 pro Zeiteinheit jeweils eine Teilmenge aus einem Flüssigkeitsstrom 5 der Prozessflüssigkeit zur Bildung eines zu filtrierenden Stromes 20 entnommen und mittels einer der Membranfiltrationsanlagen 19 filtriert. Hierbei kann Zuleitungselement 22 der Membranfiltrationsanlage 19 strömungstechnisch mit einem Führungselement 8 für die Prozessflüssigkeit oder auch mit einer Behandlungszone strömungstechnisch leitungsverbunden sein.

Zur kontrollierten bzw. steuerbaren Entnahme einer Teilmenge aus dem Flüssigkeitsstrom 5 zur Bildung eines Stromes 20 kann in einem Zuleitungselement 22 zu einer Membranfiltrationsanlage 19 beispielsweise ein als Durchflussregeleinrichtung 18 ausgestaltetes Verteilungsmittel 21 angeordnet sein, wie dies auf der linken Seite in Fig. 3 gezeigt ist. Alternativ kann zum Beispiel, wie auf der rechten Seite der Fig. 3 dargestellt, auch ein Drei-Wege-Verteilerventil 23 als Verteilungsmittel 21 benutzt werden. Dabei kann auch ein mit einem Ventil 18, 23 zusammenwirkendes Verteilungsmittel 21 in Form eines Fördermittels 11 bzw. einer Pumpe ausgestaltet sein, um eine kontrollierte Entnahme einer Teilmenge der Prozessflüssigkeit aus dem Führungselement 8 zu ermöglichen. Grundsätzlich kann ein als Fördermittel 11 ausgestaltetes Verteilungsmittel 21 auch alleinig verwendet werden. Bevorzugt wird allerdings auf das Anordnen eines zusätzlichen Fördermittels 11 in einem Zuleitungselement 22 einer Membranfiltrationsanlage 19 verzichtet und, wie auf der linken Seite in Fig. 3 dargestellt, die Entnahme einer Teilmenge der Prozessflüssigkeit mittels eines in einem Führungselement 8 der Vorrichtung 1 angeordnetem Fördermittels 11 bewerkstelligt.

Nach erfolgter Filtration wird ein filtrierter Strom 46 der Prozessflüssigkeit, wie anhand des in Fig. 3 links gezeigten Beispiels, wieder einer Behandlungszone 4 und/oder, wie anhand des in Fig. 3 rechts gezeigten Beispiels, wieder einem Führungselement 8 zugeführt. Hierzu kann ein Ableitungselement 24 einer Membranfiltrationsanlage 19 wiederum mit einer Behandlungszone 4 oder mit einem Führungselement 8 für die Prozessflüssigkeit strömungstechnisch leitungsverbunden sein.

Die in Fig. 3 auf der linken Seite dargestellte Behandlungszone 4 kann beispielsweise wiederum als Aufwärmzone für die Lebensmittel bzw. Behältnisse ausgebildet sein, die in Fig. 3 mittig dargestellte Behandlungszone 4 zur Pasteurisierung der Lebensmittel und die in Fig. 3 rechts gezeichnete Behandlungszone 4 zur Abkühlung der Lebensmittel bzw. Behältnisse dienen. Dementsprechend wird im laufenden Behandlungsbetrieb der Vorrichtung 1 der mittig angeordneten Pasteurisierungszone 4 ein Flüssigkeitsstrom 5 mit hoher Temperatur der Prozessflüssigkeit 3 zugeleitet, wohingegen den Behandlungszonen 4, zur Erwärmung bzw. Abkühlung der Lebensmittel bzw. Behältnisse, Flüssigkeitsströme 5 mit vergleichsweise niedriger Temperatur zugeführt werden.

Wie in Fig. 3 angedeutet, wird insbesondere zur Schonung einer Membranfiltrationsanlage 19 bevorzugt ein Flüssigkeitsstrom 5 mit verhältnismäßig geringer Temperatur zur Bildung eines zu filtrierenden Stromes 20 der Prozessflüssigkeit herangezogen. Im in Fig. 3 dargestellten Ausführungsbeispiel sind daher die Zuleitungselemente 22 der Membranfiltrationsanlagen 19 mit den zu den Aufwärmungs- bzw. Abkühlungs-Behandlungszonen, also den links bzw. rechts in Fig. 3 dargestellten Behandlungszonen 4, führenden Führungselementen 8 strömungstechnisch leitungsverbunden. Die beiden Führungselemente 8, mit welchen die Zuleitungselemente 22 leitungsverbunden sind, beinhalten jeweils einen Flüssigkeitsstrom 5 mit verhältnismäßig geringer Temperatur der Prozessflüssigkeit. Bevorzugt ist die zumindest eine Membranfiltrationsanlage 19 zur Bildung eines zu filtrierenden Stromes 20 der Prozessflüssigkeit an solchen Stellen mit Führungselementen 8 strömungstechnisch leitungsverbunden ist, dass die in den Führungselementen 8 beinhaltete bzw. geführte Prozessflüssigkeit eine Temperatur von weniger als 80 °C und insbesondere weniger als 50 °C aufweist. Bevorzugt ist die zumindest eine Membranfiltrationsanlage 19 zur Bildung eines zu filtrierenden Stromes 20 der Prozessflüssigkeit an solchen Stellen mit Führungselementen 8 der Vorrichtung 1 strömungstechnisch leitungsverbunden ist, dass zur Bildung wenigstens eines zu filtrierenden Stromes 20 Prozessflüssigkeit mit einer Temperatur zwischen 40°C und 50°C herangezogen wird. Wie sich gezeigt hat, ist die Membranfiltration bzw. die Filtrationsleistung eines Stromes 20 einer Prozessflüssigkeit in diesem Temperaturbereich besonders effizient.

Wie weiters in der Fig. 3 dargestellt ist, kann hierbei zusätzlich vorgesehen sein, dass ein Zuleitungselement 22 einer Membranfiltrationsanlage 19 mit einem temperierbaren Durchflussbehälter 47 für die Prozessflüssigkeit leitungsverbunden ist. Ein derartiger Durchflussbehälter 47 kann beispielsweise als Pufferspeicher mit integriertem Wärmetauscher oder als Pufferspeicher mit integrierter Elektroheizung oder Dergleichen ausgebildet sein. Auf diese Weise kann ein zu filtrierender Strom 20 durch Heranziehen der Prozessflüssigkeit aus dem Durchflussbehälter 47 gebildet werden.

Alternativ zum in Fig. 3 gezeigten Ausführungsbeispiel kann zur Bildung eines zu filtrierenden Stromes 20 der Prozessflüssigkeit, ein gesamter Flüssigkeitsstrom 5 der Prozessflüssigkeit herangezogen werden, wie dies in Fig. 4 schematisch dargestellt ist. Im in Fig. 4 dargestellten Beispiel ist die Membranfiltrationsanlage 19 strömungstechnisch seriell in einem zu einer Behandlungszone 4 führenden Führungselement 8 angeordnet bzw. deren Zuleitungselement 22 und Ableitungselement 24 jeweils mit dem zu einer Behandlungszone 4 führenden Führungselement 8 leitungsverbunden. Dadurch wird der gesamte durch das Führungselement 8 geführte Flüssigkeitsstrom 5 der Prozessflüssigkeit 3 durch die Membranfiltrationsanlage 19 geleitet und im in Fig. 4 dargestellten Beispiel nach erfolgter Filtration in eine Behandlungszone 4 eingeleitet. Bei einer derartigen Anordnung einer Membranfiltrationsanlage 19 kann aufgrund des Druckverlustes über die Membranfiltrationsanlage 19 ein Anordnen eines zusätzlichen Fördermittels 11 zur Einbringung der Prozessflüssigkeit 3 in eine Behandlungszone 4 nach erfolgter Mikro- bzw. Ultrafiltration erforderlich sein.

Dabei kann eine Zuführung eines filtrierten Stromes 46 der Prozessflüssigkeit in eine Behandlungszone 4 nach erfolgter Filtration vorteilhafterweise ohne Fördermittel 11 bewerkstelligt werden. Hierzu kann es zweckmäßig sein, dass Ableitungselemente 24 einer Membranfiltrationsanlage 19 zum Beispiel mit einer Behandlungszone 4 derart leitungsverbunden sind, dass zumindest ein filtrierter Strom 46 der Prozessflüssigkeit der Behandlungszone 4 unter Einwirkung der Schwerkraft, in freiem Gefälle zugeführt werden kann. Ein solches Ausführungsbeispiel ist in Fig. 5 dargestellt, wobei wiederum für gleiche Teile gleiche Bezugszeichen bzw. Bauteilbezeichnungen wie in den vorangegangenen Fig. 1 bis 4 verwendet werden, um unnötige Wiederholungen zu vermeiden. In Fig. 5 ist ein Ausführungsbeispiel für eine leitungstechnische Verbindung einer Membranfiltrationsanlage 19 mit einer Behandlungszone 4 dargestellt, bei welcher ein von der Membranfiltrationsanlage 19 zur Behandlungszone 4 führendes Ableitungselement 24 derart angeordnet ist, dass ein stetiges Gefälle von oben nach unten in Richtung von der Membranfiltrationsanlage 19 zu der Behandlungszone 4 ausgebildet ist, wodurch der von der Membranfiltrationsanlage 19 zur Behandlungszone 4 geführte, filtrierte Strom 46 der Prozessflüssigkeit 3 unter Schwerkrafteinwirkung fließen kann. Zur Einbringung des filtrierten Stromes 46 der Prozessflüssigkeit 3 in die Behandlungszone 4 kann bzw. können in einfacher Art und Weise eine oder mehrere Öffnung(en) 25 in der Behandlungszone 4 ausgestaltet, bzw. mit den Ableitungselementen 24 leitungsverbunden sein, sodass der filtrierte Strom 46 in die Behandlungszone 4 fließen kann.

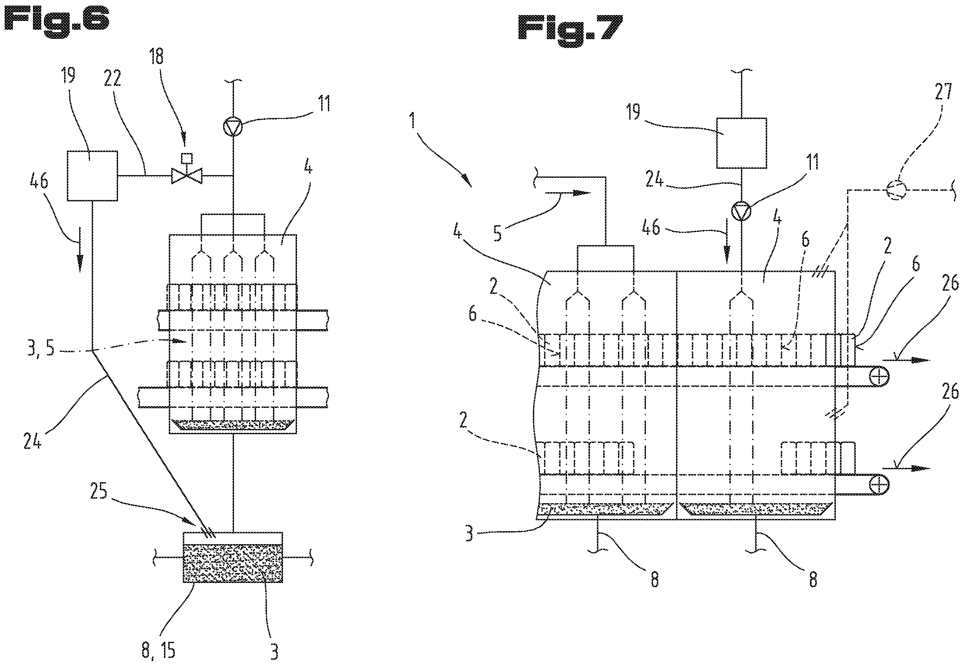

In Fig. 6 ist eine weitere und gegebenenfalls für sich eigenständige Ausführungsform der Vorrichtung 1 ausschnittsweise gezeigt, wobei wiederum für gleiche Teile gleiche Bezugszeichen bzw. Bauteilbezeichnungen, wie in den vorangegangenen Fig. 1 bis 5 verwendet werden. Um unnötige Wiederholungen zu vermeiden, wird auf die detaillierte Beschreibung in den vorangegangenen Fig. 1 bis 5 hingewiesen bzw. Bezug genommen. Fig. 6 zeigt in zu dem in Fig. 5 dargestellten Ausführungsbeispiel eine Anordnung zur Zuführung eines filtrierten Stromes 46 der Prozessflüssigkeit 3 in ein Führungselement 8 für die Prozessflüssigkeit 3, zum Beispiel einen Flüssigkeitstank 15. Die Ableitungselemente 24 erstrecken sich wiederum in stetigem Gefälle von oben nach unten in Richtung von der Membranfiltrationsanlage 19 zum Flüssigkeitstank 8, 15, sodass der filtrierte Strom 46 durch die Öffnung(en) 25 in den Flüssigkeitstank 8, 15 fließen kann.

In Fig. 7 ist eine weitere und gegebenenfalls für sich eigenständige Ausführungsform der Vorrichtung 1 ausschnittsweise gezeigt, wobei wiederum für gleiche Teile gleiche Bezugszeichen bzw. Bauteilbezeichnungen, wie in den vorangegangenen Fig. 1 bis 6 verwendet werden. Um unnötige Wiederholungen zu vermeiden, wird auf die detaillierte Beschreibung in den vorangegangenen Fig. 1 bis 6 hingewiesen bzw. Bezug genommen. In Fig. 7 ist eine Behandlungszone 4 zur Spülung der Außenseite 6 von mit dem Lebensmittel befüllten und verschlossenen Behältnissen 2 ausgestaltet, welche zumindest eine Behandlungszone 4 bezogen auf die Transportrichtung 26 der Behältnisse 2 durch die Behandlungszonen 4, am Ende der Behandlungszonenstrecke angeordnet ist. Zur Reinigung der Behältnisse 2 wird der Behandlungszone 4 ein filtrierter Strom 46 der Prozessflüssigkeit 3 zugeführt. Die Behandlungszone 4 ist zu diesem Zweck mit einem Ableitungselement 24 einer Membranfiltrationsanlage 19 strömungstechnisch leitungsverbunden. Weiters kann der Behandlungszone 4 beispielsweise ein Gebläse 27 zur Trocknung der Behältnisse 2 mittels Trocknungsluft zugeordnet, oder eine andere Trocknungsvorrichtung vorgesehen sein.

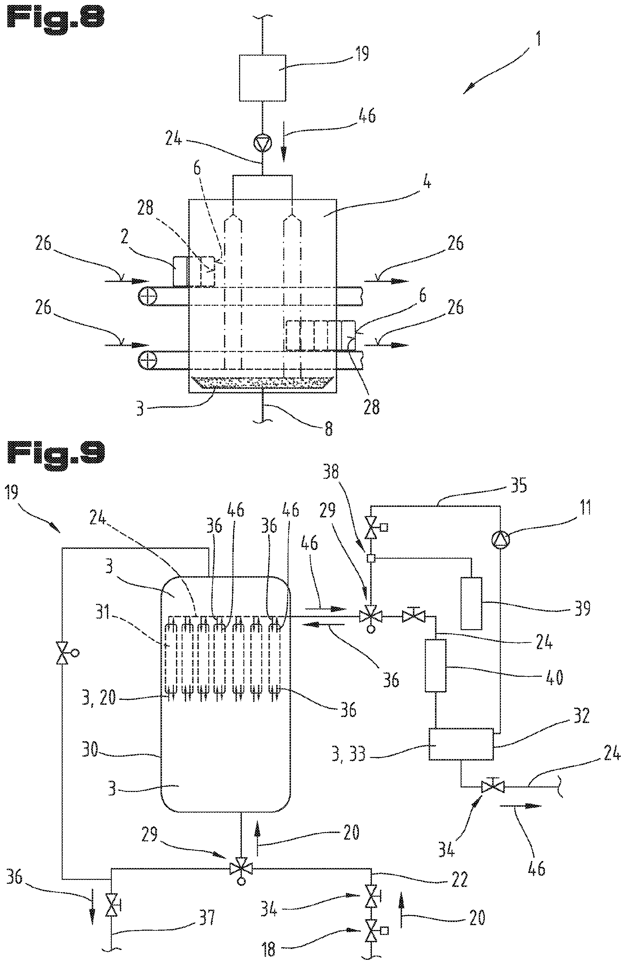

In Fig. 8 ist eine weitere und gegebenenfalls für sich eigenständige Ausführungsform der Vorrichtung 1 ausschnittsweise gezeigt, wobei wiederum für gleiche Teile gleiche Bezugszeichen bzw. Bauteilbezeichnungen, wie in den vorangegangenen Fig. 1 bis 7 verwendet werden. Um unnötige Wiederholungen zu vermeiden, wird auf die detaillierte Beschreibung in den vorangegangenen Fig. 1 bis 7 hingewiesen bzw. Bezug genommen. In der Fig. 8 ist eine Behandlungszone 4 zur Spülung bzw. Reinigung der Innenseite 28 und der Außenseite 6 von unbefüllten bzw. unverschlossenen Behältnissen 2 dargestellt, wobei die dargestellte Behandlungszone 4, bezogen auf die Transportrichtung 26 der Behältnisse 2 durch die Behandlungszonen 4, eingangsseitig der Behandlungszonenstrecke angeordnet ist. Zum Zwecke der Reinigung der unverschlossenen Behältnisse 2 wird der Behandlungszone 4 wiederum ein filtrierter Strom 46 der Prozessflüssigkeit 3 zugeführt. Die Behandlungszone 4 ist zu diesem Zweck mit einem Ableitungselement 24 einer Membranfiltrationsanlage 19 strömungstechnisch leitungsverbunden.

In Fig. 9 ist ein Ausführungsbeispiel für eine Ausgestaltungsform einer Membranfiltrationsanlage 19 dargestellt. Um unnötige Wiederholungen zu vermeiden, wird wiederum auf die detaillierte Beschreibung in den vorangegangenen Fig. 1 bis 8 hingewiesen bzw. Bezug genommen, bzw. werden für gleiche Teile gleiche Bezugszeichen bzw. Bauteilbezeichnungen, wie in den vorangegangenen Fig. 1 bis 8 verwendet. Der Membranfiltrationsanlage 19 wird im Filtrationsbetrieb, wie bereits ausführlich beschrieben, ein zu filtrierender Strom 20 der Prozessflüssigkeit 3 durch die Zuleitungselemente 22 zugeführt, wobei eine pro Zeiteinheit zugeführte Teilmenge zum Beispiel mittels einer Durchflussregeleinrichtung 18 festlegbar ist. Der zu filtrierende Strom 20 der Prozessflüssigkeit 3 kann beispielsweise über ein Drei-Wege-Ventil 29 in einen Druckbehälter 30 eingeleitet werden, in welchem Filtermembranmodule 31 zur Filtration der Prozessflüssigkeit 3 angeordnet sind.

Die in Fig. 9 gezeigten Filtermembranmodule 31 können Membranen verschiedenster Art umfassen. Die Membranen können homogen oder inhomogen in ihrem Aufbau sein und im Querschnitt verschiedene Symmetrien aufweisen. Insbesondere können poröse Membranen in Kapillar- bzw. Hohlfaserausführung und/oder Flachmembranen verwendet werden. Die Membranen können dabei aus diversen Materialien aufgebaut sein. Beispiele für geeignete Membranmaterialien sind Polyethylen, Polypropylen, Polyethersulfon, Polyvinylidenfluorid, Ethylen-Propylen-Dien-Kautschuk (EPDM), Polyurethan oder Zelluloseacetat. Bevorzugt werden Membranmaterialien in hydrophiler Ausbildung verwendet. Alternativ und/oder zusätzlich zu Kunststoffmembranen können auch keramische Materialien zur Bildung der Membranen der Filtermembranmodule 31 verwendet werden. Insbesondere sind chlorbeständige Membranmaterialien geeignet, welche gegenüber einer Chlorbelastung von mehr als 200.000 ppm*h, und bevorzugt mehr als 2.000.000 ppm*h dauerbeständig sind.

Die gezeigten Ausführungsbeispiele zeigen einen Betrieb der Vorrichtung 1 bzw. der Membranfiltrationsanlage(n) 19 im Überdruckbetrieb. Alternativ bzw. zusätzlich können in der Vorrichtung 1 zumindest abschnittsweise auch Unterdruckzonen ausgebildet sein, insbesondere ist ein Unterdruckbetrieb einer Membranfiltrationsanlage 19 denkbar. Dabei können beispielsweise in den Ableitungselementen 24 Saugvorrichtungen (nicht dargestellt) angeordnet sein, mittels welcher ein filtrierter Strom 46 der Prozessflüssigkeit 3 aus einem Filtermembranmodul 31 abgezogen werden kann. Deshalb sind die Filtermembranen der Filtermembranmodule 31 bevorzugt gegenüber Überdruck und Unterdruck beständig ausgeführt und für transmembrane Drücke bzw. Druckdifferenzen von mindestens 1.000 mbar geeignet, ohne dass es zu einer permanenten Verblockung der Membran im Dauerbetrieb der Membranfiltrationsanlage 19 kommt. Je nach Bedarf können auch Membranen eingesetzt werden, welche für Drücke von beispielsweise 2.000 mbar und bis zu 5.000 mbar über die jeweilige Membran geeignet sind. Im Filtrationsbetrieb beträgt die transmembrane Druckdifferenz bevorzugt weniger als 5 bar, insbesondere weniger als 2 bar, und besonders bevorzugt 1 bar oder weniger. Bevorzugt werden poröse Membranen verwendet, wobei der wirksame Porendurchmesser einer jeweiligen Membran in einem Bereich zwischen 0,01 m und 1 m liegen kann, insbesondere sind Membranen mit wirksamen Porendurchmessern zwischen 0,05 m und 0,5 m für die Filtermembranmodule 31 der Membranfiltrationsanlage(n) 19 geeignet.

Im in Fig. 9 gezeigten Ausführungsbeispiel ist ein sogenannter 'Outside-In'-Betrieb der Membranfiltrationsanlage 19 dargestellt, bei welchem der zu filtrierende Strom 20 der Prozessflüssigkeit 3 im Filtrationsbetrieb von außen in die Filtermembranmodule 31 eintritt, über die Filtermembranen der Filtermembranmodule 31 filtriert, und ein filtrierter Strom 46 der Prozessflüssigkeit 3 mittels Ableitungselementen 24 aus dem Inneren der Filtermembranmodule 31 abgeleitet wird. Alternativ zum in Fig. 9 gezeigten Ausführungsbeispiel ist auch ein sogenannter 'Inside-Out'-Betrieb möglich, bei welchem ein zu filtrierender Strom 20 der Prozessflüssigkeit 3 im Filtrationsbetrieb ins Innere der Filtermembranmodule 31 geführt wird und ein filtrierter Strom 46 der Prozessflüssigkeit 3 an der Außenseite der Filtermembranmodule 31 austritt. Weiters sind betreffend den Fluss des Stromes 20 der Prozessflüssigkeit 3 in einem Filtermembranmodule 31 sowohl ein sogenannter 'cross-flow'-Betrieb als auch eine zyklische 'dead-end'-Verschaltung möglich. Schließlich sind auch getauchte Membrankonfigurationen, bei welchen ein filtrierter Strom 46 der Prozessflüssigkeit 3 mittels Unterdruck abgesaugt wird, möglich. Insbesondere bei einer Ausführung einer Membranfiltrationsanlage 19 in getauchter Konfiguration, kann zum Entgegenwirken der Deckschichtbildung auf den Membranoberflächen, auch eine zyklische oder a-zyklische Luftblasenspülung bzw. Luftverwirbelung vorgesehen sein, bzw. durchgeführt werden.

Im in Fig. 9 gezeigten Ausführungsbeispiel wird der filtrierte Strom 46 der Prozessflüssigkeit 3 nach Durchtritt der Prozessflüssigkeit 3 durch die Filtermembranmodule 31 und erfolgter Filtration über die Ableitungselemente 24 wieder aus der Membranfiltrationsanlage 19 abgeführt. Dabei kann es, wie in Fig. 9 gezeigt, zweckmäßig sein, in der Ableitung 24 einen Vorlagebehälter 32 mit Überlauf anzuordnen, welcher in Abhängigkeit seiner Dimensionen zur Zwischenspeicherung eines gewissen Volumens der gefilterten Prozessflüssigkeit 3 bzw. eines Filtrats 33 ausgestaltet ist. Insbesondere kann dieses Filtrat 33 der Prozessflüssigkeit 3 zur Reinigung durch Rückspülen, unter Umkehr der Flussrichtung über die Filtermembranmodule 31, genutzt werden.

Zur Durchführung eines Reinigungsbetriebs für die Filtermembranmodule 31 sind in den Zuleitungselementen 22 und den Ableitungselementen 24 Absperrmittel 34 angeordnet, welche ein strömungstechnisches Abtrennen der Membranfiltrationsanlage 19 von den weiteren baulichen Elementen der Vorrichtung zur Behandlung von Lebensmitteln bzw. Behältnissen erlauben. Weiters ist in dem Vorlagebehälter 32 und/oder einer sich zwischen dem Vorlagebehälter 32 und dem Ableitungselement 24 der Membranfiltrationsanlage 19 erstreckenden Rückspülleitung 35 zumindest ein Fördermittel 11 angeordnet. So kann durch entsprechendes Umschalten der Drei-Wege-Ventile 29 die Flussrichtung in der Membranfiltrationsanlage 19 umgekehrt werden, sodass die Prozessflüssigkeit 3 in zum Filtrationsbetrieb umgekehrter Richtung 36 über die Filtermembranmodule 31 strömt. Zum Abführen des im Zuge der Reinigung unter Umkehrung der Flussrichtung über die Filtermembranen der Membranfilteranlage 19 anfallenden Flüssigkeitsabfalls ist der Membranfiltrationsanlage 19 zumindest eine absperrbare Flüssigkeitsabfallleitung 37 zugeordnet. Eine, der abgeführten Menge an Flüssigkeitsabfall entsprechende Menge, an frischer Prozessflüssigkeit 3 kann zum Beispiel durch die in Fig. 2 dargestellte Zufuhrvorrichtung 17 für frische Prozessflüssigkeit 3 erfolgen.

Wie in Fig. 9 weiters dargestellt, kann in einem Ableitungselement 24 bzw. in der Rückspülleitung 35 der einer Membranfiltrationsanlage 19 eine Dosiereinrichtung 38 angeordnet sein, mittels welcher der Prozessflüssigkeit 3 bzw. dem Filtrat 33 der Prozessflüssigkeit 3 sowohl im Filtrationsbetrieb, als auch im Reinigungsbetrieb für die Membranfiltrationsanlage 19 Chemikalien aus einer oder mehreren Chemikalienquellen 39 beigemengt werden können. Ein Beimengen von Chemikalien kann im Filtrationsbetrieb über das in der Ableitung 24 angeordnete Drei-Wege-Ventil 29 erfolgen. In der Ableitung 24 der Membranfiltrationsanlage 19 kann weiters eine Adsorptionsvorrichtung 40 angeordnet sein, mittels welcher ein Entfernen bzw. Abscheiden von in einem filtrierten Strom 46 der Prozessflüssigkeit 3 gelöster bzw. suspendierter oder dispergierter Substanzen möglich ist.

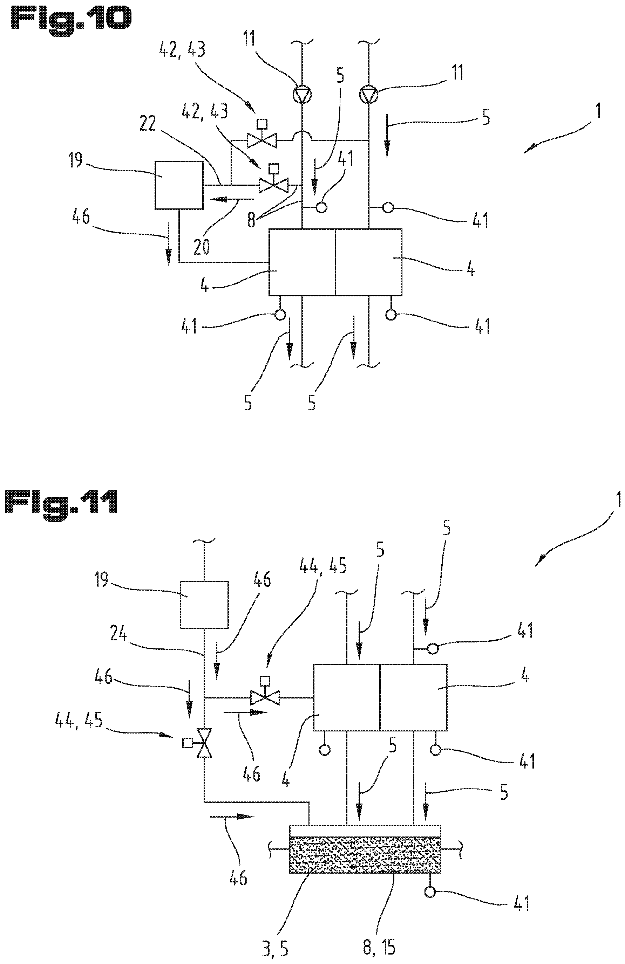

In Fig. 10 ist eine weitere und gegebenenfalls für sich eigenständige Ausführungsform der Vorrichtung 1 ausschnittsweise gezeigt, wobei wiederum für gleiche Teile gleiche Bezugszeichen bzw. Bauteilbezeichnungen, wie in den vorangegangenen Fig. 1 bis 9 verwendet werden. Um unnötige Wiederholungen zu vermeiden, wird auf die detaillierte Beschreibung in den vorangegangenen Fig. 1 bis 9 hingewiesen bzw. Bezug genommen. In Fig. 10 sind Sensoren 41 dargestellt, welche zur kontinuierlichen Überwachung des Verunreinigungsgrades, insbesondere durch Messen der Trübung der Prozessflüssigkeit, ausgestaltet sind. Dabei können die Sensoren 41 in den Führungselementen 8 und/oder in den Behandlungszonen 4 der Vorrichtung 1 angeordnet sein.

Die Messwerte der Sensoren 41 können beispielsweise herangezogen werden, um eine Membranfiltrationsanlage 19 mittels Schaltelementen und Leitungselementen verschiedenen Behandlungszonen 4 bzw. Führungselementen 8 für Flüssigkeitsströme der Prozessflüssigkeit zuzuordnen. Selbstverständlich kann ein Umschalten zwischen Führungselementen 8 und/oder Behandlungszonen 4 dabei auch in Abhängigkeit von Messungen anhand aus der Vorrichtung 1 entnommener Stichproben erfolgen.

Beispielweise kann vorgesehen sein, dass ein Strom 20 der Prozessflüssigkeit 3 zur Filtration mittels einer Membranfiltrationsanlage 19 durch Umschalten zwischen oder Mischen von unterschiedlichen Flüssigkeitsströmen 5 der Prozessflüssigkeit 3 aus verschiedenen Führungselementen 8 in Abhängigkeit von im Zuge von Inline-Messungen und/oder Stichprobenmessungen erhaltenen Messwerten gebildet wird. Außerdem kann vorgesehen sein, dass ein filtrierter Strom 46 der Prozessflüssigkeit 3 in Abhängigkeit von im Zuge von Inline-Messungen und/oder Stichprobenmessungen erhaltenen Messwerten, durch Einleiten und/oder Verteilen des filtrierten Stromes 46 in verschiedene Flüssigkeitsströme 5 in verschiedenen Führungselementen 8 und/oder Behandlungszonen 4 zurückgeführt wird.

Zum Zwecke eines Umschaltens einer Membranfiltrationsanlage 19 auf verschiedene Führungselemente 8 können den Zuleitungselementen 22 einer Membranfiltrationsanlage 19 zum Beispiel zwei Umschaltmittel 42, 42 zugeordnet sein, wie dies in Fig. 10 veranschaulicht ist. Die beiden dargestellten Umschaltmittel 42, 42 sind dabei mit zwei verschiedenen, die Prozessflüssigkeit beinhaltenden, Führungselementen 8, 8 strömungstechnisch derart leitungsverbunden, dass eine Bildung eines Stromes 20 der Prozessflüssigkeit zwecks Filtration wahlweise aus einem der beiden Flüssigkeitsströme 5, 5 der Prozessflüssigkeit in den Führungselementen 8, 8, oder aus beiden Flüssigkeitsströmen 5, 5 erfolgen kann. Zu diesem Zweck können die beiden Umschaltmittel 42, 42 als sogenannte ,Auf/Zu-Ventile' ausgebildet sein, sodass jedes der beiden Umschaltmittel 42, 42 die Zufuhr von Prozessflüssigkeit in die Membranfiltrationsanlage 19 strömungstechnisch öffnen oder sperren kann.

Alternativ zum in Fig. 10 gezeigten Ausführungsbeispiel wäre natürlich auch ein einziges, als 3-Wege-Umschaltmittel (in Fig. 10 nicht dargestellt) ausgebildetes, Umschaltmittel 42, zum Umschalten der Zuleitungselemente 22 der Membranfiltrationsanlage 19 auf eines der beiden Führungselemente 8 geeignet. Das als 3-Wege-Umschaltmittel 42 ausgebildete Umschaltmittel 42 wäre wiederum einerseits den Zuleitungselementen 22 der Membranfiltrationsanlage 19 zugeordnet und andererseits mit zwei verschiedenen Führungselementen 8, 8 leitungsverbunden.

In Fig. 10 und im Folgenden wird zum besseren Verständnis die Darstellung und Beschreibung mittels 2-Wege-Ventilen beibehalten, wobei an dieser Stelle angemerkt wird, dass die Ausgestaltungsform bzw. Anordnung einer verschaltbaren Membranfiltrationsanlage 19 auf zahlreiche Arten gebildet werden kann und nicht auf die in Fig. 10 dargestellten und im Folgenden dargelegten Ausführungsbeispiele beschränkt ist.

Anstelle von Umschaltmitteln 42 können einem Zuleitungselement 22 einer Membranfiltrationsanlage 19 auch zwei Mischmittel 43, 43 zugeordnet sein, wie es in Fig. 10 angedeutet ist. Die Mischmittel 43, 43 sind wiederum mit zwei verschiedenen, die Prozessflüssigkeit beinhaltenden, Führungselementen 8, 8 strömungstechnisch derart leitungsverbunden, dass eine Bildung des Stromes 20 der Prozessflüssigkeit zwecks Filtration wiederum wahlweise aus einem der beiden Flüssigkeitsströme 5, 5 der Prozessflüssigkeit, oder aus beiden Flüssigkeitsströmen 5, 5 in den Führungselementen 8, 8 , oder durch Entnahme und Mischung festlegbarer Teilmengen aus den beiden Flüssigkeitsströmen 5, 5 der Prozessflüssigkeit erfolgen kann. Zu diesem Zweck können die Mischmittel 43, 43 beispielsweise als Durchflussregelventile ausgestaltet sein.

Selbstverständlich können einer Membranfiltrationsanlage 19 auch mehr als zwei Umschaltmittel 42 und/oder Mischmittel 43 zugeordnet sein, welche dementsprechend mit mehr als zwei Führungselementen 8 leitungsverbunden sein können.

In Fig. 11 ist eine weitere und gegebenenfalls für sich eigenständige Ausführungsform der Vorrichtung 1 ausschnittsweise gezeigt, wobei wiederum für gleiche Teile gleiche Bezugszeichen bzw. Bauteilbezeichnungen, wie in den vorangegangenen Fig. 1 bis 10 verwendet werden. Um unnötige Wiederholungen zu vermeiden, wird auf die detaillierte Beschreibung in den vorangegangenen Fig. 1 bis 10 hingewiesen bzw. Bezug genommen. In dem in Fig. 11 dargestellten Ausführungsbeispiel sind einer Ableitung 24 einer Membranfiltrationsanlage 19 zwei Umschaltelemente 44, 44 zugeordnet. Ein Umschaltelement 44 ist mit einem als Flüssigkeitstank 15 ausgebildeten Führungselement 8 strömungstechnisch leitungsverbunden. Das andere Umschaltelement 44 ist mit einer Behandlungszone 4 strömungstechnisch leitungsverbunden. Durch diese Ausgestaltungsform kann eine Zuführung eines filtrierten Stromes 46 der Prozessflüssigkeit 3 wahlweise in das als Flüssigkeitstank 15 ausgebildete Führungselement 8 oder die Behandlungszone 4, oder sowohl in das Führungselement 8, als auch in die Behandlungszone 4 erfolgen.

Zu diesem Zweck können die beiden Umschaltelemente 44, 44 in Fig. 11 wiederum als sogenannte "Auf/Zu-Ventile" ausgebildet sein, sodass jedes der beiden Umschaltelemente 44, 44 die Abführung eines filtrierten Stromes 46 aus der Membranfiltrationsanlage 19 in die Behandlungszone 4 und/oder in das wenigstens eine Führungselement 8 strömungstechnisch öffnen oder sperren kann.

Anstelle von Umschaltelementen 44 können einem Ableitungselement 24 einer Membranfiltrationsanlage 19 auch zwei Verteilerelemente 45, 45 zugeordnet sein, wie es in Fig. 11 angedeutet ist. Ein Verteilerelement 45 ist wiederum mit einem als Flüssigkeitstank 15 ausgebildeten Führungselement 8 strömungstechnisch leitungsverbunden. Das andere Verteilerelement 45 ist mit einer Behandlungszone 4 strömungstechnisch leitungsverbunden. Durch diese Ausgestaltungsform kann eine Zuführung des filtrierten Stromes 46 der Prozessflüssigkeit 3 wieder wahlweise in das als Flüssigkeitstank 15 ausgebildete Führungselement 8 oder die Behandlungszone 4 erfolgen. Alternativ können dem Flüssigkeitstank 15 und der Behandlungszone 4 jeweils festlegbare Teilmengen des filtrierten Stromes 46 der Prozessflüssigkeit 3 zugeführt werden. Zu diesem Zweck können die Verteilerelemente 45, 45 beispielsweise wiederum als Durchflussregelventile ausgestaltet sein.

Wiederum können einer Membranfiltrationsanlage 19 auch mehr als zwei Umschaltelemente 44 und/oder Verteilerelemente 45 zugeordnet sein, welche dementsprechend mit mehreren Führungselementen 8 und/oder mehreren Behandlungszonen 4 leitungsverbunden sein können.

Die Ausführungsbeispiele zeigen mögliche Ausführungsvarianten des Verfahrens bzw. der Vorrichtung zur Behandlung von Lebensmitteln und/oder Behältnissen, wobei an dieser Stelle bemerkt sei, dass die Erfindung nicht auf die speziell dargestellten Ausführungsvarianten derselben eingeschränkt ist, sondern vielmehr auch diverse Kombinationen der einzelnen Ausführungsvarianten untereinander möglich sind und diese Variationsmöglichkeit aufgrund der Lehre zum technischen Handeln durch gegenständliche Erfindung im Können des auf diesem technischen Gebiet tätigen Fachmannes liegt.

Die Erfindung wird durch die Merkmale der Ansprüche definiert.

Sämtliche Angaben zu Wertebereichen in gegenständlicher Beschreibung sind so zu verstehen, dass diese beliebige und alle Teilbereiche daraus mit umfassen, z.B. ist die Angabe 1 bis 10 so zu verstehen, dass sämtliche Teilbereiche, ausgehend von der unteren Grenze 1 und der oberen Grenze 10, mit umfasst sind, d.h. sämtliche Teilbereiche beginnen mit einer unteren Grenze von 1 oder größer und enden bei einer oberen Grenze von 10 oder weniger, z.B. 1 bis 1,7 oder 3,2 bis 8,1 oder 5,5 bis 10.

Der Ordnung halber sei abschließend darauf hingewiesen, dass zum besseren Verständnis des Aufbaus der Vorrichtung zur Behandlung von Lebensmitteln und/oder Behältnissen diese bzw. deren Bestandteile teilweise unmaßstäblich und/oder vergrößert und/oder verkleinert dargestellt wurden.

**Bezugszeichenaufstellung**

| | | | |
|---|---|---|---|
| 1 | Vorrichtung | 30 | Druckbehälter |
| 2 | Behältnis | 31 | Filtermembranmodul |
| 3 | Prozessflüssigkeit | 32 | Vorlagebehälter |
| 4 | Behandlungszone | 33 | Filtrat |
| 5 | Flüssigkeitsstrom | 34 | Absperrmittel |
| 6 | Außenseite | 35 | Rückspülleitung |
| 7 | Sprühdüse | 36 | Richtung |
| 8 | Führungselement | 37 | Flüssigkeitsabfallleitung |
| 9 | Sammelwanne | 38 | Dosiereinrichtung |
| 10 | Transportmittel | 39 | Chemikalienquelle |
| 11 | Fördermittel | 40 | Adsorptionsvorrichtung |
| 12 | Heizmittel | 41 | Sensor |
| 13 | Kühlmittel | 42 | Umschaltmittel |
| 14 | Flüssigkeitstank | 43 | Mischmittel |
| 15 | Flüssigkeitstank | 44 | Umschaltelement |
| 16 | Entleerungsvorrichtung | 45 | Verteilerelement |
| 17 | Zufuhrvorrichtung | 46 | Strom |
| 18 | Durchflussregeleinrichtung | 47 | Durchflussbehälter |
| 19 | Membranfiltrationsanlage | | |
| 20 | Strom | | |
| 21 | Verteilungsmittel | | |
| 22 | Zuleitungselement | | |
| 23 | Drei-Wege-Verteilerventil | | |
| 24 | Ableitungselement | | |
| 25 | Öffnung | | |
| 26 | Transportrichtung | | |
| 27 | Gebläse | | |
| 28 | Innenseite | | |
| 29 | Drei-Wege-Ventil | | |

## Patentansprüche

1. Verfahren zur Behandlung von Lebensmitteln in zumindest einer Behandlungszone (4), wobei die zu behandelnden Lebensmittel vor der Behandlung in Behältnisse (2) abgefüllt werden, die Behältnisse (2) verschlossen werden, und die Behältnisse (2) in eine Behandlungszone (4) eingebracht und/oder durch eine Behandlungszone (4) befördert werden, und wobei der Behandlungszone (4) oder den Behandlungszonen (4) jeweils zumindest ein Flüssigkeitsstrom (5) einer Prozessflüssigkeit (3) zum Einwirken auf die Behältnisse (2) zugeführt wird, wobei die Prozessflüssigkeit (3) eine Außenseite (6) der Behältnisse (2) umströmt, und wobei die Prozessflüssigkeit (3) temperiert wird und die Behandlung der Lebensmittel in einer Behandlungszone (4) durch Wärmeübertragung mittels der Prozessflüssigkeit (3) durchgeführt wird und in zumindest einer Behandlungszone (4) die Lebensmittel mittels einer erhitzten Prozessflüssigkeit (3) pasteurisiert werden, und wobei die Prozessflüssigkeit (3) nach erfolgter Behandlung der Lebensmittel aus der Behandlungszone (4) wieder abgeführt wird, und wobei die Prozessflüssigkeit (3) zur Behandlung der Lebensmittel zwecks Wiederverwendung im Verfahren im Kreis wieder in die Behandlungszone (4) oder in die Behandlungszonen (4) geführt wird, **dadurch gekennzeichnet, dass** im kontinuierlichen Behandlungsbetrieb aus der pro Zeiteinheit insgesamt im Kreis durch alle vorhandenen Behandlungszonen (4) geführten Prozessflüssigkeit (3) pro Zeiteinheit eine Teilmenge der Prozessflüssigkeit (3) zur Bildung wenigstens eines Stroms (20) der Prozessflüssigkeit (3) herangezogen wird, und der wenigstens eine gebildete Strom (20) der Prozessflüssigkeit (3) zur Reinigung der Prozessflüssigkeit (3) mittels zumindest einer Membranfiltrationsanlage (19) filtriert wird, und ein filtrierter Strom (46) nach dem Filtrationsvorgang zumindest teilweise einem die Prozessflüssigkeit (3) beinhaltenden und/oder führenden Element (8) oder einer Behandlungszone (4) wieder zugeführt wird, wobei mittels zumindest eines verstellbaren Verteilungsmittels (21) oder mehrerer zusammenwirkender Verteilungsmittel (21) pro Zeiteinheit eine festlegbare Menge der Prozessflüssigkeit (3) aus zumindest einem die Prozessflüssigkeit (3) beinhaltenden und/oder führenden Element (8) zur Bildung des wenigstens einen Stromes (20) der Prozessflüssigkeit (3) entnommen wird, und wobei die im kontinuierlichen Behandlungsbetrieb aus zumindest einem die Prozessflüssigkeit (3) beinhaltenden und/oder führenden Element (8) pro Zeiteinheit insgesamt zur Bildung wenigstens eines Stromes (20) der Prozessflüssigkeit (3) herangezogene Prozessflüssigkeitsmenge so gewählt wird, dass bezogen auf die pro Zeiteinheit insgesamt durch alle vorhandenen Behandlungszonen (4) geführte Menge an Prozessflüssigkeit (3) pro Zeiteinheit zumindest 1 % und weniger als 25 % zur Bildung wenigstens eines Stromes (20) der Prozessflüssigkeit (3) herangezogen werden, und diese pro Zeiteinheit insgesamt gebildete Teilmenge der Prozessflüssigkeit (3) mittels zumindest einer Membranfiltrationsanlage (19) filtriert wird.

2. Verfahren nach Anspruch 1, **dadurch gekennzeichnet, dass** die Temperaturen der jeweiligen Flüssigkeitsströme (5) der Prozessflüssigkeit (3) vor der Zuführung in eine Behandlungszone (4) separat für jede Behandlungszone (4) eingestellt werden.

3. Verfahren nach einem der vorangehenden Ansprüche, **dadurch gekennzeichnet, dass** die zu behandelnden Lebensmittel aufeinanderfolgend in zumindest einer Behandlungszone (4) erwärmt werden, in zumindest einer Behandlungszone (4) pasteurisiert werden und in zumindest einer Behandlungszone (4) abgekühlt werden.

4. Verfahren nach Anspruch 3, **dadurch gekennzeichnet, dass** ein Flüssigkeitsstrom (5) der Prozessflüssigkeit (3) wenigstens einer Behandlungszone (4) zum Erwärmen der Lebensmittel und/oder Behältnisse (2) bei einer Temperatur zwischen 40 °C und 50 °C zugeführt wird.

5. Verfahren nach einem der vorangehenden Ansprüche, **dadurch gekennzeichnet, dass** die im kontinuierlichen Behandlungsbetrieb aus zumindest einem die Prozessflüssigkeit (3) beinhaltenden und/oder führenden Element (8) pro Zeiteinheit insgesamt zur Bildung wenigstens eines Stromes (20) der Prozessflüssigkeit (3) herangezogene Prozessflüssigkeitsmenge so gewählt wird, dass durch die Filtration des Stromes (20) oder der Ströme (20) eine Entfernungsrate für Mikroorganismen erzielbar ist, welche größer ist, als die Wachstumsrate der Mikroorganismen in der Prozessflüssigkeit (3) im gleichen Zeitintervall.

6. Verfahren nach einem der vorangehenden Ansprüche, **dadurch gekennzeichnet, dass** im kontinuierlichen Behandlungsbetrieb das insgesamt in einer Vorrichtung (1) zur Behandlung von Lebensmitteln und/oder Behältnissen (2) enthaltene Volumen an Prozessflüssigkeit (3) pro Tag zumindest 1 Mal und bevorzugt zwischen 2 Mal und 10 Mal über eine oder mehrere Membranfiltrationsanlage(n) (19) geführt und filtriert wird.

7. Verfahren nach einem der vorangehenden Ansprüche, **dadurch gekennzeichnet, dass** Prozessflüssigkeit (3) zur Bildung wenigstens eines zu filtrierenden Stromes (20) bei einer Temperatur der Prozessflüssigkeit (3) von weniger als 80°C und insbesondere weniger als 50 °C herangezogen und/oder entnommen werden.

8. Verfahren nach einem der Ansprüche 3 bis 7, **dadurch gekennzeichnet, dass** zur Bildung wenigstens eines zu filtrierenden Stromes (20) der Prozessflüssigkeit, Prozessflüssigkeit (3) mit einer Temperatur zwischen 40 °C und 50 °C herangezogen wird.

9. Verfahren nach einem der vorangehenden Ansprüche, **dadurch gekennzeichnet, dass** ein filtrierter Strom (46) der Prozessflüssigkeit (3) bei Umgebungsdruck in freiem Gefälle wieder in zumindest ein die Prozessflüssigkeit (3) beinhaltendes und/oder führendes Element (8) und/oder in eine Behandlungszone (4) zurückgeführt wird.

10. Verfahren nach einem der vorangehenden Ansprüche, **dadurch gekennzeichnet, dass** ein filtrierter Strom (46) der Prozessflüssigkeit (3) zumindest teilweise einer im Verfahren zur Behandlung von in verschlossenen Behältnissen (2) abgefüllten Lebensmitteln am Ende des Prozesses angeordneten Behandlungszone (4) zur Spülung der Außenseite (6) der mit Lebensmitteln befüllten und verschlossenen Behältnissen (2) zugeführt wird.

11. Verfahren nach einem der vorangehenden Ansprüche, **dadurch gekennzeichnet, dass** ein filtrierter Strom (46) der Prozessflüssigkeit (3) zumindest teilweise einer im Verfahren zur Behandlung von in verschlossenen Behältnissen (2) abgefüllten Lebensmitteln eingangs angeordneten Behandlungszone (4) zur Reinigung der Innenseite (28) und der Außenseite (6) von ungefüllten Behältnissen (2) zugeführt wird.

12. Verfahren nach einem der vorangehenden Ansprüche, **dadurch gekennzeichnet, dass** ein Strom (46) der Prozessflüssigkeit (3) nach Filtration mittels der zumindest einen Membranfiltrationsanlage (19) in einen Vorlagebehälter (32) geführt wird und via einem am Vorlagebehälter (32) ausgestalteten Überlauf wieder in zumindest ein die Prozessflüssigkeit (3) beinhaltendes und/oder führendes Element (8) und/oder in eine Behandlungszone (4) zurückgeführt wird.

13. Verfahren nach Anspruch 12, **dadurch gekennzeichnet, dass** in festlegbaren Zeitintervallen eine Membranfiltrationsanlage (19) zwecks Reinigung von Filtermembranen im laufenden Betrieb vom Rest der Vorrichtung (1) zur Behandlung von in verschlossenen Behältnissen (2) abgefüllten Lebensmitteln strömungstechnisch getrennt wird und das in dem Vorlagebehälter (32) gesammelte Filtrat (33) der Prozessflüssigkeit (3) unter Umkehrung der Flussrichtung über die Filtermembranen, im Vergleich zum Filtrationsbetrieb, durch eine Membranfiltrationsanlage (19) geführt wird.

14. Verfahren nach Anspruch 13, **dadurch gekennzeichnet, dass** das im Zuge der Reinigung unter Umkehrung der Flussrichtung über die Filtermembranen der Membranfilteranlage (19) anfallenden Flüssigkeitsabfall abgeführt wird und durch eine entsprechende Menge an frischer Prozessflüssigkeit (3) ersetzt wird.

15. Verfahren nach einem der vorangehenden Ansprüche, **dadurch gekennzeichnet, dass** bei Bedarf sowohl im Behandlungsbetrieb und Filtrationsbetrieb, als auch im Reinigungsbetrieb für die zumindest eine Membranfiltrationsanlage (19) einem zu filtrierenden Strom (20) der Prozessflüssigkeit (3) und/oder einem filtrierten Strom (46) der Prozessflüssigkeit (3) mittels einer Dosiereinrichtung (38) Chemikalien aus einer oder mehreren Chemikalienquellen (39) beigemengt werden.

16. Verfahren nach einem der vorangehenden Ansprüche, **dadurch gekennzeichnet, dass** der Verunreinigungsgrad insbesondere durch Messen der Trübung der Prozessflüssigkeit (3) mittels Sensoren (41) in Führungselementen (8) einer Behandlungszone (4) und/oder in einer Behandlungszone (4) kontinuierlich überwacht wird.

17. Verfahren nach einem der vorangehenden Ansprüche, **dadurch gekennzeichnet, dass** ein Strom (20) der Prozessflüssigkeit (3) bedarfsabhängig aus unterschiedlichen, die Prozessflüssigkeit (3) beinhaltenden und/oder führenden Elementen (8) gebildet wird, mittels zumindest einer Membranfiltrationsanlage (19) filtriert wird und ein filtrierter Strom (46) nach dem Filtrationsvorgang in zumindest ein die Prozessflüssigkeit (3) beinhaltendes und/oder führendes Element (8) und/oder in zumindest eine Behandlungszone (4) zurückgeführt wird.

18. Verfahren nach Anspruch 17, **dadurch gekennzeichnet, dass** ein Strom (20) der Prozessflüssigkeit (3) zur Filtration mittels einer Membranfiltrationsanlage (19) durch Umschalten zwischen oder Mischen von unterschiedlichen Flüssigkeitsströmen (5) der Prozessflüssigkeit (3) aus verschiedenen Führungselementen (8) in Abhängigkeit von im Zuge von Inline-Messungen und/oder Stichprobenmessungen erhaltenen Messwerten gebildet, und ein filtrierter Strom (46) der Prozessflüssigkeit (3) in Abhängigkeit von im Zuge von Inline-Messungen und/oder Stichprobenmessungen erhaltenen Messwerten, durch Einleiten und/oder Verteilen des filtrierten Stromes (46) in verschiedene Flüssigkeitsströme (5) in verschiedenen Führungselementen (8) und/oder Behandlungszonen (4) zurückgeführt wird.

19. Verfahren nach einem der vorangehenden Ansprüche, **dadurch gekennzeichnet, dass** einer Behandlungszone (4) mehr als eine Membranfiltrationsanlage (19) zugeordnet wird und dass den einer jeweiligen Behandlungszone (4) zugeführten Flüssigkeitsströmen (5) der Prozessflüssigkeit (3) bedarfsabhängig eine bestimmte Menge zur Bildung eines Stromes (20) der Prozessflüssigkeit (3) entnommen, und ein Strom (20) mittels einer oder mehrerer jeweilig einer Behandlungszone (4) zugeordneter Membranfiltrationsanlage(n) (19) gefiltert wird.

20. Vorrichtung (1) für die Behandlung von in verschlossenen Behältnissen (2) befindlichen Lebensmitteln mittels einer Prozessflüssigkeit (3), umfassend zumindest eine Behandlungszone (4), welche Behandlungszone (4) zur Applikation der Prozessflüssigkeit (3) auf eine Außenseite (6) der verschlossenen Behältnisse (2) ausgestaltet ist, sodass die Prozessflüssigkeit (3) die Außenseite (6) eines verschlossenen Behältnisses (2) umströmt, Transportmittel (10) zum Transport der Behältnisse (2) durch die Behandlungszone(n) (4), sowie Führungselemente (8) zum Zuführen von Flüssigkeitsströmen (5) der Prozessflüssigkeit (3) in eine Behandlungszone (4) und Führungselemente (8) zur Abführung von Flüssigkeitsströmen (5) der Prozessflüssigkeit (3) aus einer Behandlungszone (4), weitere Führungselemente (8) zur Beinhaltung und/oder Führung der Prozessflüssigkeit (3) in der Vorrichtung (1) und wenigstens ein Fördermittel (11) zur Förderung von Flüssigkeitsströmen (5) der Prozessflüssigkeit (3) in den Führungselementen (8), wobei die Führungselemente (8) derart ausgestaltet und angeordnet sind, dass die Prozessflüssigkeit (3) zur Behandlung der Lebensmittel im Kreis wieder in die Behandlungszone (4) oder in die Behandlungszonen (4) geführt werden kann, wobei zumindest ein Heizmittel (12) zur Erwärmung der Prozessflüssigkeit (3) angeordnet ist, und wobei zumindest ein Kühlmittel (13) zur Abkühlung der Prozessflüssigkeit (3) angeordnet ist, **dadurch gekennzeichnet, dass** die Vorrichtung (1) zumindest eine Membranfiltrationsanlage (19) umfasst, welche zumindest eine Membranfiltrationsanlage (19) derart mit den Führungselementen (8) und/oder mit den Behandlungszonen (4) strömungstechnisch leitungsverbunden ist, dass eine Teilmenge der pro Zeiteinheit insgesamt im Kreis durch alle vorhandenen Behandlungszonen (4) geführten Prozessflüssigkeit (3) zur Bildung wenigstens eines Stromes (20) der Prozessflüssigkeit (3) herangezogen, der gebildete Strom (20) oder die gebildeten Ströme (20) mittels der zumindest einen Membranfiltrationsanlage (19) filtriert, und ein filtrierter Strom (46) der Prozessflüssigkeit (3) einem Führungselement (8) oder einer Behandlungszone (4) wieder zugeführt werden kann, wobei die zumindest eine Membranfiltrationsanlage (19) Bypass-artig zwischen einem einen Flüssigkeitsstrom (5) führenden Führungselement (8) und einer Behandlungszone (4) oder einem weiteren Führungselement (8) angeordnet ist, und wobei ein Zuleitungselement (22) der zumindest einen Membranfiltrationsanlage (19) mit einem Führungselement (8) oder mit einer Behandlungszone (4) strömungstechnisch leitungsverbunden ist, und wobei ein Ableitungselement (24) der zumindest einen Membranfiltrationsanlage (19) mit einem Führungselement (8) oder einer Behandlungszone (4) leitungsverbunden ist, und wobei eingangsseitig der zumindest einen Membranfiltrationsanlage (19) zumindest ein verstellbares Verteilungsmittel (21) und/oder mehrere zusammenwirkende Verteilungsmittel (21) zur Entnahme einer festlegbaren Prozessflüssigkeitsmenge pro Zeiteinheit aus zumindest einem die Prozessflüssigkeit (3) beinhaltenden Führungselement (8) und zur Bildung des wenigstens einen zu filtrierenden Stromes (20) der Prozessflüssigkeit (3) angeordnet sind.

21. Vorrichtung nach Anspruch 20, **dadurch gekennzeichnet, dass** aufeinanderfolgend in Transportrichtung (26) der Lebensmittel oder Behältnisse (2) zumindest eine Behandlungszone (4) zur Erwärmung der Lebensmittel und/oder Behältnisse, zumindest eine Behandlungszone (4) zur Pasteurisierung der Lebensmittel, und zumindest eine Behandlungszone (4) zur Abkühlung der Lebensmittel und/oder Behältnisse angeordnet sind.

22. Vorrichtung nach Anspruch 20 oder 21, **dadurch gekennzeichnet, dass** die Anzahl und die Filtrationskapazität der Membranfiltrationsanlage(n) (19) derart festgelegt sind, dass die im kontinuierlichen Behandlungsbetrieb aus zumindest einem die Prozessflüssigkeit (3) beinhaltenden und/oder führenden Element (8) pro Zeiteinheit insgesamt zur Bildung wenigstens eines Stromes (20) der Prozessflüssigkeit (3) herangezogene Prozessflüssigkeitsmenge so gewählt werden kann, dass durch die Filtration des Stromes (20) oder der Ströme (20) eine Entfernungsrate für Mikroorganismen erzielbar ist, welche größer ist als die Wachstumsrate der Mikroorganismen in der Prozessflüssigkeit (3) im gleichen Zeitintervall.

23. Vorrichtung nach einem oder mehreren der Ansprüche 20 bis 22, **dadurch gekennzeichnet, dass** die Anzahl und die Filtrationskapazität der Membranfiltrationsanlage(n) (19) derart festgelegt sind, dass bezogen auf die pro Zeiteinheit insgesamt durch alle vorhandenen Behandlungszonen (4) geführte Menge an Prozessflüssigkeit (3) pro Zeiteinheit zumindest 1 % und weniger als 25 % zur Bildung wenigstens eines Stromes (20) herangezogen werden können und diese pro Zeiteinheit gebildete Teilmenge der Prozessflüssigkeit (3) mittels der Membranfiltrationsanlage(n) (19) filtriert werden kann.

24. Vorrichtung nach einem oder mehreren der Ansprüche 20 bis 23, **dadurch gekennzeichnet, dass** die Anzahl und die Filtrationskapazität der Membranfiltrationsanlage(n) (19) derart festgelegt sind, dass das insgesamt in der Vorrichtung (1) zur Behandlung von Lebensmitteln und/oder Behältnissen (2) enthaltene Volumen an Prozessflüssigkeit (3) pro Tag zumindest 1 Mal und bevorzugt zwischen 2 Mal und 10 Mal mittels der Membranfiltrationsanlage(n) (19) filtriert werden kann.

25. Vorrichtung nach einem oder mehreren der Ansprüche 20 bis 24, **dadurch gekennzeichnet, dass** die zumindest eine Membranfiltrationsanlage (19) für die Bildung eines zu filtrierenden Stromes (20) der Prozessflüssigkeit (3) an solchen Stellen mit Führungselementen (8) strömungstechnisch leitungsverbunden ist, an welchen die in den Führungselementen (8) beinhaltete Prozessflüssigkeit (3) eine Temperatur von weniger als 80°C und insbesondere weniger als 50 °C aufweist.

26. Vorrichtung nach einem oder mehreren der Ansprüche 20 bis 25, **dadurch gekennzeichnet, dass** zur Rückführung eines filtrierten Stromes (46) der Prozessflüssigkeit (3) nach erfolgter Filtration Ableitungselemente (24) von zumindest einer Membranfiltrationsanlage (19) mit zumindest einem Führungselement (8) und/oder zumindest einer Behandlungszone (4) derart leitungsverbunden sind, dass der zumindest eine filtrierte Strom (46) der Prozessflüssigkeit (3) dem oder den Führungselement(en) (8) und/oder der oder den Behandlungszone(n) (4) unter Einwirkung von Schwerkraft in freiem Gefälle zugeführt werden kann.

27. Vorrichtung nach einem oder mehreren der Ansprüche 20 bis 26, **dadurch gekennzeichnet, dass** zumindest eine Behandlungszone (4) zur Spülung der Außenseite (6) von mit Lebensmitteln befüllten und verschlossenen Behältnissen (2) ausgestaltet ist, welche Behandlungszone (4) bezogen auf die Transportrichtung (26) der Behältnisse (2) durch die Behandlungszonen (4) am Ende der Behandlungszonenstrecke angeordnet ist und welche Behandlungszone (4) zur Spülung der Behältnisse (2) durch Zuführung eines filtrierten Stromes (46) der Prozessflüssigkeit (3) mit zumindest einem Ableitungselement (24) einer Membranfiltrationsanlage (19) leitungsverbunden ist.

28. Vorrichtung nach einem oder mehreren der Ansprüche 20 bis 27, **dadurch gekennzeichnet, dass** zumindest eine Behandlungszone (4) zur Reinigung der Innenseite (28) und der Außenseite (6) von ungefüllten und unverschlossenen Behältnissen (2) ausgestaltet ist, welche Behandlungszone (4) bezogen auf die Transportrichtung (26) der Behältnisse (2) durch die Behandlungszonen (4) eingangs der Behandlungszonenstrecke angeordnet ist und zur Reinigung der Behältnisse (2) durch Zuführung eines filtrierten Stromes (46) der Prozessflüssigkeit (3) mit zumindest einem Ableitungselement (24) einer Membranfiltrationsanlage (19) leitungsverbunden ist.

29. Vorrichtung nach einem oder mehreren der Ansprüche 20 bis 28, **dadurch gekennzeichnet, dass** in einem Ableitungselement (24) der zumindest einen Membranfiltrationsanlage (19) ein Vorlagebehälter (32) mit Überlauf ausgestaltet ist.

30. Vorrichtung nach Anspruch 29, **dadurch gekennzeichnet, dass** zwecks Reinigung einer Membranfiltrationsanlage (19) Absperrmittel (34) in den Zuleitungselementen (22) und den Ableitungselementen (24) zur strömungstechnischen Abtrennung der zumindest einen Membranfiltrationsanlage (19) vom Rest der Vorrichtung (1) ausgestaltet sind und in dem Vorlagebehälter (32) und/oder einer sich zwischen dem Vorlagebehälter (32) und dem Ableitungselement (24) der Membranfiltrationsanlage (19) erstreckenden Rückspülleitung (35) zumindest ein Fördermittel (11) angeordnet ist, welches zur Förderung des in dem Vorlagebehälter (32) gesammelten Filtrats (33) der Prozessflüssigkeit (3) in, verglichen mit der Flussrichtung über die Filtermembranen im Filtrationsbetrieb, gegenläufiger Richtung (36) durch die zumindest eine Membranfiltrationsanlage (19) angeordnet ist.

31. Vorrichtung nach Anspruch 30, **dadurch gekennzeichnet, dass** zur Abführung eines im Zuge der Reinigung unter Umkehrung der Flussrichtung über die Filtermembranen der Membranfilteranlage (19) anfallenden Flüssigkeitsabfalls der Membranfiltrationsanlage (19) zumindest eine absperrbare Flüssigkeitsabfallleitung (37) zugeordnet ist und in der Vorrichtung (1) zumindest eine absperrbare Zufuhrvorrichtung (17) zum Ersetzen des abgeführten Flüssigkeitsabfalls durch frische Prozessflüssigkeit (3) angeordnet ist.

32. Vorrichtung nach einem oder mehreren der Ansprüche 20 bis 31, **dadurch gekennzeichnet, dass** in einem Ableitungselement (24) und/oder in der Rückspülleitung (35) der zumindest einen Membranfiltrationsanlage (19) eine Dosiereinrichtung (38) angeordnet ist, mittels welcher der Prozessflüssigkeit (3) und/oder einem Filtrat (33) der Prozessflüssigkeit (3), sowohl im Filtrationsbetrieb, als auch im Reinigungsbetrieb für die Membranfiltrationsanlage (19) Chemikalien aus einer oder mehreren Chemikalienquellen (39) beigemengt werden können.

33. Vorrichtung nach einem oder mehreren der Ansprüche 20 bis 32, **dadurch gekennzeichnet, dass** in Führungselementen (8) und/oder in Behandlungszonen (4) Sensoren (41) zur kontinuierlichen Überwachung des Verunreinigungsgrades, insbesondere durch Messen der Trübung der Prozessflüssigkeit (3), angeordnet sind.

34. Vorrichtung nach einem oder mehreren der Ansprüche 20 bis 33, **dadurch gekennzeichnet, dass** einem Zuleitungselement (22) einer Membranfiltrationsanlage (19) zumindest ein Umschaltmittel (42) zugeordnet ist, welches mit wenigstens zwei verschiedenen, die Prozessflüssigkeit (3) beinhaltenden, Führungselementen (8) strömungstechnisch derart leitungsverbunden ist, dass eine Bildung des zu filtrierenden Stromes (20) der Prozessflüssigkeit (3) wahlweise aus einem der Flüssigkeitsströme (5) oder mehreren Flüssigkeitsströmen (5) der Prozessflüssigkeit (3) in den Führungselementen (8) oder aus mehreren Flüssigkeitsströmen (5) der Prozessflüssigkeit (3) erfolgen kann.

35. Vorrichtung nach einem oder mehreren der Ansprüche 20 bis 34, **dadurch gekennzeichnet, dass** einem Zuleitungselement (22) einer Membranfiltrationsanlage (19) zumindest ein Mischmittel (43) zugeordnet ist, welches mit wenigstens zwei verschiedenen die Prozessflüssigkeit (3) beinhaltenden Führungselementen (8) strömungstechnisch derart leitungsverbunden ist, dass eine Bildung des zu filtrierenden Stromes (20) der Prozessflüssigkeit (3) wahlweise aus einem der Flüssigkeitsströme (5) oder mehreren Flüssigkeitsströmen (5) der Prozessflüssigkeit (3) in den Führungselementen (8) erfolgen kann oder die Bildung des zu filtrierenden Stromes (20) der Prozessflüssigkeit (3) durch die Membranfiltrationsanlage (19) durch Entnahme und Mischung festlegbarer Teilmengen aus mehreren Flüssigkeitsströmen (5) der Prozessflüssigkeit (3) erfolgen kann.

36. Vorrichtung nach einem oder mehreren der Ansprüche 20 bis 35, **dadurch gekennzeichnet, dass** einem Ableitungselement (24) einer Membranfiltrationsanlage (19) zumindest ein Umschaltelement (44) zugeordnet ist, welches mit wenigstens einem die Prozessflüssigkeit (3) beinhaltenden Führungselement (8) und/oder wenigstens einer Behandlungszone (4) strömungstechnisch derart leitungsverbunden ist, dass die Zuführung eines filtrierten Stromes (46) der Prozessflüssigkeit (3) in das wenigstens eine Führungselement (8) und/oder die wenigstens eine Behandlungszone (4) kontrolliert festlegbar ist.

37. Vorrichtung nach einem oder mehreren der Ansprüche 20 bis 36, **dadurch gekennzeichnet, dass** einem Ableitungselement (24) einer Membranfiltrationsanlage (19) zumindest ein Verteilerelement (45) zugeordnet ist, welches mit wenigstens einem die Prozessflüssigkeit (3) beinhaltenden Führungselement (8) und/oder wenigstens einer Behandlungszone (4) strömungstechnisch derart leitungsverbunden ist, dass eine Zuführung eines filtrierten Stromes (46) der Prozessflüssigkeit (3) in das wenigstens eine Führungselement (8) und/oder die wenigstens eine Behandlungszone (4) kontrolliert festlegbar ist oder festlegbare Mengen des filtrierten Stromes (46) der Prozessflüssigkeit (3) in das wenigstens eine Führungselement (8) und/oder die wenigstens eine Behandlungszone (4) zuführbar sind.

## Claims

1. A method for treating foods in at least one treatment zone (4), wherein the foods to be treated are filled into containers (2) before the treatment, the containers (2) are closed, and the containers (2) are introduced into a treatment zone (4) and/or conveyed through a treatment zone (4), and wherein the treatment zone (4) or the treatment zones (4) are each fed at least one liquid stream (5) of a process liquid (3) to act on the containers (2), wherein the process liquid (3) flows around an outside (6) of the containers (2), and wherein the process liquid (3) is tempered and the treatment of the foods is executed in a treatment zone (4) by heat transfer via the process liquid (3) and the foods are pasteurized by a heated process liquid (3) in at least one treatment zone (4), and wherein the process liquid (3) is drained again after completed treatment of the foods out of the treatment zone (4), and wherein the process liquid (3) for treating the foods is recirculated into the treatment zone (4) or the treatment zones (4) for reuse in the method, **characterized in that** during continuous treatment operation a partial quantity of the process liquid (3) out of the total process liquid (3) conducted through all existing treatment zones (4) per time unit is used per time unit to form at least one stream (20) of the process liquid (3), and the at least one formed stream (20) of the process liquid (3) is filtered by at least one membrane filtration system (19) in order to clean the process liquid (3), and after the filtration process a filtered stream (46) is at least partially fed back into an element (8) containing and/or conducting the process liquid (3) or into a treatment zone (4), wherein by means of at least one adjustable splitting means (21) or multiple cooperating splitting means (21), a specifiable quantity of the process liquid (3) is removed out of at least one element (8) containing and/or conducting the process liquid (3) per time unit in order to form the at least one stream (20) of the process liquid (3), and wherein the total process liquid quantity used per time unit to form at least one stream (20) of the process liquid (3) out of at least one element (8) containing and/or conducting the process liquid (3) during continuous treatment operation is selected in such a way that, based on the total quantity of process liquid (3) conducted through all existing treatment zones (4) per time unit, at least 1% and less than 25% is used to form at least one stream (20) of the process liquid (3) per time unit, and this total partial quantity of the process liquid (3) formed per time unit is filtered by means of at least one membrane filtration system (19).

2. The method according to claim 1, **characterized in that** the temperatures of the particular liquid streams (5) of the process liquid (3) are set separately for each treatment zone (4) before feeding into a treatment zone (4).

3. The method according to one of the preceding claims, **characterized in that** the foods to be treated are heated successively in at least one treatment zone (4), are pasteurized in at least one treatment zone (4), and are cooled in at least one treatment zone (4).

4. The method according to claim 3, **characterized in that** a liquid stream (5) of the process liquid (3) is fed into at least one treatment zone (4) for heating the foods and/or containers (2) at a temperature between 40 °C and 50 °C.

5. The method according to one of the preceding claims, **characterized in that** the process liquid quantity used to form at least one stream (20) of the process liquid (3) out of at least one element (8) containing and/or conducting the process liquid (3) during continuous treatment operation per time unit is selected in such a way that the filtration of the stream (20) or streams (20) allows for a removal rate for micro-organisms to be achieved that is larger than the growth rate of the micro-organisms in the process liquid (3) in the same time interval.

6. The method according to one of the preceding claims, **characterized in that** during continuous treatment operation the total volume of process liquid (3) contained in a device (1) for treating foods and/or containers (2) is conducted through one or more membrane filtration system(s) (19) and filtered at least 1 time and preferably between 2 times and 10 times per day.

7. The method according to one of the preceding claims, **characterized in that** process liquid (3) is taken and/or removed to form at least one stream (20) to be filtered when the process liquid (3) has a temperature of less than 80°C and in particular less than 50 °C.

8. The method according to one of claims 3 to 7, **characterized in that** process liquid (3) with a temperature of between 40 °C and 50 °C is used to form at least one stream (20) of the process liquid to be filtered.

9. The method according to one of the preceding claims, **characterized in that** a filtered stream (46) of the process liquid (3) is fed back into at least one element (8) containing and/or conducting the process liquid (3) and/or one treatment zone (4) at ambient pressure in free fall.

10. The method according to one of the preceding claims, **characterized in that** a filtered stream (46) of the process liquid (3) is at least partially fed into a treatment zone (4) arranged at the end of the process in the method for treating foods filled into closed containers (2) for rinsing the outside (6) of the closed containers (2) filled with foods.

11. The method according to one of the preceding claims, **characterized in that** a filtered stream (46) of the process liquid (3) is at least partially fed into a treatment zone (4) arranged at the start in the method for treating foods filled into closed containers (2) for cleaning the inside (28) and the outside (6) of unfilled containers (2).

12. The method according to one of the preceding claims, **characterized in that** a stream (46) of the process liquid (3) is conducted into a receiving container (32) after filtration by means of the at least one membrane filtration system (19) and recirculated into at least one element (8) containing and/or conducting the process liquid (3) and/or into a treatment zone (4) via an overflow arranged on the receiving container (32).

13. The method according to claim 12, **characterized in that** a membrane filtration system (19) is fluidically separated from the rest of the device (1) for treating foods filled into closed containers (2) at specifiable time intervals in order to clean filter membranes during ongoing operation and the filtrate (33) of the process liquid (3) collected in the receiving container (32) is conducted through a membrane filtration system (19) with reversed flow direction through the filter membranes in comparison to filtration operation.

14. The method according to claim 13, **characterized in that** liquid waste accrued during cleaning with reversed flow direction through the filter membranes of the membrane filtration system (19) is drained and replaced by an equivalent quantity of fresh process liquid (3).

15. The method according to one of the preceding claims, **characterized in that** chemicals from one or more chemical sources (39) are admixed into a stream (20) of the process liquid (3) to be filtered and/or a filtered stream (46) of the process liquid (3) by means of a dosing device (38) as needed both during treatment operation and filtration operation as well as in cleaning operation for the at least one membrane filtration system (19).

16. The method according to one of the preceding claims, **characterized in that** the degree of contamination is continuously monitored, in particular by measuring the turbidity of the process liquid (3) by means of sensors (41) in conduction elements (8) of a treatment zone (4) and/or in a treatment zone (4).

17. The method according to one of the preceding claims, **characterized in that** a stream (20) of the process liquid (3) is formed as needed out of different elements (8) containing and/or conducting the process liquid (3), is filtered by means of at least one membrane filtration system (19), and after the filtration process a filtered stream (46) is fed back into at least one element (8) containing and/or conducting the process liquid (3) and/or into at least one treatment zone (4).

18. The method according to claim 17, **characterized in that** a stream (20) of the process liquid (3) is formed for filtration by means of a membrane filtration system (19) by switching between or mixing different liquid streams (5) of the process liquid (3) from different conduction elements (8) depending on measured values obtained through in-line measurements and/or random sample measurements, and a filtered stream (46) of the process liquid (3) is fed back into different conduction elements (8) and/or treatment zones (4) by introducing and/or splitting the filtered stream (46) into different liquid streams (5) depending on measured values obtained through in-line measurements and/or random sample measurements.

19. The method according to one of the preceding claims, **characterized in that** a treatment zone (4) is assigned more than one membrane filtration system (19) and a certain quantity is removed from the liquid streams (5) of the process liquid (3) fed into a particular treatment zone (4) as needed to form a stream (20) of the process liquid (3), and a stream (20) is filtered by means of one or more membrane filtration system(s) (19) respectively assigned to a treatment zone (4).

20. A device (1) for treating foods in closed containers (2) with a process liquid (3), comprising at least one treatment zone (4), which treatment zone (4) is designed to apply the process liquid (3) to an outside (6) of the closed containers (2) such that the process liquid (3) flows around the outside (6) of a closed container (2), means of transport (10) for transporting the containers (2) through the treatment zone(s) (4), as well as conduction elements (8) for feeding liquid streams (5) of the process liquid (3) into a treatment zone (4) and conduction elements (8) for discharging liquid streams (5) of the process liquid (3) from a treatment zone (4), further conduction elements (8) for containing and/or conducting the process liquid (3) in the device (1) and at least one conveying means (11) for conveying liquid streams (5) of the process liquid (3) in the conduction elements (8), wherein the conduction elements (8) are designed and arranged such that the process liquid (3) for treating the foods can be recirculated again into the treatment zone (4) or into the treatment zones (4), wherein at least one heating means (12) for heating the process liquid (3) is arranged, and wherein at least one cooling means (13) for cooling the process liquid (3) is arranged, **characterized in that** the device (1) comprises at least one membrane filtration system (19), which at least one membrane filtration system (19) is fluidically line-connected to the conduction elements (8) and/or to the treatment zones (4) in such a way that a partial amount of the total process liquid (3) fed through all existing treatment zones (4) per time unit can be used to form at least one stream (20) of the process liquid (3), the formed stream (20) or the formed streams (20) can be filtered by means of the at least one membrane filtration system (19), and a filtered stream (46) of the process liquid (3) can be fed back into a conduction element (8) and/or a treatment zone (4), wherein the at least one membrane filtration system (19) is arranged between a conduction element (8) conducting a liquid stream (5) and a treatment zone (4) or a further conduction element (8) in bypass-like manner, and wherein a feeding element (22) of the at least one membrane filtration system (19) is fluidically line-connected to a conduction element (8) or a treatment zone (4), and wherein a draining element (24) of the at least one membrane filtration system (19) is line-connected to a conduction element (8) or a treatment zone (4), and wherein at least one adjustable splitting means (21) and/or multiple cooperating splitting means (21) are arranged on an inlet side of the at least one membrane filtration system (19) for removal of a specifiable process liquid quantity per time unit out of at least one conduction element (8) containing the process liquid (3) and for the formation of the at least one stream (20) of the process liquid (3) to be filtered.

21. The device according to claim 20, **characterized in that** at least one treatment zone (4) for heating the foods and/or containers, at least one treatment zone (4) for pasteurizing the foods, and at least one treatment zone (4) for cooling the foods and/or containers are arranged in succession in the transport direction (26) of the foods or containers (2).

22. The device according to claim 20 or 21, **characterized in that** the number and filtration capacity of the membrane filtration system(s) (19) are fixed such that the total process liquid drawn out of at least one element (8) containing and/or conducting the process liquid (3) per time unit for forming at least one stream (20) of the process liquid (3) during continuous treatment operation can be selected such that the filtration of the stream (20) or the streams (20) allows for a removal rate of micro-organisms to be achieved that is greater than the growth rate of the micro-organisms in the process liquid (3) in the same time interval.

23. The device according to one or more of claims 20 to 22, **characterized in that** the number and filtration capacity of the membrane filtration system(s) (19) are fixed such that, based on the total quantity of process liquid (3) conducted through all existing treatment zones (4) per time unit, at least 1% and less than 25% per time unit can be used to form at least one stream (20) and this partial quantity of the process liquid (3) formed per time unit can be filtered by means of the membrane filtration system(s) (19).

24. The device according to one or more of claims 20 to 23, **characterized in that** the number and filtration capacity of the membrane filtration system(s) (19) are fixed such that the total volume of process liquid (3) contained in the device (1) for treating foods and/or containers (2) can be filtered at least 1 time and preferably between 2 times and 10 times per day using the membrane filtration system(s) (19).

25. The method according to one or more of claims 20 to 24, **characterized in that** the at least one membrane filtration system (19) for forming a stream (20) of process liquid (3) to be filtered is fluidically line-connected to conduction elements (8) at positions where the process liquid (3) contained in the conduction elements (8) has a temperature of less than 80°C and in particular of less than 50 °C.

26. The device according to one or more of claims 20 to 25, **characterized in that** in order to recirculate a filtered stream (46) of the process liquid (3) after completed filtration, draining elements (24) out of at least one membrane filtration system (19) are line-connected to at least one conduction element (8) and/or at least one treatment zone (4) in such a way that the at least one filtered stream (46) of the process liquid (3) can be fed into the conduction element(s) (8) and/or the treatment zone(s) (4) under the influence of gravity in free fall.

27. The device according to one or more of claims 20 to 26, **characterized in that** at least one treatment zone (4) for rinsing the outside (6) of closed containers (2) filled with foods is provided, which treatment zone (4) is arranged at the end of the treatment zone line in the transport direction (26) of the containers (2) through the treatment zones (4) and which treatment zone (4) is line-connected to at least one draining element (24) of a membrane filtration system (19) in order to rinse the containers (2) by feeding in a filtered stream (46) of the process liquid (3).

28. The device according to one or more of claims 20 to 27, **characterized in that** at least one treatment zone (4) for cleaning the inside (28) and the outside (6) of unfilled and open containers (2) is provided, which treatment zone (4) is arranged at the start of the treatment zone line in the transport direction (26) of the containers (2) through the treatment zones (4) and is line-connected to at least one draining element (24) of a membrane filtration system (19) in order to clean the containers (2) by feeding in a filtered stream (46) of the process liquid (3).

29. The device according to one or more of claims 20 to 28, **characterized in that** a receiving container (32) with overflow is provided in a draining element (24) of the at least one membrane filtration system (19).

30. The device according to claim 29, **characterized in that** in order to clean a membrane filtration system (19), closing means (34) are provided in the feeding elements (22) and draining elements (24) to fluidically separate the at least one membrane filtration system (19) from the rest of the device (1) and at least one conveying means (11) is provided in the receiving container (32) and/or a back-flushing line (35) extending between the receiving container (32) and the draining element (24) of the membrane filtration system (19) that is designed to convey the filtrate (33) of the process liquid (3) collected in the receiving container (32) in the opposite direction (36) - as compared to the flow direction through the filter membranes during filtration operation - through the at least one membrane filtration system (19).

31. The device according to claim 30, **characterized in that** to discharge the liquid waste accrued in the course of cleaning with reversed flow direction through the filter membranes of the membrane filtration system (19), at least one closable liquid waste line (37) is assigned to the membrane filtration system (19), and at least one closable feed device (17) is arranged in the device (1) to replace the discharged liquid waste with fresh process liquid (3).

32. The device according to one or more of claims 20 to 31, **characterized in that** a dosing device (38) is arranged in a draining element (24) and/or in the back-flushing line (35) of the at least one membrane filtration system (19) by means of which chemicals from one or more chemical sources (39) can be admixed to the process liquid (3) and/or a filtrate (33) of the process liquid (3) both during filtration operation and cleaning operation the membrane filtration system (19).

33. The device according to one or more of claims 20 to 32, **characterized in that** sensors (41) are arranged in conduction elements (8) and/or in treatment zones (4) to continuously monitor the degree of contamination, in particular by measuring the turbidity of the process liquid (3).

34. The device according to one or more of claims 20 to 33, **characterized in that** at least one switching means (42) is assigned to a feeding element (22) of a membrane filtration system (19) that is fluidically line-connected to at least two different conduction elements (8) containing the process liquid (3) in such a way that the stream (20) of process liquid (3) to be filtered can be formed as desired either from one of the liquid streams (5) or multiple liquid streams (5) of the process liquid (3) in the conduction elements (8) or from multiple liquid streams (5) of the process liquid (3).

35. The device according to one or more of claims 20 to 34, **characterized in that** at least one mixing means (43) is assigned to a feeding element (22) of a membrane filtration system (19) that is fluidically line-connected to at least two different conduction elements (8) containing the process liquid (3) in such a way that the stream (20) of process liquid (3) to be filtered can be formed as desired either from one of the liquid streams (5) or multiple liquid streams (5) of the process liquid (3) in the conduction elements (8) or a stream (20) of the process liquid (3) to be filtered by the membrane filtration system (19) can be formed by removing and mixing specifiable partial quantities from multiple liquid streams (5) of the process liquid (3).

36. The device according to one or more of claims 20 to 35, **characterized in that** at least one switching means (44) is arranged in a draining element (24) of a membrane filtration system (19) that is fluidically line-connected to at least one conduction element (8) containing the process liquid (3) and/or at least one treatment zone (4) in such a way that feeding a filtered stream (46) of the process liquid (3) into the at least one conduction element (8) and/or the at least one treatment zone (4) can be determined in a controlled manner.

37. The device according to one or more of claims 20 to 36, **characterized in that** at least one splitting means (45) is assigned to a draining element (24) of a membrane filtration system (19) which is fluidically line-connected to at least one conduction element (8) containing the process liquid (3) and/or at least one treatment zone (4) in such a way that feeding a filtered stream (46) of the process liquid (3) into the at least one conduction element (8) and/or the at least one treatment zone (4) can be determined in a controlled manner or specifiable quantities of the filtered stream (46) of process liquid (3) can be fed into the at least one conduction element (8) and/or the at least one treatment zone (4).

## Revendications

1. Procédé de traitement de produits alimentaires dans au moins une zone de traitement (4), dans lequel les produits alimentaires à traiter sont placés dans des récipients (2) avant le traitement, les récipients (2) sont fermés et les récipients (2) sont introduits dans une zone de traitement (4) et/ou transportés à travers une zone de traitement (4), et dans lequel respectivement au moins un flux de liquide (5) d'un liquide de process (3) est acheminé à la zone de traitement (4) ou aux zones de traitement (4) pour agir sur les récipients (2), dans lequel le liquide de process (3) s'écoule autour d'un côté extérieur (6) des récipients (2), et dans lequel le liquide de process (3) est tempéré, et le traitement des produits alimentaires est effectué dans une zone de traitement (4) par transmission de chaleur au moyen du liquide de process (3), et les produits alimentaires sont pasteurisés dans au moins une zone de traitement (4) au moyen d'un liquide de process (3) chauffé et dans lequel, une fois le traitement des produits alimentaires effectué, le liquide de process (3) est de nouveau évacué de la zone de traitement (4), et dans lequel, en vue de la réutilisation en boucle dans le procédé, le liquide de process (3) destiné au traitement des produits alimentaires est conduit de nouveau dans la zone de traitement (4) ou dans les zones de traitement (4), **caractérisé en ce que**, dans le mode de traitement continu, une quantité partielle du liquide de process (3) est utilisée par unité de temps pour la formation d'au moins un flux (20) du liquide de process (3) à partir du liquide de process (3) conduit par unité de temps au total en boucle à travers toutes les zones de traitement (4) existantes, et le au moins un flux (20) formé du liquide de process (3) est filtré pour le nettoyage du liquide de process (3) au moyen d'au moins une installation de filtration par membrane (19), et, après le processus de filtration, un flux filtré (46) est de nouveau acheminé au moins partiellement à un élément (8) contenant et/ou conduisant le liquide de process (3) ou à une zone de traitement (4), dans lequel, au moyen d'au moins un moyen de répartition (21) réglable ou de plusieurs moyens de répartition (21) qui coopèrent, une quantité pouvant être fixée du liquide de process (3) est prélevée par unité de temps à partir d'au moins un élément (8) contenant et/ou conduisant le liquide de process (3) pour la formation du au moins un flux (20) du liquide de process (3), et dans lequel la quantité de liquide de process utilisée dans le mode de traitement continu à partir d'au moins un élément (8) contenant et/ou conduisant le liquide de process (3) par unité de temps au total pour la formation d'au moins un flux (20) du liquide de process (3) est choisie de telle sorte que, rapporté à la quantité de liquide de process (3) conduite par unité de temps au total à travers toutes les zones de traitement (4) existantes, au moins 1% et moins de 25 % sont utilisés par unité de temps pour la formation d'au moins un flux (20) du liquide de process (3), et cette quantité partielle du liquide de process (3) formée au total par unité de temps est filtrée au moyen d'au moins une installation de filtration par membrane (19).

2. Procédé selon la revendication 1, **caractérisé en ce que** les températures des flux de liquide (5) respectifs du liquide de process (3) sont réglées séparément pour chaque zone de traitement (4) avant l'acheminement dans une zone de traitement (4).

3. Procédé selon l'une des revendications précédentes, **caractérisé en ce que** les produits alimentaires à traiter sont chauffés les uns après les autres dans au moins une zone de traitement (4), sont pasteurisés dans au moins une zone de traitement (4) et sont refroidis dans au moins une zone de traitement (4).

4. Procédé selon la revendication 3, **caractérisé en ce qu'**un flux de liquide (5) du liquide de process (3) est conduit au moins à une zone de traitement (4) pour le chauffage des produits alimentaires et/ou des récipients (2) à une température entre 40 °C et 50 °C.

5. Procédé selon l'une des revendications précédentes, **caractérisé en ce que** la quantité de liquide de process utilisée dans le mode de traitement continu à partir d'au moins un élément (8) contenant et/ou conduisant le liquide de process (3) par unité de temps au total pour la formation d'au moins un flux (20) du liquide de process (3) est choisie de telle sorte que, par la filtration du flux (20) ou des flux (20), il est possible d'atteindre un taux d'enlèvement pour des microorganismes qui est supérieur au taux de croissance des microorganismes dans le liquide de process (3) dans le même intervalle de temps.

6. Procédé selon l'une des revendications précédentes, **caractérisé en ce que**, dans le mode de traitement continu, le volume de liquide de process (3) contenu au total dans un dispositif (1) de traitement de produits alimentaires ou de récipients (2) est conduit et filtré au moins 1 fois et de préférence entre 2 fois et 10 fois par jour par le biais d'une ou de plusieurs installation(s) de filtration par membrane (19).

7. Procédé selon l'une des revendications précédentes, **caractérisé en ce que**, pour la formation d'au moins un flux (20) à filtrer, du liquide de process (3) est utilisé et/ou prélevé à une température du liquide de process (3) inférieure à 80 °C et en particulier inférieure à 50 °C.

8. Procédé selon l'une des revendications 3 à 7, **caractérisé en ce que**, pour la formation d'au moins un flux (20) à filtrer du liquide de process, il est utilisé du liquide de process (3) à une température entre 40 °C et 50 °C.

9. Procédé selon l'une des revendications précédentes, **caractérisé en ce qu'**un flux filtré (46) du liquide de process (3) est, à la pression ambiante, par gravité, reconduit de nouveau dans au moins un élément (8) contenant et/ou conduisant le liquide de process (3) et/ou dans une zone de traitement (4).

10. Procédé selon l'une des revendications précédentes, **caractérisé en ce que**, pour le lavage du côté extérieur (6) des récipients (2) remplis de produits alimentaires et fermés, un flux filtré (46) du liquide de process (3) est acheminé au moins partiellement à une zone de traitement (4) disposée à la fin du processus dans le procédé de traitement de produits alimentaires mis en place dans des récipients (2) fermés.

11. Procédé selon l'une des revendications précédentes, **caractérisé en ce que**, pour le nettoyage du côté intérieur (28) et du côté extérieur (6) de récipients (2) non remplis, un flux filtré (46) du liquide de process (3) est acheminé au moins partiellement à une zone de traitement (4) disposée à l'entrée dans le procédé de traitement de produits alimentaires mis en place dans des récipients (2) fermés.

12. Procédé selon l'une des revendications précédentes, **caractérisé en ce que**, après filtration au moyen de la au moins une installation de filtration par membrane (19), un flux (46) du liquide de process (3) est conduit dans un réservoir de stockage (32) et est reconduit, via un trop-plein constitué sur le réservoir de stockage (32), de nouveau dans au moins un élément (8) contenant et/ou conduisant le liquide de process (3) et/ou dans une zone de traitement (4).

13. Procédé selon la revendication 12, **caractérisé en ce que**, à des intervalles de temps pouvant être fixés, une installation de filtration par membrane (19) est, à des fins de nettoyage de membranes filtrantes pendant le fonctionnement, séparée fluidiquement du reste du dispositif (1) de traitement de produits alimentaires placés dans des récipients (2) fermés, et le filtrat (33) du liquide de process (3) collecté dans le réservoir de stockage (32) est, avec une inversion du sens du flux, conduit via les membranes filtrantes, par rapport au mode de filtration, à travers une installation de filtration par membrane (19).

14. Procédé selon la revendication 13, **caractérisé en ce que** les déchets liquides apparaissant au cours du nettoyage avec inversion du sens du flux via les membranes filtrantes de l'installation de filtration par membrane (19) sont évacués et remplacés par une quantité correspondante de liquide de process (3) frais.

15. Procédé selon l'une des revendications précédentes, **caractérisé en ce que**, en cas de besoin, aussi bien dans le mode traitement et le mode filtration que dans le mode nettoyage pour la au moins une installation de filtration par membrane (19), des produits chimiques en provenance d'une ou de plusieurs sources de produits chimiques (39) sont ajoutés à un flux (20) à filtrer du liquide de process (3) et/ou à un flux filtré (46) du liquide de process (3) au moyen d'un dispositif de dosage (38).

16. Procédé selon l'une des revendications précédentes, **caractérisé en ce que** le degré de contamination est surveillé en continu en particulier par le mesurage de la turbidité du liquide de process (3) au moyen de capteurs (41) dans des éléments de guidage (8) d'une zone de traitement (4) et/ou dans une zone de traitement (4).

17. Procédé selon l'une des revendications précédentes, **caractérisé en ce que**, en fonction des besoins un flux (20), du liquide de process (3) est formé de différents éléments (8) contenant et/ou conduisant le liquide de process (3), est filtré au moyen d'au moins une installation de filtration par membrane (19), et un flux filtré (46) est, après le processus de filtration, reconduit dans au moins un élément (8) contenant et/ou conduisant le liquide de process (3) et/ou dans au moins une zone de traitement (4).

18. Procédé selon la revendication 17, **caractérisé en ce qu'**un flux (20) du liquide de process (3) est, pour la filtration au moyen d'une installation de filtration par membrane (19), formé par commutation entre ou par mélange de différents flux de liquide (5) du liquide de process (3) à partir de différents éléments de guidage (8) en fonction de valeurs de mesure obtenues au cours de mesures en ligne et/ou de mesures aléatoires, et un flux filtré (46) du liquide de process (3) est, en fonction de valeurs de mesure obtenues au cours de mesures en ligne et/ou de mesures aléatoires, reconduit, par l'introduction et/ou la répartition du flux filtré (46) dans différents flux de liquide (5), dans différents éléments de guidage (8) et/ou zones de traitement (4).

19. Procédé selon l'une des revendications précédentes, **caractérisé en ce que** plus d'une installation de filtration par membrane (19) est affectée à une zone de traitement (4), et **en ce qu'**une quantité définie destinée à la formation d'un flux (20) du liquide de process (3) est prélevée en fonction des besoins à partir des flux de liquide (5) du liquide de process (3) conduits à une zone de traitement (4) respective, et un flux (20) est filtré au moyen d'une ou de plusieurs installation(s) de filtration par membrane (19) affectée(s) respectivement à une zone de traitement (4).

20. Dispositif (1) de traitement de produits alimentaires situés dans des récipients (2) fermés au moyen d'un liquide de process (3), comprenant au moins une zone de traitement (4), laquelle zone de traitement (4) est constituée pour l'application du liquide de process (3) sur un côté extérieur (6) des récipients (2) fermés de telle sorte que le liquide de process (3) s'écoule autour du côté extérieur (6) d'un récipient (2) fermé, des moyens de transport (10) destinés au transport des récipients (2) à travers la / les zone(s) de traitement (4), ainsi que des éléments de guidage (8) destinés à l'acheminement de flux de liquide (5) du liquide de process (3) dans une zone de traitement (4), et des éléments de guidage (8) destinés à l'évacuation de flux de liquide (5) du liquide de process (3) à partir d'une zone de traitement (4), d'autres éléments de guidage (8) destinés à contenir et/ou à guider le liquide de process (3) dans le dispositif (1), et au moins un moyen de transfert (11) destiné au transfert de flux de liquide (5) du liquide de process (3) dans les éléments de guidage (8), dans lequel les éléments de guidage (8) sont constitués et disposés de telle sorte que le liquide de process (3) peut, pour le traitement en boucle des produits alimentaires en boucle, être conduit de nouveau dans la zone de traitement (4) ou dans les zones de traitement (4), dans lequel au moins un moyen de chauffage (12) est disposé pour le chauffage du liquide de process (3), et dans lequel au moins un moyen de refroidissement (13) est disposé pour le refroidissement du liquide de process (3), **caractérisé en ce que** le dispositif (1) comprend au moins une installation de filtration par membrane (19), laquelle au moins une installation de filtration par membrane (19) est raccordée fluidiquement par conduite aux éléments de guidage (8) et/ou aux zones de traitement (4) de telle sorte qu'une quantité partielle du liquide de process (3) conduit par unité de temps au total en boucle à travers toutes les zones de traitement (4) existantes peut être utilisée pour la formation d'au moins un flux (20) du liquide de process (3), le flux (20) formé ou les flux (20) formés peut / peuvent être filtré(s) au moyen de la au moins une installation de filtration par membrane (19), et un flux (46) filtré du liquide de process (3) peut être conduit de nouveau à un élément de guidage (8) ou à une zone de traitement (4), dans lequel la au moins une installation de filtration par membrane (19) est disposée en forme de dérivation entre un élément de guidage (8) guidant un flux de liquide (5) et une zone de traitement (4) ou un autre élément de guidage (8), et dans lequel un élément d'alimentation (22) de la au moins une installation de filtration par membrane (19) est raccordé par conduite fluidiquement à un élément de guidage (8) ou à une zone de traitement (4), et dans lequel un élément de dérivation (24) de la au moins une installation de filtration par membrane (19) est raccordé par conduite à un élément de guidage (8) ou à une zone de traitement (4), et dans lequel, côté entrée de la au moins une installation de filtration par membrane (19), au moins un moyen de répartition (21) réglable et/ou plusieurs moyens de répartition (21) qui coopèrent est / sont disposé(s) pour le prélèvement par unité de temps d'une quantité de liquide de process pouvant être fixée à partir d'au moins un élément de guidage (8) contenant le liquide de process (3) et pour la formation du au moins un flux (20) à filtrer du liquide de process (3).

21. Dispositif selon la revendication 20, **caractérisé en ce que**, successivement dans la direction de transport (26) des produits alimentaires ou récipients (2), il est disposé au moins une zone de traitement (4) destinée au chauffage des produits alimentaires et/ou récipients, au moins une zone de traitement (4) destinée à la pasteurisation des produits alimentaires, et au moins une zone de traitement (4) destinée au refroidissement des produits alimentaires et/ou récipients.

22. Dispositif selon la revendication 20 ou 21, **caractérisé en ce que** le nombre et la capacité de filtration de la / des installation(s) de filtration par membrane (19) sont fixés de telle sorte que la quantité de liquide de process utilisée dans le mode de traitement continu à partir d'au moins un élément (8) contenant et/ou conduisant le liquide de process (3) par unité de temps au total pour la formation d'au moins un flux (20) du liquide de process (3) est choisie de telle sorte que la filtration du flux (20) ou des flux (20) permet d'atteindre un taux d'élimination pour des microorganismes qui est supérieur au taux de croissance des microorganismes dans le liquide de process (3) dans le même intervalle de temps.

23. Dispositif selon l'une ou plusieurs des revendications 20 à 22, **caractérisé en ce que** le nombre et la capacité de filtration de la / des installation(s) de filtration par membrane (19) sont fixés de telle sorte que, rapporté à la quantité de liquide de process (3) guidée par unité de temps au total à travers toutes les zones de traitement (4), au moins 1 % et moins de 25 % peuvent être utilisés par unité de temps pour la formation d'au moins un flux (20), et cette quantité partielle du liquide de process (3) formée par unité de temps peut être filtrée au moyen de l'installation / des installation(s) de filtration par membrane (19).

24. Dispositif selon l'une ou plusieurs des revendications 20 à 23, **caractérisé en ce que** le nombre et la capacité de filtration de la / des installation(s) de filtration par membrane (19) sont fixés de telle sorte que le volume de liquide de process (3) contenu au total dans le dispositif (1) pour le traitement de produits alimentaires et/ou de récipients (2) peut être filtré au moins 1 fois et de préférence entre 2 fois et 10 fois par jour au moyen de la / des installation(s) de filtration par membrane (19).

25. Dispositif selon l'une ou plusieurs des revendications 20 à 24, **caractérisé en ce que**, pour la formation d'un flux (20) à filtrer, la au moins une installation de filtration par membrane (19) est raccordée par conduite fluidiquement à des éléments de guidage (8) à des emplacements où le liquide de process (3) contenu dans les éléments de guidage (8) présente une température inférieure à 80 °C et en particulier inférieure à 50 °C.

26. Dispositif selon l'une ou plusieurs des revendications 20 à 25, **caractérisé en ce que**, pour le retour d'un flux filtré (46) du liquide de process (3) une fois la filtration effectuée, des éléments de dérivation (24) d'au moins une installation de filtration par membrane (19) sont raccordés par conduite à au moins un élément de guidage (8) et/ou à au moins une zone de traitement (4) de telle sorte que le au moins un flux filtré (46) du liquide de process (3) peut être acheminé à l'élément ou aux élément(s) de guidage (8) et/ou à la ou aux zone(s) de traitement (4) en présence de l'action de la force de la pesanteur par gravité.

27. Dispositif selon l'une ou plusieurs des revendications 20 à 26, **caractérisé en ce qu'**il est constitué au moins une zone de traitement (4) pour le lavage du côté extérieur (6) de récipients (2) remplis de produits alimentaires et fermés, laquelle zone de traitement (4) est disposée à l'extrémité du parcours de la zone de traitement par rapport au sens de transport (26) des récipients (2) à travers les zones de traitement (4), et laquelle zone de traitement (4) est, pour le lavage des récipients (2) par l'acheminement d'un flux filtré (46) du liquide de process (3), raccordée par conduite à au moins un élément de dérivation (24) d'une installation de filtration par membrane (19).

28. Dispositif selon l'une ou plusieurs des revendications 20 à 27, **caractérisé en ce qu'**il est constitué au moins une zone de traitement (4) pour le nettoyage du côté intérieur (28) et du côté extérieur (6) de récipients (2) non remplis et non fermés, laquelle zone de traitement (4) est disposée côté entrée du parcours de la zone de traitement par rapport au sens de transport (26) des récipients (2) à travers les zones de traitement (4) et est, pour le nettoyage des récipients (2) par l'acheminement d'un flux filtré (46) du liquide de process (3), raccordée par conduite à au moins un élément de dérivation (24) d'une installation de filtration par membrane (19).

29. Dispositif selon l'une ou plusieurs des revendications 20 à 28, **caractérisé en ce qu'**un réservoir de stockage (32) avec trop-plein est constitué dans un élément de dérivation (24) de la au moins une installation de filtration par membrane (19).

30. Dispositif selon la revendication 29, **caractérisé en ce que**, en vue du nettoyage d'une installation de filtration par membrane (19), des moyens de blocage (34) sont constitués dans les éléments d'alimentation (22) et les éléments de dérivation (24) pour la séparation fluidique de la au moins une installation de filtration par membrane (19) par rapport au reste du dispositif (1) et, dans le réservoir de stockage (32) et/ou dans une conduite de rétro-lavage (35) s'étendant entre le réservoir de stockage (32) et l'élément de dérivation (24) de l'installation de filtration par membrane (19), il est disposé au moins un moyen de transfert (11), lequel est disposé pour le transfert du filtrat (33) du liquide de process (3) collecté dans le réservoir de stockage (32) en sens inverse (36), par rapport au sens du flux, via les membranes filtrantes dans le mode filtration à travers la au moins une installation de filtration par membrane (19).

31. Dispositif selon la revendication 30, **caractérisé en ce qu'**au moins une conduite de déchets liquides (37) pouvant être bloquée est affectée pour l'évacuation de déchets liquides de l'installation de filtration par membrane (19) apparaissant au cours du nettoyage avec inversion du sens du flux via les membranes filtrantes de l'installation de filtration par membrane (19), et, dans le dispositif (1), au moins un dispositif d'acheminement (17) pouvant être bloqué est disposé pour le remplacement des déchets liquides évacués par du liquide de process (3) frais.

32. Dispositif selon l'une ou plusieurs des revendications 20 à 31, **caractérisé en ce que**, dans un élément de dérivation (24) et/ou dans la conduite de rétro-lavage (35) de la au moins installation de filtration par membrane (19), il est disposé un dispositif de dosage (38) au moyen duquel des produits chimiques en provenance d'une ou de plusieurs sources de produits chimiques (39) peuvent être ajoutés au liquide de process (3) et/ou à un filtrat (33) du liquide de process (3), aussi bien dans le mode filtration que dans le mode nettoyage pour l'installation de filtration par membrane (19).

33. Dispositif selon l'une ou plusieurs des revendications 20 à 32, **caractérisé en ce que** des capteurs (41) destinés à la surveillance continue du degré de contamination, en particulier par le mesurage de la turbidité du liquide de process (3), sont disposés dans des éléments de guidage (8) et/ou dans des zones de traitement (4).

34. Dispositif selon l'une ou plusieurs des revendications 20 à 33, **caractérisé en ce qu'**au moins un moyen de commutation (42) est affecté à un élément d'alimentation (22) d'une installation de filtration par membrane (19), lequel est raccordé par conduite fluidiquement à au moins deux éléments de guidage (8) différents contenant le liquide de process (3) de telle sorte qu'une formation du flux (20) à filtrer du liquide de process (3) peut être effectuée au choix à partir d'un des flux de liquide (5) ou de plusieurs flux de liquide (5) du liquide de process (3) dans les éléments de guidage (8) ou à partir de plusieurs flux de liquide (5) du liquide de process (3).

35. Dispositif selon l'une ou plusieurs des revendications 20 à 34, **caractérisé en ce qu'**au moins un moyen de mixage (43) est affecté à un élément d'alimentation (22) d'une installation de filtration par membrane (19), lequel est raccordé par conduite fluidiquement à au moins deux éléments de guidage (8) différents contenant le liquide de process (3) de telle sorte qu'une formation du flux (20) à filtrer du liquide de process (3) peut être effectuée au choix à partir d'un des flux de liquide (5) ou de plusieurs flux de liquide (5) du liquide de process (3) dans les éléments de guidage (8), ou la formation du flux (20) à filtrer du liquide de process (3) à travers l'installation de filtration par membrane (19) peut être effectuée par prélèvement et mixage de quantités partielles pouvant être fixées à partir de plusieurs flux de liquide (5) du liquide de process (3).

36. Dispositif selon l'une ou plusieurs des revendications 20 à 35, **caractérisé en ce qu'**au moins un élément de commutation (44) est affecté à un élément de dérivation (24) d'une installation de filtration par membrane (19), lequel est raccordé par conduite fluidiquement à au moins un élément de guidage (8) contenant le liquide de process (3) et/ou à au moins une zone de traitement (4) de telle sorte que l'acheminement d'un flux filtré (46) du liquide de process (3) dans le au moins un élément de guidage (8) et/ou dans la au moins une zone de traitement (4) peut être fixé de façon contrôlée.

37. Dispositif selon l'une ou plusieurs des revendications 20 à 36, **caractérisé en ce qu'**au moins un élément de répartition (45) est affecté à un élément de dérivation (24) d'une installation de filtration par membrane (19), lequel est raccordé par conduite fluidiquement à au moins un élément de guidage (8) contenant le liquide de process (3) et/ou à au moins une zone de traitement (4) de telle sorte qu'un acheminement d'un flux filtré (46) du liquide de process (3) dans le au moins un élément de guidage (8) et/ou dans la au moins une zone de traitement (4) peut être fixé de façon contrôlée, ou des quantités pouvant être fixées du flux filtré (46) du liquide de process (3) peuvent être acheminées dans le au moins un élément de guidage (8) et/ou dans la au moins une zone de traitement (4).
